# EUROPEAN PATENT SPECIFICATION

(11) **EP 2 652 126 B1**
(45) Date of publication and mention of the grant of the patent: **13.07.2016**
(21) Application number: 11849864.1
(22) Date of filing: 15.12.2011
(51) Int. Cl.: C12N 5/071, A61K 35/50, C12N 5/073, C12N 5/0775, A61K 35/12

(54) **TREATMENT OF IMMUNE-RELATED DISEASES AND DISORDERS USING AMNION DERIVED ADHERENT CELLS**
BEHANDLUNG IMMUNBEDINGTER ERKRANKUNGEN UND STÖRUNGEN UNTER VERWENDUNG VON AUS AMNION GEWONNENEN ADHÄRENTEN ZELLEN
TRAITEMENT DE MALADIES ET DE TROUBLES LIÉS À LA RÉPONSE IMMUNE AU MOYEN DE CELLULES ADHÉRENTES DÉRIVÉES DE L'AMNIOS

(30) Priority: 17.12.2010 US 201061424593 P
(43) Date of publication of application: 23.10.2013
(73) Proprietor: Anthrogenesis Corporation, Warren, NJ 07059 (US)
(72) Inventor: ABBOT, Stewart, Warren, NJ 07059 (US); EDINGER, James, W., Belford, NJ 07718 (US); FRANCKI, Aleksandar, Annandale, NJ 08801 (US); JANKOVIC, Vladimir, New York, NY 10044 (US); LIANG, Bitao, Closter, NJ 07624 (US)
(74) Representative: Bezzubova, Olga
(86) International application number: PCT/US2011/065158
(87) International publication number: WO 2012/083021

(56) References cited:
- WO-A1-2010/059828
- WO-A2-2008/100498
- WO-A2-2011/064669
- US-A1- 2009 124 573
- US-A1- 2010 124 569
- BILIC GROZDANA ET AL: "Comparative Characterization of Cultured Human Term Amnion Epithelial and Mesenchymal Stromal Cells for Application in Cell Therapy", CELL TRANSPLANTATION, vol. 17, no. 8, 2008, pages 955-968, XP9177111, ISSN: 0963-6897
- XIN LI ET AL: "Human Placenta-Derived Adherent Cells Prevent Bone loss, Stimulate Bone formation, and Suppress Growth of Multiple Myeloma in Bone", STEM CELLS, ALPHAMED PRESS, DAYTON, OH, US, vol. 29, no. 2, 23 November 2010 (2010-11-23), pages 263-273, XP002631698, ISSN: 1066-5099, DOI: 10.1002/STEM.572 [retrieved on 2011-02-24]
- PAROLINI O ET AL: "Review: Preclinical studies on placenta-derived cells and amniotic membrane: An update", PLACENTA, W.B. SAUNDERS, GB, vol. 32, 13 December 2010 (2010-12-13), pages S186-S195, XP028160648, ISSN: 0143-4004, DOI: 10.1016/J.PLACENTA.2010.12.016 [retrieved on 2010-12-21]
- PAROLINI ORNELLA ET AL: "Concise review: isolation and characterization of cells from human term placenta: outcome of the first international Workshop on Placenta Derived Stem Cells", STEM CELLS, ALPHAMED PRESS, INC, UNITED STATES, vol. 26, no. 2, 1 February 2008 (2008-02-01), pages 300-311, XP002513690, ISSN: 1549-4918, DOI: 10.1634/STEMCELLS .2007-0594

## Description

### 1. FIELD

Provided herein are methods of modulating, *e.g.*, reducing, an immune response, *in vivo* or *in vitro*, comprising contacting one or more type of immune cell with an effective amount of amnion derived adherent cells, referred to as AMDACs. Also provided herein are methods of treating individuals having an unwanted or inappropriate immune response, e.g., an autoimmune disease, graft-versus-host disease, allergy or asthma, comprising administering AMDACs to such individuals.

### 2. BACKGROUND

Inflammatory and immune-related diseases and conditions such as autoimmune diseases, graft-versus-host disease, allergies and asthma continue to be important medical problems. There is a need for improved therapeutics for such conditions.

### 3. SUMMARY

In one aspect, provided herein is a method of treating an individual having, or at risk of developing, a disease or disorder associated with or caused by an inappropriate or unwanted immune response, comprising administering to the individual a therapeutically effective amount of amnion-derived adherent cells (AMDACs), or culture medium conditioned by AMDACs, wherein said therapeutically effective amount is an amount sufficient to cause a detectable improvement in one or more symptoms of said disease, disorder or condition, and wherein said AMDACs are OCT-4⁻ (negative for OCT-4, also known as POU5F1 or octamer binding protein 4) as determinable by reverse transcriptase polymerase chain reaction (RT-PCR), and are adherent to tissue culture plastic.

In a specific embodiment, the disease or disorder is caused by or associated with a pro-inflammatory response, *e.g.,* a Th1 response or a Th17 response, and administering said AMDACs suppresses said pro-inflammatory response, *e.g.*, said Th1 response or Th17 response. In a specific embodiment, the method comprises contacting T cells (*e.g.,* CD4⁺ T lymphocytes or leukocytes) that mediate said Th1 or Th17 response with AMDACs. In a specific embodiment, said contacting detectably reduces Th1 cell maturation. In a specific embodiment of the method, said contacting detectably reduces the production of one or more of lymphotoxin-1α (LT-1α), interleukin-1β (IL-1β), IL-12, IL-17, IL-21, IL-23, tumor necrosis factor alpha (TNFα) and/or interferon gamma (IFNγ) by said T cells *(e.g.,* in said individual), or by an antigen-producing cell. In another specific embodiment of the method, said contacting potentiates or upregulates a regulatory T cell (Treg) phenotype, and/or reduces expression in a dendritic cell (DC) and/or macrophage of biomolecules that promote a Th1 and/or Th17 response (*e.g.*, CD80, CD83, CD86, ICAM-1, HLA-II). In a specific embodiment, said T cells are also contacted with IL-10, *e.g.*, exogenous IL-10 or IL-10 not produced by said T cells, *e.g.*, recombinant IL-10.

In a specific embodiment of any of the above methods of treatment, said disease or disorder is an allergy, asthma, or a reaction to an antigen exogenous to said individual. In another specific embodiment of any of the above methods of treatment, said disease or disorder is graft-versus-host disease.

In another specific embodiment of any of the above methods of treatment, said disease or disorder is an autoimmune disease. In certain specific embodiments, said autoimmune disease is inflammatory bowel disease (*e.g.*, Crohn's disease or ulcerative colitis), multiple sclerosis, rheumatoid arthritis, psoriasis, lupus erythematosus, diabetes, mycosis fungoides, or scleroderma. In certain other specific embodiments, said autoimmune disease is one or more of Addison's disease, alopecia areata, ankylosing spondylitis, antiphospholipid antibody syndrome, antiphospholipid syndrome (primary or secondary), asthma, autoimmune gastritis, autoimmune hemolytic anemia, autoimmune hepatitis, autoimmune inner ear disease, autoimmune lymphoproliferative disease, autoimmune thrombocytopenic purpura, Balo disease, Behcet's disease, bullous pemphigoid, cardiomyopathy, celiac disease, Chagas disease, chronic inflammatory demyelinating polyneuropathy, cicatrical pemphigoid (*e.g.*, mucous membrane pemphigoid), cold agglutinin disease, degos disease, dermatitis hepatiformis, dermatomyositis (juvenile), essential mixed cryoglobulinemia, Goodpasture's syndrome, Graves' disease, Guillain-Barre syndrome, Hashimoto's thyroiditis (Hashimoto's disease; autoimmune thyroditis), idiopathic pulmonary fibrosis, idiopathic thrombocytopenia purpura, IgA nephropathy, juvenile arthritis, lichen planus, Ménière disease, mixed connective tissue disease, morephea, myasthenia gravis, narcolepsy, neuromyotonia, pediatric autoimmune neuropsychiatric disorders (PANDAs), pemphigus vulgaris, pernicious anemia, polyarteritis nodosa, polychondritis, polymyalgia rheumatica, polymyositis (*e.g.*, with dermatomyositis), primary agammaglobulinemia, primary biliary cirrhosis, Raynaud disease (Raynaud phenomenon), Reiter's syndrome, relapsing polychondritis, rheumatic fever, Sjogren's syndrome, stiff-person syndrome (Moersch-Woltmann syndrome), Takayasu's arteritis, temporal arteritis (giant cell arteritis), uveitis, vasculitis (*e.g.*, vasculitis not associated with lupus erythematosus), vitiligo, and/or Wegener's granulomatosis.

In a specific embodiment, in which the autoimmune disease is inflammatory bowel disease, said inflammatory bowel disease is Crohn's disease. In more specific embodiments, said Crohn's disease is gastroduodenal Crohn's disease, jejunoileitis, ileocolitis, or Crohn's colitis. In another specific embodiment, said inflammatory bowel disease is ulcerative colitis. In certain specific embodiments, said ulcerative colitis is pancolitis, limited colitis, distal colitis, or proctitis. In certain specific embodiments, said symptom of inflammatory bowel disease, *e.g.*, Crohn's disease, is one or more of inflammation and swelling of a part of the gastrointestinal (GI) tract, abdominal pain, frequent emptying of the bowel, diarrhea, rectal bleeding, anemia, weight loss, arthritis, skin problems, fever, thickening of the intestinal wall, formation of scar tissue in the intestine of the individual, formation of sores or ulcers in the intestine of the individual, development of one or more fistulas in the wall of the intestinal of the individual, development of one or more fissures in the anus of the individual, development of nutritional deficiencies (*e.g.*, deficiencies in one or more of proteins, calories, vitamins), development of kidney stones, or development of gallstones. In certain other specific embodiments, said symptom of inflammatory bowel disease, *e.g.*, ulcerative colitis, is one or more of abdominal pain, bloody diarrhea, fevers, nausea, abdominal cramps, anemia, fatigue, weight loss, loss of appetite, rectal bleeding, loss of bodily fluids and nutrients, skin lesions, joint pain, and growth failure. In another more specific embodiment, the symptom is osteoporosis, eye inflammation, or liver disease.

In another embodiment of the methods of treatment disclosed herein, said disease or disorder is scleroderma. In specific embodiments, the scleroderma is diffuse scleroderma, limited scleroderma (CREST syndrome), morphea, or linear scleroderma. In certain specific embodiments, said symptoms of scleroderma comprise one or more of hardening of the skin of the face, hardening of the skin of the fingers, Reynaud's syndrome, inappropriate vasoconstriction in an extremity, calcinosis, telangiectasia, or esophageal dysmotility.

In another embodiment of the methods of treatment disclosed herein, said disease or disorder is psoriasis. In certain specific embodiments, said symptom of psoriasis is one or more of psoriatic arthritis, scaling of the skin, redness of the skin, thickening of the skin, formation of plaques, discoloration under the nail plate, pitting of the nails, lines going across the nails, thickening of the skin under the nail, onycholysis, development of pustules, joint or connective tissue inflammation, inflammation of the skin, or exfoliation of the skin.

In another embodiment of the methods of treatment disclosed herein, said disease or disorder is multiple sclerosis. In certain specific embodiments, said symptom of multiple sclerosis is one or more of a sensory disturbance in a limb of the individual, optic nerve dysfunction, pyramidal tract dysfunction, bladder dysfunction, bowel dysfunction, sexual dysfunction, ataxia, or diplopia.

In another embodiment of the methods of treatment disclosed herein, said disease or disorder is rheumatoid arthritis. In certain specific embodiments, said rheumatoid arthritis involves one or more of pyoderma gangrenosum, neutrophilic dermatosis, Sweet's syndrome, viral infection, erythema nodosum, lobular panniculitis, atrophy of digital skin, palmar erythema, diffuse thinning (rice paper skin), skin fragility, subcutaneous nodules on an exterior surface, *e.g.,* on the elbows, fibrosis of the lungs (*e.g.,* as a consequence of methotrexate therapy), Caplan's nodules, vasculitic disorders, nail fold infarcts, neuropathy, nephropathy, amyloidosis, muscular pseudohypertrophy, endoscarditis, left ventricular failure, valulitis, scleromalacia, mononeuritis multiplex, or atlanto-axial subluxation.

In another embodiment of the methods of treatment disclosed herein, said disease or disorder is lupus erythematosus. In certain specific embodiments, said symptom of lupus erythematosus is one or more of malar rash, development of thick red scaly patches on the skin, alopecia, mouth ulcers, nasal ulcers, vaginal ulcers, skin lesions, joint pain, anemia deficiency, iron deficiency, lower than normal platelet and white blood cell counts, antiphospholipid antibody syndrome, presence of anticardiolipin antibody in the blood, pericarditis, myocarditis, endocarditis, lung inflammation, pleural inflammation, pleuritis, pleural effusion, lupus pneumonitis, pulmonary hypertension, pulmonary emboli, pulmonary hemorrhage, autoimmune hepatitis, jaundice, presence of antinuclear antibody (ANA) in the blood, presence of smooth muscle antibody (SMA) in the blood, presence of liver/kidney microsomal antibody (LKM-1) in the blood, presence of anti-mitochondrial antibody (AMA) in the blood, hematuria, proteinuria, lupus nephritis, renal failure, development of membranous glomerulonephritis with "wire loop" abnormalities, seizures, psychosis, abnormalities in the cerebrospinal fluid, deficiency in CD45 phosphatase and/or increased expression of CD40 ligand in T cells of the individual, lupus gastroenteritis, lupus pancreatitis, lupus cystitis, autoimmune inner ear disease, parasympathetic dysfunction, retinal vasculitis, systemic vasculitis, increased expression of FcεRIγ, increased and sustained calcium levels in T cells, increase of inositol triphosphate in the blood, reduction in protein kinase C phosphorylation, reduction in Ras-MAP kinase signaling, or a deficiency in protein kinase A I activity.

In another embodiment of the methods of treatment disclosed herein, said disease or disorder is diabetes. In certain specific embodiments, said symptom is one or more of abnormally high blood sugar, lack of insulin resistance as determinable by a glucose tolerance test, fatigue, or loss of consciousness.

In another embodiment of the methods of treatment disclosed herein, said disease, disorder or condition is mycosis fungoides (Alibert-Bazin syndrome). In certain specific embodiments, said mycosis fungoides is in the patch phase, the skin tumor phase, the skin redness (erythroderma) stage, or the lymph node stage. In certain other specific embodiments, said symptom of mycosis fungoides is one or more of development of flat red patches that are itchy, development of flat, red patches that are raised and hard (plaques), development of raised lumps (nodules), development of large red itchy scaly areas over the body, cracking of the skin of the palms and soles, thickening of the skin of the palms and soles, or inflammation of the lymph nodes.

The AMDACs useful in the methods of treatment disclosed herein may be identified by different combinations of cellular and genetic markers. In a specific embodiment, for example, said AMDACs are OCT-4⁻ as determinable by RT-PCR. In another embodiment, the AMDACs are CD49f⁺, as determinable by flow cytometry. In yet another embodiment, the AMDACs are OCT-4⁻ and CD49f⁺ as determinable by RT-PCR and flow cytometry, respectively. In still another embodiment, the AMDACs are CD49f⁺, CD105⁺, and CD200⁺ as determinable by immunolocalization, *e.g.*, flow cytometry. In another embodiment, the AMDACs are OCT-4⁻ as determinable by RT-PCR and CD49f⁺, CD105⁺, and CD200⁺ as determinable by immunolocalization, *e.g.*, flow cytometry. In another specific embodiment, said AMDACs are positive for VEGFR1/Flt-1 (vascular endothelial growth factor receptor 1) or CD309 (also known as VEGFR2/KDR (vascular endothelial growth factor receptor 2)), as determinable by immunolocalization, *e.g.*, flow cytometry. In another specific embodiment, said AMDACs are CD90⁺ and CD117⁻ as determinable by flow cytometry, and HLA-G-, as determinable by RT-PCR. In another specific embodiment, said AMDACs are OCT-4⁻ and HLA-G⁻, as determinable by RT-PCR, and CD49f⁺, CD90⁺, CD105⁺, and/or CD117⁻ as determinable by immunolocalization, *e.g.*, flow cytometry. In another specific embodiment, any of the above AMDACs are additionally one or more of CD9⁺, CD10⁺, CD44⁺, CD54⁺, CD98⁺, Tie-2⁺ (angiopoietin receptor), TEM-7⁺ (tumor endothelial marker 7), CD31⁻, CD34⁻, CD45⁻, CD133⁻, CD143⁻, CD146⁻, or CXCR4⁻(chemokine (C-X-C motif) receptor 4) as determinable by immunolocalization, *e.g.*, flow cytometry. In another specific embodiment, any of the above AMDACs are additionally CD9^{+,} CD10⁺, CD44⁺, CD54⁺, CD98⁺, Tie-2⁺, TEM-7⁺, CD31⁻, CD34⁻, CD45⁻, CD133⁻, , CD143⁻, CD146⁻, and CXCR4⁻ as determinable by immunolocalization, *e.g.*, flow cytometry.

In another specific embodiment, the AMDACs are GFAP⁺, *e.g.,* as determinable by a short-term neural differentiation assay (see, *e.g.,* Section 6.3.3, below). In another specific embodiment, the AMDACs are beta-tubulin III (Tuj1)⁺, *e.g.,* as determinable by a short-term neural differentiation assay (see, *e.g.,* Section 6.3.3, below). In another specific embodiment, the AMDACs are OCT-4⁻, GFAP⁺, and beta-tubulin III (Tuj 1)⁺. In another specific embodiment, the AMDACs described herein are OCT-4⁻, CD200⁺, CD105⁺, and CD49f⁺. In another specific embodiment, the AMDACs are CD200⁺, CD105⁺, CD90⁺, and CD73⁺. In another specific embodiment, the AMDACs described herein are CD117⁻ and are not selected using an antibody to CD117. In another specific embodiment, the AMDACs are CD 146- and are not selected using an antibody to CD 146. In another specific embodiment, the AMDACs are OCT-4- and do not express CD34 following induction with VEGF as determinable by RT-PCR and/or immunolocalization (*e.g.*, flow cytometry). In another specific embodiment, said AMDACs are OCT-4⁻, as determinable by RT-PCR, and CD49f⁺, HLA-G⁻, CD90⁺, CD105⁺, CD117⁻, and CD200⁺, as determinable by immunolocalization, *e.g.,* flow cytometry.

In another specific embodiment, the AMDACs useful in the methods described herein are neurogenic, as determined by a short-term neural differentiation assay (see, *e.g.,* Section 6.3.3, below). In another specific embodiment, the AMDACs useful in the methods described herein are non-chondrogenic as determined by an in vitro chondrogenic potential assay (see, *e.g.,* Section 6.3.2, below). In another specific embodiment, the AMDACs useful in the methods described herein are non-osteogenic as determined by an osteogenic phenotype assay (see, *e.g.,* Section 6.3.1, below). In another specific embodiment, the AMDACs described herein are non-osteogenic after being cultured for up to 6 weeks (e.g., for 2 weeks, for 4 weeks, or for 6 weeks) in DMEM at pH 7.4 (High glucose) supplemented with 100 nM Dexamethasone, 10 mM β-glycerol phosphate, 50 µM L-ascorbic acid-2-phosphate, wherein osteogenesis is assessed using von Kossa staining; alizarin red staining; or by detecting the presence of osteopontin, osteocalcin, osteonectin, and/or bone sialoprotein by, e.g., RT-PCR.

In more specific embodiments, any of the above AMDACs additionally: (a) express one or more of CD9, CD 10, CD44, CD54, CD98, CD200, Tie-2, TEM-7, VEGFR1/Flt-1, or VEGFR2/KDR (CD309), as determinable by immunolocalization, *e.g.*, flow cytometry; (b) lack expression of one or more of CD31, CD34, CD38, CD45, CD133, CD143, CD144, CD146, CD271, CXCR4, HLA-G, or VE-cadherin, as determinable by immunolocalization, *e.g.*, flow cytometry; (c) lack expression of SOX2, as determinable by RT-PCR; (d) express mRNA for one or more of ACTA2, ADAMTS1, AMOT, ANG, ANGPT1, ANGPT2, ANGPTL1, ANGPTL2, ANGPTL4, BAI1, c-myc, CD44, CD140a, CD140b, CD200, CD202b, CD304, CD309, CEACAM1, CHGA, COL15A1, COL18A1, COL4A1, COL4A2, COL4A3, Connexin-3, CSF3, CTGF, CXCL12, CXCL2, DNMT3B, ECGF1, EDG1, EDIL3, ENPP2, EPHB2, FBLN5, F2, FGF1, FGF2, FIGF, FLT4, FN1, FST, FOXC2, Galectin-1, GRN, HGF, HEY1, HSPG2, IFNB1, IL8, IL12A, ITGA4, ITGAV, ITGB3, KLF-4, MDK, MMP2, MYOZ2, NRP2, PDGFB, PF4, PGK1, PROX1, PTN, SEMA3F, SERPINB5, SERPINC1, SERPINF1, TGFA, TGFB1, THBS1, THBS2, TIE1, TIMP2, TIMP3, TNF, TNNC1, TNNT2, TNFSF15, VASH1, VEGF, VEGFB, VEGFC, or VEGFR1/FLT1; (e) produce one or more of the proteins CD49d, Connexin-43, HLA-ABC, Beta 2-microglobulin, CD349, CD318, PDL1, CD106, Galectin-1, ADAM 17, angiotensinogen precursor, filamin A, alpha-actinin 1, megalin, macrophage acetylated LDL receptor I and II, activin receptor type IIB precursor, Wnt-9 protein, glial fibrillary acidic protein, astrocyte, myosin-binding protein C, or myosin heavy chain, nonmuscle type A; (f) secrete one or more of vascular endothelial growth factor (VEGF), hepatocyte growth factor (HGF), interleukin-8 (IL-8), monocyte chemotactic protein-3 (MCP-3), FGF2, Follistatin, G-CSF, EGF, ENA-78, GRO, IL-6, MCP-1, PDGF-BB, TIMP-2, uPAR, or galectin-1 into culture medium in which the AMDACs grows; (g) express one or more of micro RNAs miR-17-3p, miR-18a, miR-18b, miR-19b, miR-92, or miR-296 at a higher level than an equivalent number of bone marrow-derived mesenchymal stem cells; (h) express one or more of micro RNAs miR-20a, miR-20b, miR-221, miR-222, miR-15b, or miR-16 at a lower level than an equivalent number of bone marrow-derived mesenchymal stem cells; (i) express one or more of miRNAs miR-17-3p, miR-18a, miR-18b, miR-19b, miR-92, miR-20a, miR-20b, miR-296, miR-221, miR-222, miR-15b, and/or miR-16; or (j) express increased levels of one or more of CD202b, IL-8 or VEGF when cultured in less than about 5% O₂, compared to expression of CD202b, IL-8 or VEGF when cultured under 21% O₂. In a more specific embodiment, said AMDACs are OCT-4⁻, as determinable by RT-PCR, and CD49f⁺, HLA-G⁻, CD90⁺, CD105⁺, CD117⁻, and CD200⁺, as determinable by immunolocalization, *e.g.*, flow cytometry. In another specific embodiment, said AMDACs are OCT-4⁻, as determinable by RT-PCR, and CD49f⁺, HLA-G⁻, CD90⁺, CD105⁺, and CD117⁻, as determinable by immunolocalization, *e.g.,* flow cytometry, and wherein said AMDACs additionally: (a) express CD9, CD10, CD44, CD54, CD98, CD200, Tie-2, TEM-7, VEGFR1/Flt-1, and VEGFR2/KDR (CD309), as determinable by immunolocalization, *e.g.*, flow cytometry; (b) lack expression of CD31, CD34, CD38, CD45, CD133, CD143, CD144, CD146, CD271, CXCR4, HLA-G, and VE-cadherin, as determinable by immunolocalization, *e.g.*, flow cytometry; (c) lack expression of SOX2, as determinable by RT-PCR; (d) express mRNA for ACTA2, ADAMTS1, AMOT, ANG, ANGPT1, ANGPT2, ANGPTL1, ANGPTL2, ANGPTL4, BAI1, c-myc, CD44, CD140a, CD140b, CD200, CD202b, CD304, CD309, CEACAM1, CHGA, COL15A1, COL18A1, COL4A1, COL4A2, COL4A3, Connexin-3, CSF3, CTGF, CXCL12, CXCL2, DNMT3B, ECGF1, EDG1, EDIL3, ENPP2, EPHB2, FBLN5, F2, FGF1, FGF2, FIGF, FLT4, FN1, FST, FOXC2, Galectin-1, GRN, HGF, HEY1, HSPG2, IFNB1, IL8, IL12A, ITGA4, ITGAV, ITGB3, KLF-4, MDK, MMP2, MYOZ2, NRP2, PDGFB, PF4, PGK1, PROX1, PTN, SEMA3F, SERPINB5, SERPINC1, SERPINF1, TGFA, TGFB1, THBS1, THBS2, TIE1, TIMP2, TIMP3, TNF, TNNC1, TNNT2, TNFSF15, VASH1, VEGF, VEGFB, VEGFC, and VEGFR1/FLT1 as determinable by RT-PCR; (e) produce the proteins CD49d, Connexin-43, HLA-ABC, Beta 2-microglobulin, CD349, CD318, PDL1, CD106, Galectin-1, ADAM 17, angiotensinogen precursor, filamin A, alpha-actinin 1, megalin, macrophage acetylated LDL receptor I and II, activin receptor type IIB precursor, Wnt-9 protein, glial fibrillary acidic protein, astrocyte, myosin-binding protein C, and/or myosin heavy chain, nonmuscle type A; (f) secrete VEGF, HGF, IL-8, MCP-3, FGF2, Follistatin, G-CSF, EGF, ENA-78, GRO, IL-6, MCP-1, PDGF-BB, TIMP-2, uPAR, and Galectin-1 into culture medium in which the cell grows; (g) express micro RNAs miR-17-3p, miR-18a, miR-18b, miR-19b, miR-92, and miR-296 at a higher level than an equivalent number of bone marrow-derived mesenchymal stem cells; (h) express micro RNAs miR-20a, miR-20b, miR-221, miR-222, miR-15b, and miR-16 at a lower level than an equivalent number of bone marrow-derived mesenchymal stem cells; (i) express miRNAs miR-17-3p, miR-18a, miR-18b, miR-19b, miR-92, miR-20a, miR-20b, miR-296, miR-221, miR-222, miR-15b, and miR-16; or (j) express increased levels of CD202b, IL-8 and/or VEGF when cultured in less than about 5% O₂, compared to expression of CD202b, IL-8 and/or VEGF under 21% O₂.

In other embodiments, for example, the amnion derived adherent cells are adherent to tissue culture plastic, and are OCT-4⁻, as determinable by RT-PCR for 30 cycles, *e.g.,* as compared to an appropriate control cell line, such as an embryonal carcinoma-derived stem cell line (*e.g.,* NTERA-2, *e.g.,* available from the American Type Culture Collection, ATCC Number CRL-1973). In a specific embodiment, the cells are OCT-4⁻, as determinable by RT-PCR, and VEGFR1/Flt-1⁺ (vascular endothelial growth factor receptor 1) and/or VEGFR2/KDR⁺ (vascular endothelial growth factor receptor 2, also known as kinase insert domain receptor), as determinable by immunolocalization, *e.g.*, flow cytometry. In another specific embodiment, the cells are OCT-4⁻, as determinable by RT-PCR, and CD49f⁺ (integrin-α6⁺), as determinable by immunolocalization, *e.g.*, flow cytometry. In a specific embodiment, said cells are OCT-4⁻, as determinable by RT-PCR, and HLA-G⁻, as determinable by RT-PCR. In another specific embodiment, said cells are OCT-4⁻, as determinable by RT-PCR, and CD90⁺, CD105⁺, or CD117⁻ as determinable by immunolocalization, *e.g.*, flow cytometry. In a more specific embodiment, said OCT-4⁻ cells are CD90⁺, CD105⁺, and CD117⁻. In another specific embodiment, the cells are OCT-4⁻, and do not express SOX2, *e.g.,* as determinable by RT-PCR for 30 cycles.

In another embodiment, said OCT-4⁻ cells are one or more of CD29⁺, CD73⁺, ABC-p⁺, and CD38⁻, as determinable by immunolocalization, *e.g.*, flow cytometry.

In another specific embodiment, said OCT-4⁻ amnion derived adherent cells are additionally one or more of CD9⁺, CD10⁺, CD44⁺, CD54⁺, CD98⁺, Tie-2⁺ (angiopoietin receptor), TEM-7⁺ (tumor endothelial marker 7), CD31⁻, CD34⁻, CD45⁻, CD133⁻, CD143⁻ (angiotensin-I-converting enzyme, ACE), CD 146- (melanoma cell adhesion molecule), CXCR4⁻ (chemokine (C-X-C motif) receptor 4) as determinable by immunolocalization, *e.g.*, flow cytometry. In a more specific embodiment, said amnion derived adherent cells are CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻, and CXCR4- as determinable by immunolocalization, *e.g.*, flow cytometry. In another more specific embodiment, the amnion derived adherent cells provided herein are OCT-4⁻, as determinable by RT-PCR; VEGFR1/Flt-1⁺ and/or VEGFR2/KDR⁺, as determinable by immunolocalization, *e.g.*, flow cytometry; and one or more, or all, of CD31⁻, CD34⁻, CD45⁻, CD133⁻, and/or Tie-2⁻ as determinable by immunolocalization, *e.g.*, flow cytometry. In a specific embodiment, the amnion derived adherent cells express at least 2 log less PCR-amplified mRNA for OCT-4 at, *e.g.,* >20 cycles (*e.g.,* 20-30 cycles), than an equivalent number of NTERA-2 cells. In another specific embodiment, said OCT-4- cells are additionally VE-cadherin⁻ (CD144⁻) as determinable by immunolocalization, *e.g.*, flow cytometry. In another specific embodiment, said OCT-4- cells are additionally positive for CD105⁺ and CD200⁺ as determinable by immunolocalization, *e.g.*, flow cytometry. In another specific embodiment, said OCT-4- cells do not express CD34, *e.g.,* as detected by immunolocalization, *e.g.*, flow cytometry after exposure to 1 to 100 ng/mL VEGF (vascular endothelial growth factor) for 4 to 21 days.

In another embodiment, the amnion derived adherent cells are adherent to tissue culture plastic, and are OCT-4⁻ and SOX-2⁻, as determinable by RT-PCR. In yet another embodiment, said cells are CD90⁺, CD105⁺, and CD117⁻, as determinable by immunolocalization, *e.g.*, flow cytometry. In a specific embodiments, the OCT-4⁻, SOX-2⁻ amnion derived adherent cells are additionally HLA-G⁻ or CD271⁻, as determinable by immunolocalization, *e.g.*, flow cytometry. In a more specific embodiment, said cells are OCT-4⁻ and SOX-2⁻, as determinable by RT-PCR; and CD90⁺, CD105⁺, CD117⁻, CD271⁻ and HLA-G⁻, as determinable by immunolocalization, *e.g.*, flow cytometry.

In another embodiment of, and in addition to, any of the above AMDACs, said cell is adherent to tissue culture plastic, and positive for CD309 (also known as VEGFR2/KDR⁺).

The amnion derived adherent cells useful in the methods of treatment disclosed herein, in another embodiment, are adherent to tissue culture plastic, are OCT-4⁻, as determinable by RT-PCR at, *e.g.,* >20 cycles, such as 20-30 cycles, and are one or more of VEGFR2/KDR⁺, CD9⁺, CD54⁺, CD105⁺, CD200⁺, or VE-cadherin⁻, as determinable by immunolocalization, *e.g.*, flow cytometry. In a specific embodiment, said cells are OCT-4⁻, as determinable by RT-PCR at, *e.g.,* >20 cycles, *e.g.,* 20-30 cycles, and VEGFR2/KDR⁺, CD9⁺, CD54⁺, CD105⁺, CD200⁺, and VE-cadherin⁻, as determinable by immunolocalization, *e.g.,* flow cytometry. In another specific embodiment, the cells do not express CD34, *e.g.*, as detected by immunolocalization, *e.g.*, flow cytometry, after exposure to 1 to 100 ng/mL VEGF for 4 to 21 days.

In another embodiment, the amnion derived adherent cells are OCT-4⁻, CD49f⁺, HLA-G⁻, CD90⁺, CD105⁺, and CD117⁻. In a more specific embodiment, said cells are one or more of CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻ (melanoma cell adhesion molecule), or CXCR4⁻, as determinable by immunolocalization, *e.g.*, flow cytometry. In a more specific embodiment, said cells are CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻, and CXCR4- as determinable by immunolocalization, *e.g.*, flow cytometry. In another specific embodiment, said cells are VEGFR1/Flt-1⁺ and/or VEGFR2/KDR⁺, as determinable by immunolocalization, *e.g.*, flow cytometry; and one or more of CD31⁻, CD34⁻, CD45⁻, CD133⁻, and/or Tie-2⁺ as determinable by immunolocalization, *e.g.*, flow cytometry. In another specific embodiment, said cell is additionally VEGFR1/Flt-1⁺, VEGFR2/KDR⁺, CD31⁻, CD34⁻, CD45⁻, CD133⁻, and Tie-2⁺ as determinable by immunolocalization, *e.g.*, flow cytometry.

In another embodiment, the amnion derived adherent cells disclosed herein do not express mRNA for one or more of ANGPT4, ANGPTL3, BGLAP, CD31, CD34, CDH5, CXCL10, DLX5, FGA, FGF4, FLT3, HLA-G, IFNG, LECT1, LEP, MMP-13, NANOG, Nestin, PLG, POU5F1, PRL, PROK1, SOX2, TERT, TNMD, and/or XLKD1 as determinable by RT-PCR, *e.g.,* for 30 cycles. In another embodiment, the amnion derived adherent cells provided herein do not constitutively express one or more of invariant chain, HLA-DR-DP-DQ, CD6, or CD271, as determinable by flow cytometry, *i*.*e*., the amnion derived adherent cells do not express these markers under normal, unstimulated conditions.

In a specific embodiment, the AMDACs described herein are telomerase⁻, as measured by, *e.g.,* RT-PCR and/or telomeric repeat amplification protocol (TRAP) assays. In another specific embodiment, the AMDACs described herein do not express mRNA for telomerase reverse transcriptase (TERT) as determinable by RT-PCR, *e.g.,* for 30 cycles. In another specific embodiment, the AMDACs described herein are NANOG-, as measured by RT-PCR. In another specific embodiment, the AMDACs described herein do not express mRNA for NANOG as determinable by RT-PCR, *e.g.,* for 30 cycles. In a specific embodiment, the AMDACs described herein are (sex determining region Y)-box 2 (SOX2)⁻. In another specific embodiment, the AMDACs described herein do not express mRNA for SOX2 as determinable by RT-PCR, *e.g.,* for 30 cycles.

Further provided herein is an isolated population of cells comprising amnion derived adherent cells, wherein the population of cells is therapeutically effective in the methods of treatment disclosed herein. Such populations of cells can comprise any of the amnion derived adherent cells, described by any of the combinations of markers, as disclosed herein. In specific embodiments, at least about 50%, 60%, 70%, 80%, 90%, 95%, 98% or 99% of cells in said population are such amnion derived adherent cells. In other specific embodiments, at least 25%, 35%, 45%, 50%, 60%, 75%, 85% or more of the cells in the isolated population of cells comprising amnion derived adherent cells are not OCT-4⁺.

In certain embodiments, the methods of treatment provided herein comprise additionally administering a second type of stem cell to said individual. In a specific embodiment, the isolated population of amnion derived adherent cells additionally comprises a second type of cell, *e.g.,* stem cells or progenitor cells. In specific embodiments, the AMDACs disclosed herein comprise at least 10%, 15%, 20%, 25%, 30%, 35%, 40%, 45%, 50%, 55%, 60%, 65%, 70%, 80%, 85%, 90% , 95% or at least 98% of cells in said population. In other specific embodiments, at least 25%, 35%, 45%, 50%, 60%, 75%, 85% or more of the cells in the population of cells comprising amnion derived adherent cells and a second type of cell are not OCT-4⁺. In a specific embodiment, the second type of cells are contained within or isolated from placental blood, umbilical cord blood, crude bone marrow or other tissues. In a more specific embodiment, said second type of cells are embryonic stem cells, stem cells isolated from peripheral blood, stem cells isolated from placental blood, stem cells isolated from placental perfusate, stem cells isolated from placental tissue, stem cells isolated from umbilical cord blood, umbilical cord stem cell *(e.g.,* stem cells from umbilical cord matrix or Wharton's jelly), bone marrow-derived mesenchymal stem cells, mesenchymal stromal cells, hematopoietic stem cells or progenitor cells, *e.g.,* CD34⁺ cells, somatic stem cell, adipose stem cells, induced pluripotent stem cells, or the like. In another more specific embodiment, said second type of cells comprise at least 10%, 15%, 20%, 25%, 30%, 35%, 40%, 45%, or 50% of cells in said population.

In another specific embodiment, any of the above AMDACs, or second type of cells, are, or have been, proliferated in culture. In another specific embodiment, any of the above cells are from a culture of such cells that has been passaged at least 1, 2, 3, 4, 5, 6, 7, 8, 9, 10, 11, 12, 13, 14, 15, 16, 17, 18, 19, or 20 times, or more. In another specific embodiment, any of the above cells are from a culture of such cells that has doubled at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or at least 50 times, or more.

In other embodiments, the methods of treatment disclosed herein comprise administering the AMDACs to an affected individual, in a composition, *e.g.*, a pharmaceutical composition. In specific embodiments, the composition is a matrix or scaffold, *e.g.,* a natural tissue matrix or scaffold, for example, a permanent or degradable decellularized tissue matrix or scaffold; or synthetic matrix or scaffold. In a more specific embodiment, said matrix or scaffold is shaped in the form of a bead, tube or other three-dimensional form. In another more specific embodiment, said matrix is a decellularized tissue matrix. In another specific embodiment, the composition comprises one or more of the isolated amnion derived adherent cells provided herein, or population of cells comprising the amnion derived adherent cells, in a physiologically-acceptable solution, *e.g.*, a saline solution, culture medium or the like.

In another specific embodiment of the methods of treatment provided herein, said cells are administered to said individual by injection. In another specific embodiment, said cells are administered to said individual by intravenous infusion. In another specific embodiment of the method of treatment, said cells are administered to said individual by implantation in said individual of a matrix or scaffold comprising amnion derived adherent cells, as described above.

The isolated amnion derived adherent cells and cell populations provided herein are not the isolated placental stem cells or cell populations described, *e.g.*, in U.S. Patent No. 7,255,879 or U.S. Patent Application Publication No. 2007/0275362, the disclosures of which are hereby incorporated by reference herein in their entireties. The isolated amnion derived adherent cells provided herein are also not endothelial progenitor cells, amniotic epithelial cells, trophoblasts, cytotrophoblasts, embryonic germ cells, embryonic stem cells, cells obtained from the inner cell mass of an embryo, or cells obtained from the gonadal ridge of an embryo.

As used herein, the term "about" means, *e.g.*, within 10% of a stated figure or value.

As used herein, the term "stem cell" defines the functional properties of any given cell population that can proliferate extensively, *e.g.*, up to about 40 population doublings, but not necessarily infinitely, and can differentiate, *e.g.,* can differentiate *in vitro*, into multiple cell types.

As used herein, the term "progenitor cell" defines the functional properties of any given cell population that can proliferate extensively, *e.g.*, up to about 40 population doublings, but not necessarily infinitely, and can differentiate, *e.g.,* can differentiate *in vitro* into a restricted set of cell types, which is generally more restricted in comparison to that of a stem cell.

As used herein, the term "derived" means isolated from or otherwise purified. For example, amnion derived adherent cells are isolated from amnion. The term "derived" encompasses cells that are cultured from cells isolated directly from a tissue, *e.g.,* the amnion, and cells cultured or expanded from primary isolates.

As used herein, "immunolocalization" means the detection of a compound, *e.g.*, a cellular marker, using an immune protein, *e.g.,* an antibody or fragment thereof in, for example, flow cytometry, fluorescence-activated cell sorting, magnetic cell sorting, *in situ* hybridization, immunohistochemistry, or the like.

As used herein, the term "isolated cells" means cells, *e.g.*, AMDACs, that are substantially separated from other cells of the tissue, *e.g.*, amnion, from which the cells are obtained or derived. Cells are "isolated" if at least about 20%, 30%, 40%, 50%, 60%, 70%, 80%, 90%, 95%, or at least about 99% of other cells, with which the cells are naturally associated, are removed from the cells, *e.g.,* during collection and/or culture of the cells.

As used herein, the term "isolated population of cells" means a population of cells that is substantially separated from other cells of the tissue, *e.g.*, amnion or placenta, from which the population of cells is obtained or derived. A population of cells is "isolated" if at least about 20%, 30%, 40%, 50%, 60%, 70%, 80%, 90%, 95%, or at least 99% of the cells with which the population of cells, or cells from which the population of cells is derived, is naturally associated are removed from the cell, *e.g.,* during collection and/or culture of amnion derived adherent cells.

As used herein, a cell or population of cells is "positive" for a particular marker when that marker is detectable above background, *e.g.,* by immunolocalization, *e.g.,* by flow cytometry; or by RT-PCR, *etc.* For example, a cell or cell population is described as positive for, *e.g.,* CD105 if CD105 is detectable on the cell, or in the cell population, in an amount detectably greater than background (in comparison to, *e.g.,* an isotype control). In the context of, *e.g.*, antibody-mediated detection, "positive," as an indication a particular cell surface marker is present, means that the marker is detectable using an antibody, *e.g.*, a fluorescently-labeled antibody, specific for that marker; "positive" also means that a cell or population of cells displays that marker in a amount that produces a signal, *e.g.,* in a cytometer, ELISA, or the like, that is detectably above background. For example, a cell is "CD105⁺" where the cell is detectably labeled with an antibody specific to CD105, and the signal from the antibody is detectably higher than a control (*e.g.*, background). Conversely, "negative" in the same context means that the cell surface marker is not detectable using an antibody specific for that marker compared to background. For example, a cell or population of cells is "CD34⁻" where the cell or population of cells is not detectably labeled with an antibody specific to CD34. Unless otherwise noted herein, cluster of differentiation ("CD") markers are detected using antibodies. For example, OCT-4 can be determined to be present, and a cell is OCT-4⁺, if mRNA for OCT-4 is detectable using RT-PCR, *e.g.,* for 30 cycles.

### 4. BRIEF DESCRIPTION OF THE FIGURES

FIG. 1 shows expression of stem cell-related genes by amnion derived adherent cells and NTERA-2 cells.
FIG. 2 shows the expression of TEM-7 on the cell surface of amnion derived adherent cells (AMDACs).

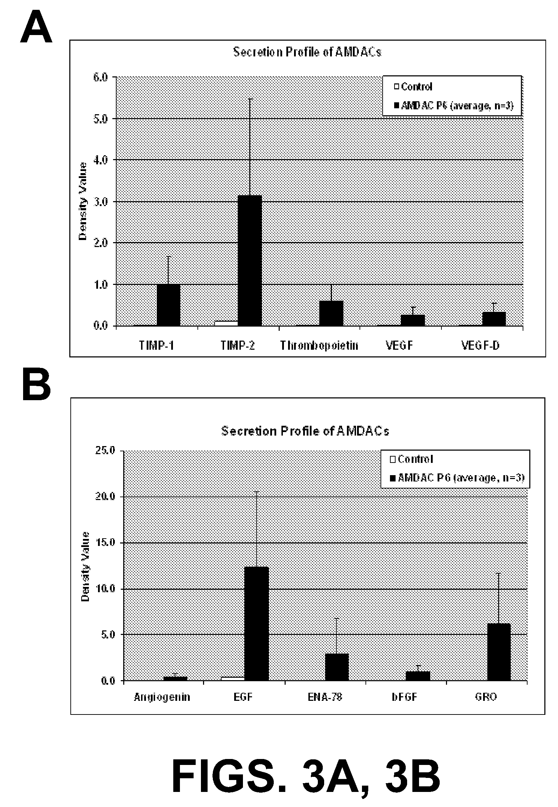

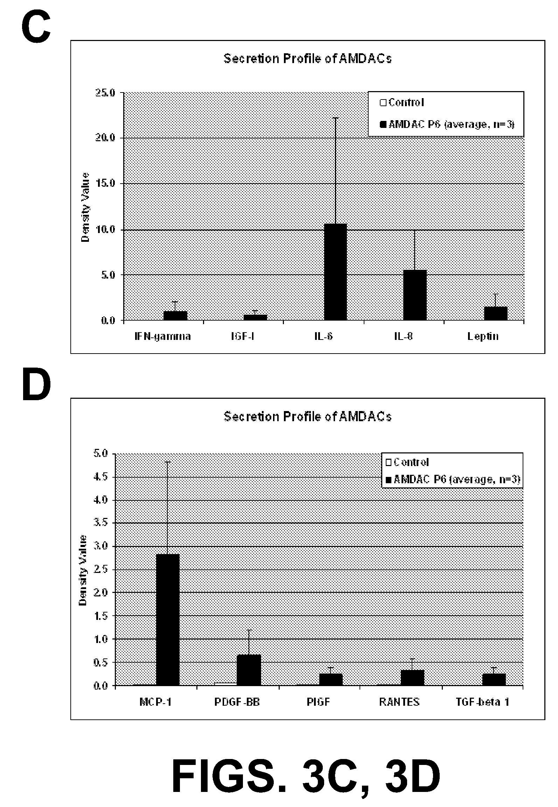

FIGS. 3A-3D show the secretion of selected angiogenic proteins by amnion derived adherent cells. FIG. 3A: Secretion by passage six AMDACs (n = 3) of tissue inhibitor of metalloprotease-1 (TIMP-1), TIMP-2, thrombopoietin, vascular endothelial growth factor (VEGF), and VEGF-D. FIG. 3B: Secretion by passage six AMDACs (n = 3) of angiogenin, epidermal growth factor (EGF), epithelial neutrophil-activating peptide 78 (ENA-78), basic fibroblast growth factor (bFGF), and growth-regulated oncogene alpha (GRO). FIG. 3C: Secretion by passage six AMDACs (n = 3) of interferon gamma (IFN-gamma), insulin-like growth factor-1 (IGF-1), interleukin-6 (IL-6), IL-8, and leptin. FIG. 3D: Secretion by passage six AMDACs (n = 3) of monocyte chemotactic protein-1 (MCP-1), platelet-derived growth factor (PDGF)-BB, placental growth factor (P1GF), rantes, and transforming growth factor-beta (TGF-beta).
FIG. 4 demonstrates the ability of AMDACs to inhibit T cell proliferation in vitro. NHDF: neonatal human dermal fibroblasts. Bars to left for AMDAC, NHDF: CD4+ T cell suppression compared to absence of AMDACs or NHDFs. Bars to right for AMDAC, NHDF: CD8+ T cell suppression compared to absence of AMDACs or NHDFs. Y axis: percent suppression attributable to AMDACs or NHDFs as compared to T cell proliferation in the absence of AMDACs or NHDFs.
FIG. 5 demonstrates that media conditioned by AMDACs induces suppression of TNF-alpha production by T cells. Y axis: percent suppression of production of TNF-α by bulk T cells in the presence of AMDACs or NHDFs as compared to production of TNF-α in the absence of AMDACs or NHDFs.
FIG. 6 shows suppression by AMDACs of Th1 T cells. Pan T base: percent of Th1 T cells in the absence of AMDACs. 100K, 75K, 50K, 25K: percent Th1 T cells in the presence of 100,000, 75,000, 50,000, and 25,000 AMDACs, respectively.
FIG. 7 shows suppression by AMDACs of Th17 T cells in a dose-dependent manner. 100K, 80K, 60K, 40K: percent Th17 T cells (in the absence of AMDACs) remaining after coculture with 100,000, 80,000, 60,000, and 40,000 AMDACs, respectively.
FIG. 8 shows increase of FoxP3 Treg cells by AMDACs. Baseline: percent of FoxP3 Treg cells in total T cells in the absence of AMDACs. 100K, 75K, 50K, 25K: percent FoxP3 Treg cells in the presence of 100,000, 75,000, 50,000, and 25,000 AMDACs, respectively.
FIGS. 9A-9C depict flow cytometry results of DC populations as assessed by CD86 and HLA-DR expression. All: SSC: side scatter gate. Cell type: dendritic cells (DC) alone, or DC + AMDACs. LPS+IFN-γ: cells stimulated (+) or not stimulated (-) with bacterial lipopolysaccharide and interferon gamma. FIG. 9A: DC labeled with anti-CD86-phycoerythrin (PE). FIG. 9B: DC labeled with anti-HLA-DR-PerCP Cy5.5. FIG. 9C: DC labeled with anti-IL-12-PE (Y-axis) and anti-CD11c-FITC.

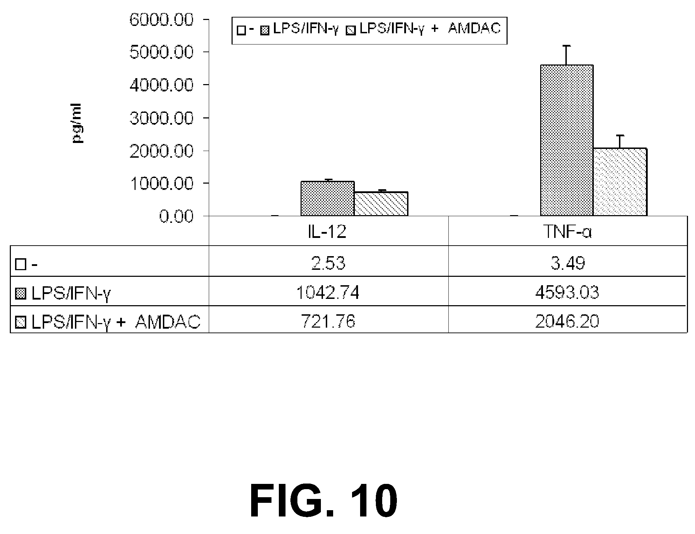

FIG. 10 depicts suppression of production of tumor necrosis factor-alpha (TNF-α) and interleukin-12 (IL-12 by bacterial lipopolysaccharide (LPS)-stimulated dendritic cells (DCs). For each condition (IL-12 or TNF-α production), the left column is the production of the cytokine by DCs in the presence of LPS and interferon-gamma (IFN-γ), and the right column is the production of the cytokine by DCs in the presence of LPS, IFN-γ, and AMDACs. "□ -" indicates condition in which DCs were not stimulated with either LPS or IFN-γ. Numbers to the right of each condition indicate the number of picograms of IL-12 or TNF-α produced by DC in each condition.
FIG. 11 depicts AMDAC-mediated suppression of natural killer (NK) cell proliferation. X axis: number of days of culture of NK cell precursors with (left bars) or without (right bars) AMDACs. Y axis: number of NK cells at each day of culture indicated.
FIG. 12 depicts AMDAC suppression of NK cell cytotoxicity. X axis: number of AMDACs per well in a coculture with NK cells and K562 cells (a human immortalized myelogenous leukaemia cell line) as targets. Y axis: Percent NK cytotoxicity, calculated as (1 - total number of K562 cells in the sample ÷ total K562 cells in a control containing no NK cells) X 100.
FIG. 13 shows that in a mouse multiple sclerosis model, mice receiving AMDACs (G9) showed distinct improvement over mice receiving control vehicle (G1) over days 12-24 of treatment (13A) and that mouse body weights were higher in AMDAC-treated mice over days 12-28 of treatment (13B).
FIG. 14 shows that a mouse multiple sclerosis model, mice receiving AMDACs (G9) developed symptoms later than mice receiving control vehicle (G1) only.

### 5. DETAILED DESCRIPTION

### 5.1 IMMUNOMODULATION USING AMNION DERIVED ADHERENT CELLS

In one aspect, provided herein are methods for the modulation, *e.g.,* suppression, of the activity, *e.g.*, proliferation, of an immune cell, or plurality of immune cells, by contacting the immune cell(s) with a plurality of the amnion derived adherent cells (AMDACs) described herein. The immune cells can be cells *in vitro*, or can be part of an immune response *in vivo.* In certain embodiments, provided herein is a method of suppressing an immune response in an individual, wherein the immune response is, *e.g.,* an autoimmune disease, graft-versus-host disease, asthma or an allergy, comprising contacting a plurality of the individual's immune cells that are part of the immune response with a plurality of amnion derived adherent cells for a time sufficient for said amnion derived adherent cells to detectably suppress the immune response, *e.g.*, wherein said amnion derived adherent cells detectably suppress T cell proliferation in, *e.g.,* a mixed lymphocyte reaction (MLR) assay or a regression assay.

Amnion derived adherent cells useful in modulating an immune response, or the activity of immune cells, are the amnion derived adherent cells described elsewhere herein *(see* Section 5.4). Amnion derived adherent cells used for immunosuppression can be derived or obtained from the amnion of a single placenta or the amnions from multiple placentas. Amnion derived adherent cells used for immunosuppression can also be derived from a single species, *e.g.,* the species of the intended recipient or the species of the immune cells the function of which is to be reduced or suppressed, or can be derived from multiple species.

An "immune cell" in the context of this method, and the methods disclosed herein, means any cell of the immune system, particularly T cells and natural killer (NK) cells. Thus, in various embodiments of the method, amnion derived adherent cells are contacted with a plurality of immune cells, wherein the plurality of immune cells are, or comprises, a plurality of T cells *(e.g.,* a plurality of CD3⁺ T cells, CD4⁺ T cells and/or CD8⁺ T cells) and/or natural killer cells. An "immune response" in the context of the method can be any response by an immune cell to a stimulus normally perceived by an immune cell, *e.g.,* a response to the presence of an antigen. In various embodiments, an immune response can be the proliferation of T cells (*e.g.,* CD3⁺ T cells, CD4⁺ T cells and/or CD8⁺ T cells) as part of an autoimmune disease, graft-versus-host disease, allergy or asthma. The immune response can also be any activity of a natural killer (NK) cell, the maturation of a dendritic cell, or the like. The immune response can also be a local, tissue- or organ-specific, or systemic effect of an activity of one or more classes of immune cells, *e.g.,* the immune response can be inflammation, formation of inflammation-related scar tissue, and the like.

"Contacting" in the context of the methods provided herein encompasses bringing the amnion derived adherent cells and immune cells together in a single container (*e.g.*, culture dish, flask, vial, *etc*.) or *in vivo,* for example, in the same individual *(e.g.,* mammal, for example, human). In a preferred embodiment, the contacting is for a time sufficient, and with a sufficient number of amnion derived adherent cells and immune cells, that a change in an immune function of the immune cells is detectable. More preferably, in various embodiments, said contacting is sufficient to suppress immune function *(e.g.,* T cell proliferation in response to an antigen) by at least 50%, 60%, 70%, 80%, 90% or 95%, compared to the immune function in the absence of the amnion derived adherent cells. Such suppression in an *in vivo* context can be determined in an *in vitro* assay (*see* below); that is, the degree of suppression in the *in vitro* assay can be extrapolated, for a particular number of amnion derived adherent cells and a number of immune cells in a recipient individual, to a degree of suppression in the individual.

In certain embodiments, provided herein are methods of using amnion derived adherent cells to modulate an immune response, or the activity of a plurality of one or more types of immune cells, *in vitro.* Contacting the amnion derived adherent cells and plurality of immune cells can comprise combining the amnion derived adherent cells and immune cells in the same physical space such that at least a portion of the plurality of amnion derived adherent cells interacts with at least a portion of the plurality of immune cells; maintaining the amnion derived adherent cells and immune cells in separate physical spaces with common medium; or can comprise contacting medium from one or a culture of amnion derived adherent cells or immune cells with the other type of cell (for example, obtaining culture medium from a culture of amnion derived adherent cells and resuspending isolated immune cells in the medium). In a specific example, the contacting is performed in a Mixed Lymphocyte Reaction (MLR). In another specific example, the contacting is performed in a regression assay or beat T cell reaction (BTR) assay.

Such contacting can, for example, take place in an experimental setting designed to determine the extent to which a particular plurality of amnion derived adherent cells is immunomodulatory, *e.g.*, immunosuppressive. Such an experimental setting can be, for example, a mixed lymphocyte reaction (MLR) or regression assay. Procedures for performing the MLR and regression assays are well-known in the art. *See, e.g.* Schwarz, "The Mixed Lymphocyte Reaction: An In Vitro Test for Tolerance," J. Exp. Med. 127(5):879-890 (1968); Lacerda et al., "Human Epstein-Barr Virus (EBV)-Specific Cytotoxic T Lymphocytes Home Preferentially to and Induce Selective Regressions of Autologous EBV-Induced B Lymphoproliferations in Xenografted C.B-17 Scid/Scid Mice," J. Exp. Med. 183:1215-1228 (1996). In a preferred embodiment, an MLR is performed in which pluralities of amnion derived adherent cells are contacted with a plurality of immune cells (e.g., lymphocytes, for example, CD3⁺, CD4⁺ and/or CD8⁺ T lymphocytes).

The MLR can be used to determine the immunosuppressive capacity of a plurality of amnion derived adherent cells. For example, a plurality of amnion derived adherent cells can be tested in an MLR comprising combining CD4⁺ or CD8⁺ T cells, dendritic cells (DC) and amnion derived adherent cells in a ratio, *e.g.,* of about 10:1:2, wherein the T cells are stained with a dye such as, *e.g.,* CFSE that partitions into daughter cells, and wherein the T cells are allowed to proliferate for about 6 days. The plurality of amnion derived adherent cells is immunosuppressive if the T cell proliferation at 6 days in the presence of amnion derived adherent cells is detectably reduced compared to T cell proliferation in the presence of DC and absence of amnion derived adherent cells. In such an MLR, amnion derived adherent cells are either thawed or harvested from culture. About 20,000 amnion derived adherent cells are resuspended in 100 µl of medium (RPMI 1640, 1 mM HEPES buffer, antibiotics, and 5% pooled human serum), and allowed to attach to the bottom of a well for 2 hours. CD4⁺ and/or CD8⁺ T cells are isolated from whole peripheral blood mononuclear cells using Miltenyi magnetic beads. The cells are CFSE stained, and a total of 100,000 T cells (CD4⁺ T cells alone, CD8⁺ T cells alone, or equal amounts of CD4⁺ and CD8⁺ T cells) are added per well. The volume in the well is brought to 200µl, and the MLR is allowed to proceed.

In one embodiment, therefore, provided herein is a method of suppressing an immune response comprising contacting a plurality of immune cells with a plurality of amnion derived adherent cells for a time sufficient for said amnion derived adherent cells to detectably suppress T cell proliferation in a mixed lymphocyte reaction (MLR) assay, a bead T cell reaction (BTR) assay or in a regression assay. In one embodiment, said amnion derived adherent cells used in the MLR, BTR or regression assays represent a sample or aliquot of amnion derived adherent cells from a larger population of amnion derived adherent cells.

Populations of amnion derived adherent cells obtained from different placentas, or different tissues within the same placenta, can differ in their ability to modulate an activity of an immune cell, *e.g.,* can differ in their ability to suppress T cell activity or proliferation or NK cell activity. It is thus desirable to determine, prior to use, the capacity of a particular population of amnion derived adherent cells for immunosuppression. Such a capacity can be determined, for example, by testing a sample of the amnion derived adherent cell population in an MLR or regression assay. In one embodiment, an MLR is performed with the sample, and a degree of immunosuppression in the assay attributable to the amnion derived adherent cells is determined. This degree of immunosuppression can then be attributed to the amnion derived adherent cell population that was sampled. Thus, the MLR can be used as a method of determining the absolute and relative ability of a particular population of amnion derived adherent cells to suppress immune function. The parameters of the MLR can be varied to provide more data or to best determine the capacity of a sample of amnion derived adherent cells to immunosuppress. For example, because immunosuppression by amnion derived adherent cells appears to increase roughly in proportion to the number of amnion derived adherent cells present in the assay, the MLR can be performed with, in one embodiment, two or more numbers of amnion derived adherent cells, *e.g.,* 1 x 10³, 3 x 10³, 1 x 10⁴ and/or 3 x 10⁴ amnion derived adherent cells per reaction. The number of amnion derived adherent cells relative to the number of T cells in the assay can also be varied. For example, amnion derived adherent cells and T cells in the assay can be present in any ratio of, *e.g.* about 100:1 to about 1:100, preferably about 1:5, though a relatively greater number of amnion derived adherent cells or T cells can be used.

The regression assay or BTR assay can be used in similar fashion.

Therefore, provided herein are methods of using amnion derived adherent cells to modulate an immune response, or the activity of a plurality of one or more types of immune cells, *in vivo.* Amnion derived adherent cells and immune cells can be contacted, *e.g.,* in an individual that is a recipient of a plurality of amnion derived adherent cells. Where the contacting is performed in an individual, in one embodiment, the contacting is between exogenous amnion derived adherent cells (that is, amnion derived adherent cells not derived from the individual or an amnion associated with the individual; allogeneic cells) and a plurality of immune cells endogenous to the individual. In other embodiments, the amnion derived adherent cells are endogenous to the individual, e.g., sharing genetic identity with the individual, e.g., are autologous to the individual. In specific embodiments, the immune cells within the individual are CD3⁺ T cells, CD4⁺ T cells, CD8⁺ T cells, and/or NK cells.

The amnion derived adherent cells can be administered to the individual in a ratio, with respect to the known or expected number of immune cells, *e.g.,* T cells, in the individual, of from about 10:1 to about 1:10, preferably about 1:5. However, a plurality of amnion derived adherent cells can be administered to an individual in a ratio of, in non-limiting examples, about 10,000:1, about 1,000:1, about 100:1, about 10:1, about 1:1, about 1:10, about 1:100, about 1:1,000 or about 1:10,000. Generally, about 1 x 10⁵ to about 1 x 10⁸ amnion derived adherent cells per recipient kilogram, preferably about 1 x 10⁶ to about 1 x 10⁷ amnion derived adherent cells per recipient kilogram can be administered to effect immunosuppression. In various embodiments, a plurality of amnion derived adherent cells administered to an individual or subject comprises at least, about, or no more than, 1 x 10⁵, 3 x 10⁵, 1 x 10⁶, 3 x 10⁶, 1 x 10⁷ , 3 x10⁷, 1 x 10⁸, 3 x 10⁸, 1 x 10⁹, 3 X 10⁹ amnion derived adherent cells, or more.

The amnion derived adherent cells can also be administered with one or more second types of stem cells, *e.g.*, mesenchymal stem cells from bone marrow, or placental stem cells as described in U.S. Patent No. 7,468,276 or U.S. Patent Application Publication No. 2007/0275362, the disclosures of which are hereby incorporated by reference in their entireties. Such second stem cells can be administered to an individual with amnion derived adherent cells in a ratio of, *e.g.,* about 1:10 to about 10:1.

To facilitate contacting, or proximity of, the amnion derived adherent cells and immune cells *in vivo,* the amnion derived adherent cells can be administered to the individual by any route sufficient to bring the amnion derived adherent cells and immune cells into contact with each other. For example, the amnion derived adherent cells can be administered to the individual, *e.g*., intravenously, intramuscularly, intraperitoneally, intraocularly, parenterally, intrathecally, or directly into an organ, *e.g.,* pancreas. For *in vivo* administration, the amnion derived adherent cells can be formulated as a pharmaceutical composition, as described in Section 5.8.1, below.

The method of immunosuppression can additionally comprise the addition of one or more immunosuppressive agents, particularly in the *in vivo* context. In one embodiment, the plurality of amnion derived adherent cells are contacted with the plurality of immune cells *in vivo* in an individual, and a composition comprising an immunosuppressive agent is administered to the individual. Immunosuppressive agents are well-known in the art and include, e.g., anti-T cell receptor antibodies (monoclonal or polyclonal, or antibody fragments or derivatives thereof), anti-IL-2 receptor antibodies (*e.g.*, Basiliximab (SIMULECT^{®}) or daclizumab (ZENAPAX)^{®}), anti T cell receptor antibodies (*e.g*., Muromonab-CD3), azathioprine, corticosteroids, cyclosporine, tacrolimus, mycophenolate mofetil, sirolimus, calcineurin inhibitors, and the like. In a specific embodiment, the immumosuppressive agent is a neutralizing antibody to macrophage inflammatory protein (MIP)-1α or MIP-1β. Preferably, the anti-MIP-1α or MIP-1β antibody is administered in an amount sufficient to cause a detectable reduction in the amount of MIP-1α and/or MIP-1β in said individual.

Amnion derived adherent cells, in addition to suppression of proliferation of T cells, have other anti-inflammatory effects on cells of the immune system which can be beneficial in the treatment of, *e.g.*, autoimmune disease, graft-versus-host disease, asthma or allergies. For example, amnion derived adherent cells, *e.g., in vitro* or *in vivo,* as when administered to an individual, reduce an immune response mediated by a Th1 and/or a Th17 T cell subset. In another aspect, provided herein is a method of inhibiting a pro-inflammatory response, *e.g.,* a Th1 response or a Th17 response, either *in vivo* or *in vitro*, comprising contacting T cells (*e.g.,* CD4⁺ T lymphocytes or leukocytes) with amnion derived adherent cells, *i*.*e*., the amnion derived adherent cells described herein. In a specific embodiment, said contacting detectably reduces Th1 cell maturation. In a specific embodiment of the method, said contacting detectably reduces the production of one or more of lymphotoxin-1a (LT-1α), interleukin-1β (IL-1β), IL-12, IL-17, IL-21, IL-23, tumor necrosis factor alpha (TNFα) and/or interferon gamma (IFNγ) by said T cells or by an antigen-producing cell. In another specific embodiment of the method, said contacting potentiates or upregulates a regulatory T cell (Treg) phenotype, and/or reduces expression in a dendritic cell (DC) and/or macrophage of biomolecules that promote a Th1 and/or Th17 response (e.g., CD80, CD83, CD86, ICAM-1, HLA-II). In a specific embodiment, said T cells are also contacted with IL-10, *e.g.,* exogenous IL-10 or IL-10 not produced by said T cells, *e.g.*, recombinant IL-10.

In another embodiment, provided herein is a method of reducing the production of pro-inflammatory cytokines from macrophages, comprising contacting the macrophages with an effective amount of amnion derived adherent cells. In another embodiment, provided herein is a method of increasing a number of tolerogenic cells, promoting tolerogenic functions of immune cells, and/or upregulating tolerogenic cytokines, *e.g.*, from macrophages, comprising contacting immune system cells with an effective amount of amnion derived adherent cells. In a specific embodiment, said contacting causes activated macrophages to produce detectably more IL-10 than activated macrophages not contacted with said amnion derived adherent cells. In another embodiment, provided herein is a method of upregulating, or increasing the number of, anti-inflammatory T cells, comprising contacting immune system cells with an effective amount of amnion derived adherent cells.

In one embodiment, provided herein is a method of inhibiting a Th1 response in an individual having, or experiencing a symptom of an immune-related disease or condition, *e.g.,* an autoimmune disease, graft-versus-host disease, asthma or allergy, comprising administering to the individual an effective amount of amnion derived adherent cells, wherein said effective amount is an amount that results in a detectable decrease in a Th1 response in the individual. In another embodiment, provided herein is a method of inhibiting a Th17 response in an individual having, or experiencing a symptom of, an immune-related disease or condition, comprising administering to the individual an effective amount of amnion derived adherent cells, wherein said effective amount is an amount that results in a detectable decrease in a Th17 response in the individual. In specific embodiments of these methods, said administering detectably reduces the production, by T cells or antigen presenting cells in said individual, of one or more of IL-1β, IL-12, IL-17, IL-21, IL-23, TNFα and/or IFNγ. In another specific embodiment of the method, said contacting potentiates or upregulates a regulatory T cell (Treg) phenotype, or modulates production in a dendritic cell (DC) and/or macrophage in said individual of markers the promote a Th1 or Th17 response. In another specific embodiment, the method comprises additionally administering IL-10 to said individual.

In another aspect, provided herein are amnion derived adherent cells, as described herein, that have been genetically engineered to express one or more anti-inflammatory cytokines. In a specific embodiment, said anti-inflammatory cytokines comprise IL-10.

### 5.2 METHODS OF TREATMENT USING AMNION DERIVED ADHERENT CELLS

Provided herein are methods of treating an individual having a disease or disorder, wherein the disease or disorder is caused by, or is associated with, an inappropriate or undesirable immune response, *e.g.,* a disease, disorder or condition that can be treated by immunosuppression, comprising administering to the individual a therapeutically effective amount (that is, number of) amnion derived adherent cells as described herein. In a specific embodiment, the therapeutically effective amount is an amount sufficient to detectably suppress an immune response in the individual. Such an immune response can be, *e.g.,* proliferation of T cells in an MLR or regression assay performed using T cells from the individual. In another specific embodiment, a therapeutically effective amount is an amount of amnion derived adherent cells, the administration of which results in a detectable improvement in, or reduction in the progression of, one or more symptoms of the disease or disorder.

Individuals having a disease or disorder associated with or caused by an inappropriate or undesirable immune response can be treated with a plurality of amnion derived adherent cells, and, optionally, one or more therapeutic agents, at any time during the progression of the disease. In certain embodiments, the individual has, or is at risk of developing, multiple sclerosis; an inflammatory bowel disease, *e.g.*, Crohn's disease or ulcerative colitis; graft-versus-host disease; rheumatoid arthritis; diabetes; psoriasis; mycosis fungoides; or one of the other immune-related diseases or disorders listed herein. For example, the individual can be treated immediately after diagnosis, or within 1, 2, 3, 4, 5, 6 days of diagnosis, or within 1, 2, 3, 4, 5, 6, 7, 8, 9, 10, 15, 20, 25, 30, 35, 40, 45, 50 or more weeks, or 1, 2, 3, 4, 5, 6, 7, 8, 9, 10 or more years after diagnosis. The individual can be treated once, or multiple times during the clinical course of the disease. The individual can be treated, as appropriate, during an acute attack, during remission, or during a chronic degenerative phase.

The amnion derived adherent cells useful in the treatment of such a disease, disorder or condition can be any of the AMDACs disclosed herein. In a specific non-limiting embodiment, the amnion derived adherent cells express CD49f, CD105 and CD200, and do not express OCT-4. In various embodiments, the AMDACs administered to the individual can comprise AMDACs defined by, or characterizable by, any of the combinations of protein or genetic markers described herein.

In one embodiment, the method comprises administering to the individual at least one dose of about 300 million amnion derived adherent cells. The particular dosage, however, can vary according to the individual's physical characteristics, *e.g.*, weight, and can range from 1 million to 10 billion AMDACs per dose, preferably between 10 million and 1 billion AMDACs per dose, or between 100 million and 500 million AMDACs per dose. The administration is preferably intravenous, but can be by any medically-acceptable route for the administration of live cells, *e.g.*, parenterally, subcutaneously, intramuscularly, intraperitoneally, intraocularly, by lumbar puncture, and the like. In one embodiment, the amnion derived adherent cells are from a cell bank. In another embodiment, a dose of amnion derived adherent cells is contained within a blood bag or similar bag, suitable for bolus injection or administration by catheter.

In another embodiment, provided herein is a method of treating an individual having a disease or disorder, wherein the disease or disorder is caused by, or is associated with, an inappropriate or undesirable immune response, *e.g.,* a disease or disorder that can be treated beneficially by immunosuppression, comprising administering to the individual culture medium that has been conditioned by amnion derived adherent cells, in an amount sufficient to detectably suppress an immune response in the individual. Such an immune response can be, *e.g.*, proliferation of T cells in an MLR, BTR or regression assay performed using T cells from the individual.

Amnion derived adherent cells, or medium conditioned by the amnion derived adherent cells, can be administered in a single dose, or in multiple doses. In certain embodiments, *e.g.*, in which the AMDACs are administered in multiple doses, the doses can be part of a therapeutic regimen designed to relieve, *e.g.*, one or more acute symptoms of a disease or disorder, wherein the disease or disorder is caused by, or is associated with, an inappropriate or undesirable immune response. In certain other embodiments, *e.g.*, in which the AMDACs are administered in multiple doses, the doses can be part of a long-term therapeutic regimen designed to prevent, or lessen the severity, of a chronic course of such a disease or disorder.

### 5.2.1 Treatment of Multiple Sclerosis

In another aspect, provided herein is a method of treating an individual having multiple sclerosis, or a symptom associated with multiple sclerosis, comprising administering to the individual a plurality of amnion derived adherent cells, or medium conditioned by amnion derived adherent cells, in an amount and for a time sufficient to detectably modulate, e.g., suppress an immune response in the individual.

Multiple sclerosis (MS) is a chronic, recurrent inflammatory disease of the central nervous system. The disease results in injury to the myelin sheaths surrounding CNS and PNS axons, oligodendrocytes, and the nerve cells themselves. The disease is mediated by autoreactive T cells, particularly CD4⁺ T cells, that proliferate, cross the blood-brain barrier, and enter the CNS under the influence of cellular adhesion molecules and pro-inflammatory cytokines. The symptoms of MS include sensory disturbances in the limbs, optic nerve dysfunction, pyramidal tract dysfunction, bladder dysfunction, bowel dysfunction, sexual dysfunction, ataxia, and diplopia.

Four different types or clinical courses of MS have been identified. The first, relapsing/remitting MS (RRMS) is characterized by self-limiting attacks of neurological dysfunction that manifest acutely, over the course of days to weeks, followed by a period of recovery, sometimes incomplete, over several months. The second type, secondary progressive MS (SPMS), begins as RRMS but changes such that the clinical course becomes characterized by a steady deterioration in function unrelated to acute attacks. The third, primary progressive MS (PPMS), is characterized by a steady decline in function from onset, with no acute attacks. The fourth type, progressive/relapsing MS (PRMS), also begins with a progressive course, with occasional attacks superimposed on the progressive decline in function.

Persons having MS are generally evaluated using a motor skills assessment, optionally with an MRI. For example, one motor skills assessment, the expanded disability status scale, scores gradations in an affected individual's abilities, as follows:
0.0 Normal neurological examination
1.0 No disability, minimal signs in one FS
1.5 No disability, minimal signs in more than one FS
2.0 Minimal disability in one FS
2.5 Mild disability in one FS or minimal disability in two FS
3.0 Moderate disability in one FS, or mild disability in three or four FS. Fully ambulatory.
3.5 Fully ambulatory but with moderate disability in one FS and more than minimal disability in several others
4.0 Fully ambulatory without aid, self-sufficient, up and about some 12 hours a day despite relatively severe disability; able to walk without aid or rest some 500 meters
4.5 Fully ambulatory without aid, up and about much of the day, able to work a full day, may otherwise have some limitation of full activity or require minimal assistance; characterized by relatively severe disability; able to walk without aid or rest some 300 meters.
5.0 Ambulatory without aid or rest for about 200 meters; disability severe enough to impair full daily activities (work a full day without special provisions)
5.5 Ambulatory without aid or rest for about 100 meters; disability severe enough to preclude full daily activities
6.0 Intermittent or unilateral constant assistance (cane, crutch, brace) required to walk about 100 meters with or without resting
6.5 Constant bilateral assistance (canes, crutches, braces) required to walk about 20 meters without resting
7.0 Unable to walk beyond approximately five meters even with aid, essentially restricted to wheelchair; wheels self in standard wheelchair and transfers alone; up and about in wheelchair some 12 hours a day
7.5 Unable to take more than a few steps; restricted to wheelchair; may need aid in transfer; wheels self but cannot carry on in standard wheelchair a full day; May require motorized wheelchair
8.0 Essentially restricted to bed or chair or perambulated in wheelchair, but may be out of bed itself much of the day; retains many self-care functions; generally has effective use of arms
8.5 Essentially restricted to bed much of day; has some effective use of arms retains some self care functions
9.0 Confined to bed; can still communicate and eat.
9.5 Totally helpless bed patient; unable to communicate effectively or eat/swallow 10.0 Death due to MS

In the above scoring system, "FS" refers to the eight functional systems measured, including pyramidal, cerebellar, brainstem, sensory, bowel and bladder, visual, cerebral, and other systems.

Other, similar scoring systems are known, including the Scripps neurological rating scale, the ambulatory index, and the multiple sclerosis functional composite score (MSFC). The progress of MS has also been assessed by a determination of the attack rate. The progress of MS has also been assessed by magnetic resonance imaging, which can detect neural lesions associated with MS *(e.g.,* new lesions, enhancing lesions, or combined unique active lesions).

Thus, in one embodiment, provided herein is a method of treating an individual having MS, *e.g.,* an individual who has been diagnosed with MS, comprising administering to the individual a plurality of amnion derived adherent cells sufficient to detectably suppress an immune response in the individual. In a specific embodiment, the MS is relapsing/remitting MS. In another specific embodiment, the MS is secondary progressive MS. In another specific embodiment, the MS is primary progressive MS. In another specific embodiment, the MS is progressive/relapsing MS. In another specific embodiment, the administering detectably improves one or more symptoms of MS in the individual. In more specific embodiments, the symptom is, *e.g.,* one or more of a sensory disturbance in the limbs, an optic nerve dysfunction, a pyramidal tract dysfunction, a bladder dysfunction, a bowel dysfunction, a sexual dysfunction, ataxia, or diplopia. In another specific embodiment, said administering results in an improvement on the EDSS scale of at least one half point. In another specific embodiment, said administering results in the maintenance of function, according to at least one MS scoring system, over the course of, *e.g.,* 1, 2, 3, 4, 5, 6, 7, 8, 9, 10, or 11 months. In another specific embodiment, said administering results in an improvement on the EDSS scale of at least one point. In another specific embodiment, said administering results in an improvement on the EDSS scale of at least two points. In other specific embodiments, said administering results in a detectable improvement on a multiple sclerosis assessment scale or on an MRI. The individual can be treated, as appropriate, during an acute attack, during remission, or during a chronic degenerative phase. In another embodiment, the AMDACs are administered to a female having MS, post-partum, to maintain the state of remission or reduced occurrence of relapse experienced during pregnancy.

Also provided herein are methods for the treatment of an individual having MS, *e.g.,* an individual who has been diagnosed as having MS, comprising administering to the individual a plurality of amnion derived adherent cells sufficient to detectably suppress an immune response in the individual, wherein the administering detectably improves one or more symptoms of MS in the individual, and additionally one or more second therapeutic agents. In one embodiment, the second therapeutic agent is one or more of Rituximab, Alenituzumab, Cladribine, Daclizumab, Natalizumab, Tysabri, or monocycline (minocycline). In other embodiments, the second therapeutic agent is a glucocorticoid. In specific embodiments, the glucocorticoid is adrenocorticotropic hormone (ACTH), methylprednisolone, or dexamethasone. In another embodiment, the second therapeutic agent is an immunomodulatory or immunosuppressive agent. In various specific embodiments, the immunomodulatory or immunosuppressive agent is IFNβ-1a, IFN-1b, gliatriamer acetate, cyclophosphamide, methotrexate, azathioprine, cladribine, cyclosporine or mitoxantrone. In other embodiments, the therapeutic agent is intravenous immunoglobulin, plasma exchange, or sulfasalazine. In another embodiment, the individual is administered any combination of the foregoing second therapeutic agents in addition to the AMDACs.

### 5.2.2 Treatment of Inflammatory Bowel Disease

In another aspect, amnion derived adherent cells are used to treat an individual having, or at risk of developing, inflammatory bowel disease (IBD), *e.g.,* Crohn's disease or ulcerative colitis. Thus, provided herein is a method of treating an individual having inflammatory bowel disease, or a symptom associated with inflammatory bowel disease, comprising administering to the individual a plurality of amnion derived adherent cells, or medium conditioned by amnion derived adherent cells, in an amount and for a time sufficient to detectably modulate, *e.g.*, suppress an immune response in the individual.

*Crohn's Disease.* In one embodiment, the IBD is Crohn's disease, sometimes referred to as ileitis or enteritis. Crohn's disease is a chronic disorder that causes inflammation of the digestive tract (also referred to as the gastrointestinal, or GI, tract). Crohn's disease can affect any part of the GI tract, from mouth to anus, but most commonly affects the lower part of the small intestine, referred to as the ileum. Five types of Crohn's disease are known. Gastroduodenal Crohn's disease affects the stomach and duodenum (the highest portion of the small intestine). Jejunoileitis is Crohn's disease of the jejunum, the longest portion of the small intestine. Ileitis is Crohn's disease of the ileum, the lower portion of the small intestine. Ileocolitis, the most common form of Crohn's disease, affects the ileum and colon. Finally, Crohn's colitis (Granulomatous colitis) affects the colon, and is distinguished from ulcerative colitis in that in Crohn's colitis, there are often areas of healthy tissue between areas of diseased tissue, and Crohn's colitis can involve only the colon, without involving the rectum. Crohn's disease is thought to arise from inappropriate reaction of the body's immune system to antigens in the GI tract, including, *e.g.*, food, beneficial bacteria, *etc*., resulting in an accumulation of white blood cells in the lining of the intestines. Inflammation associated with Crohn's disease has also been attributed to the action of the cytokine tumor necrosis factor (TNF-α).

*Ulcerative colitis.* In another embodiment, the IBD is ulcerative colitis. Ulcerative colitis is a disease that causes inflammation and sores (ulcers)in the lining of the rectum and/or colon. Ulcers form where inflammation has killed the cells that usually line the colon; the ulcers typically subsequently bleed and produce pus. When inflammation occurs in the rectum and lower part of the colon, the disease is referred to as ulcerative proctitis. If the entire colon is affected, the disease is called pancolitis. If only the left side of the colon is affected, the disease is referred to as limited or distal colitis. Symptoms of ulcerative colitis include, but are not limited to, abdominal pain, bloody diarrhea, fevers, nausea, abdominal cramps, anemia, fatigue, weight loss, loss of appetite, rectal bleeding, loss of bodily fluids and nutrients, skin lesions, joint pain, and growth failure (in children). Ulcerative colitis can also cause complications such as inflammation of the eye, liver disease, and osteoporosis.

Thus, in one embodiment, provided herein is a method of treating an individual having an inflammatory bowel disease, comprising administering a therapeutically effective amount of amnion derived adherent cells to said individual, wherein said therapeutically effective amount is an amount that results in a detectable improvement in at least one symptom of said inflammatory bowel disease (IBD). In a specific embodiment, the IBD is Crohn's disease. In a more specific embodiment, said Crohn's disease is gastroduodenal Crohn's disease, jejunoileitis, ileitis, ileocolitis, or Crohn's colitis. In another specific embodiment, the Crohn's disease is fistulizing Crohn's. In another more specific embodiment, said symptom is a symptom of Crohn's disease. In a more specific embodiment, said symptom of Crohn's disease is inflammation and swelling of a part of the GI tract, abdominal pain, frequent emptying of the bowel, and/or diarrhea. In another more specific embodiment, said symptom of Crohn's disease is rectal bleeding, anemia, weight loss, arthritis, skin problems, fever, thickening of the intestinal wall, formation of scar tissue in the intestines, formation of sores or ulcers in the intestine, development of one or more fistulas in the intestinal wall, development of one or more fissures in the anus, development of nutritional deficiencies (*e.g.*, deficiencies in one or more of proteins, calories, vitamins), development of kidney stones, development of gallstones, or diseases of the liver or biliary system.

In another more specific embodiment, the IBD is ulcerative colitis. In a more specific embodiment, said ulcerative colitis is ulcerative proctitis, pancolitis, limited colitis or distal colitis. In another more specific embodiment, said symptom is a symptom of ulcerative colitis. In a more specific embodiment, said symptom is abdominal pain, bloody diarrhea, fevers, nausea, abdominal cramps, anemia, fatigue, weight loss, loss of appetite, rectal bleeding, loss of bodily fluids and nutrients, skin lesions, joint pain, and growth failure. In another more specific embodiment, the symptom is osteoporosis, eye inflammation, or liver disease.

In certain embodiments, the amnion derived adherent cells are genetically engineered to express one or more therapeutic proteins. In specific embodiments, said one or more therapeutic proteins are IL-10, hepatocyte growth factor (HGF), vascular endothelial growth factor (VEGF) and/or fibroblast growth factor (FGF).

In another specific embodiment, said individual to whom the amnion derived adherent cells are administered is additionally administered one or more of a second therapeutic agent, wherein said second therapeutic agent comprises, *e.g.*, an anti-inflammatory agent, steroid, immune suppressor, and/or an antibiotic. Examples of anti-inflammatory drugs useful in the treatment of, *e.g.,* Crohn's disease or ulcerative colitis include, but are not limited to, mesalamine, 5-ASA (5-aminosalicylic acid) agents (*e.g.*, ASACOL® (mesalamine, delayed-release), DIPENTUM (Osalazine), PENTASA® (mesalamine controlled-release)), sulfasalazine (a combination of 5-ASA and sulfapyridine), anti-inflammatory antibodies (*e.g.*, Infliximab (REMICADE®)), and the like. Examples of steroids useful in the treatment of Crohn's disease or ulcerative colitis include, but are not limited to, cortisone, hydrocortisone, predisone, methylprednisone, and the like. Typically, as practiced in the art, the dosage of steroid is first delivered in a relatively large dose, followed by smaller dosages as inflammation subsides. Examples of immune suppressors useful in the treatment of Crohn's disease include, but are not limited to, cyclosporine A, 6-mercaptopurine or azathioprine. Any antibiotic can be used in the treatment of Crohn's disease, including, *e.g.*, ampicillin, sulfonamide, cephalosporin, tetracycline, and/or metronidazole. In another specific embodiment, the second therapy is an administration of porcine whipworms, *e.g.,* ova of *Trichuris suis.* In other specific embodiments, the second therapy is one or more of an inhibitor of TNFα (*e.g.*, Inflizimab, Adalimurmab, Certolizumab), an inhibitor of IL-6 (*e.g.*, Atilizumab), an antibody that binds to integrin α4 (*e.g.,* Natalizumab), an interferon gamma inhibitor (*e.g.,* Fonolizumab), an antibody that binds to CD4+ T cells and Th2 T-helper subsets (*e.g.*, Visilizumab), Alicaforsen (an antisense inhibitor of ICAM-1), an anti-IL-12Ab antibody, an antibody that binds to integrin α4β7 (*e.g.,* MLN02, a laboratory-produced antibody), and/or recombinant GM-CSF (*e.g.*, Sargramostim, LEUKINE®).

In certain embodiments, the individual having inflammatory bowel disease, *e.g.*, Crohn's disease or ulcerative colitis, is not refractory to any other therapy for the treatment of IBD. In another embodiment, the individual having inflammatory bowel disease, *e.g.*, Crohn's disease or ulcerative colitis, is refractory to at least one other therapy for the treatment of IBD. In a specific embodiment, the individual refractory to at least one other therapy for the treatment of IBD, *e.g.,* Crohn's disease or ulcerative colitis, is refractory to steroid therapy, *e.g.*, corticosteroid therapy.

In certain embodiments, the inflammatory bowel disease, *e.g.*, Crohn's disease or ulcerative colitis, is associated with, or is in part caused by, a detectable increase in the number of immune cells, *e.g.*, activated T cells, lymphocytes and/or macrophages. In specific embodiments, the activated lymphocytes are Th17 and/or Th1 T cells. In certain other embodiments, the inflammatory bowel disease, *e.g.*, Crohn's disease or ulcerative colitis, is associated with, or is in part caused by, a detectable increase in the blood serum concentration of IL-2, IL-12, IL-23, TNFα and/or IFNγ. As such, provided herein is a method of treating an individual having inflammatory bowel disease, *e.g.*, Crohn's disease or ulcerative colitis, comprising (1) determining a number of activated T cells, lymphocytes and/or macrophages in said individual; and (2) administering a therapeutically effective amount of amnion derived adherent cells (that is, a number of cells), as described herein, to the individual, wherein said therapeutically effective amount is an amount that detectably reduces the number of activated T cells, lymphocytes and/or macrophages in said individual as compared to prior to said administering. In another embodiment, provided herein is a method of treating an individual having inflammatory bowel disease, *e.g.*, Crohn's disease or ulcerative colitis, comprising (1) determining the concentration of IL-2, IL-12, IL-23, TNFα and/or IFNγ in a tissue, *e.g.*, blood or serum, from said individual; and (2) administering a therapeutically effective amount (that is, a number of cells) of amnion derived adherent cells, as described herein, to the individual, wherein said therapeutically effective amount is an amount that detectably reduces the concentration of IL-2, IL-12, IL-23, TNFα and/or IFNγ in said tissue from said individual as compared to prior to said administering. In another embodiment, provided herein is a method of treating an individual having inflammatory bowel disease, *e.g.*, Crohn's disease, comprising (1) determining an amount of IL-2, IL-12, IL-23, TNFα and/or IFNγ produced by T cells, lymphocytes, antigen-producing cells, and/or macrophages; and (2) administering an effective amount of amnion derived adherent cells, as described herein, to the individual, wherein said effective amount is an amount that detectably reduces an amount of IL-2, IL-12, IL-23, TNFα and/or IFNγ produced by T cells, lymphocytes, antigen-producing cells, and/or macrophages in said individual as compared to prior to said administering. In another embodiment, provided herein is a method of treating an individual having inflammatory bowel disease, *e.g.*, Crohn's disease, comprising (1) determining a number of Th1 and/or Th17 T cells in a sample from said individual; and (2) administering an effective amount of amnion derived adherent cells, as described herein, to the individual, wherein said effective amount is an amount that detectably reduces the number of Th1 and/or Th17 cells as compared to the number prior to said administering.

In certain other embodiments, in which the inflammatory bowel disease is ulcerative colitis, the disease is characterized predominantly by activated lymphocytes and neutrophils, and the activated T cell subset is Th17 (I1-23) and Th2 (IL-4, IL-5 and IL-13). Thus, provided herein is a method of treating an individual having ulcerative colitis, comprising (1) determining concentration of IL-4, IL-5, IL-13 and/or IL-23 in a tissue, *e.g.*, serum, from said individual; and (2) administering a therapeutically effective amount of amnion derived adherent cells, as described herein, to the individual, wherein said therapeutically effective amount is an amount that detectably reduces the concentration of IL-23 in said tissue from said individual as compared to prior to said administering.

Administration of AMDACs for the treatment of IBD, *e.g.,* Crohn's disease or ulcerative colitis, can be by any medically acceptable route described herein. In certain embodiments, in which the IBD is fistulizing Crohn's, the AMDACs may be administered locally, *e.g.*, directly into or adjacent to one or more fistulas.

### 5.2.3 Treatment of Graft Versus Host Disease

In another embodiment, provided herein is a method of treating an individual, *e.g.*, a transplant recipient or individual who will receive a transplant, that has, or is experiencing a symptom of, or is at risk for developing, graft-versus-host disease (GVHD), comprising administering to the individual a therapeutically effective amount of amnion derived adherent cells (that is, a number of cells), or culture medium conditioned by amnion derived adherent cells, wherein the therapeutically effective amount is an amount sufficient to cause a detectable improvement in one or more symptoms of GVHD, or sufficient to detectably reduce the onset of one or more symptoms of GVHD.

GVHD typically develops after, or as the result of, fully- or partially-allogeneic tissue transplantation, particularly after allogeneic hematopoietic stem cell transplantation, and can include one or more of dermatitis, enteritis and hepatitis that develops typically within 5-100 days of transplantation. GVHD can be acute or chronic. Acute GVHD may be characterized by the appearance of a pruritic or painful rash, typically by day 5 to 47 after transplantation. Hyperacute GVHD may also be accompanied by fever, generalized erythroderma, and desquamation. The liver may also become involved, as evidenced by raised (*e.g.*, higher than normal) levels of bilirubin, alanine aminotransferase (ALT), aspartate aminotransferase (AST), and alkaline phosphatase (AP). Acute GVHD can also involve the colon, resulting in diarrhea, internal bleeding, cramping, abdominal pain, and ileus. Chronic GVHD can occur in transplant patients who have experienced acute GVHD, or who were previously asymptomatic. Manifestations of chronic GVHD include a burning sensation in the eye, eye irritation, photophobia, and eye pain due to decreased tear secretion; dryness of the mouth, sensitivity to spicy or acidic foods, abdominal pain, dysphagia (difficulty in swallowing), odynophagia (pain on swallowing), weight loss, obstructive lung disease, muscular weakness, neuropathic pain, and/or muscle cramps.

Therefore, in specific embodiments of the method, the therapeutically effective amount of amnion derived adherent cells is an amount sufficient to cause a detectable improvement in one or more symptoms of acute GVHD, or sufficient to detectably reduce the onset of one or more symptoms of acute GVHD. In more specific embodiments, said one or more symptoms comprise dermatitis, pruritic skin, rash, enteritis, hepatitis, fever, erythroderma, desquamation, a raised level of ALT, a raised level of AST, a raised level of AP; a raised level of Bilirubin; abdominal pain, cramping, internal bleeding, or ileus. In another specific embodiment of the method, the therapeutically effective amount of amnion derived adherent cells is an amount sufficient to cause a detectable improvement in one or more symptoms of chronic GVHD, or sufficient to detectably reduce the onset of one or more symptoms of chronic GVHD, where said one or more symptoms comprise a burning sensation in the eye, eye irritation, decreased tear production, photophobia, eye pain due to decreased tear secretion dryness of the mouth, sensitivity to spicy or acidic foods, abdominal pain, dysphagia (difficulty in swallowing), odynophagia (pain on swallowing), weight loss, obstructive lung disease (including any of wheezing dyspnea and/or chronic coughing), muscular weakness, neuropathic pain, and/or muscle cramps. In other specific embodiments of the method, symptoms of acute GVHD and/or chronic GVHD comprise hyperbilirubinemia, jaundice, portal hypertension, cirrhosis, hemorrhagic conjunctivitis, psudomembrane formation, lagophthalmos, chronic keratoconjunctivitis, sicca, punctuate keratopathy, atrophy of the oral mucosa, erythema, development of lichenoid lesions of the buccal or labial mucosae, bronchiolitis obliterans, vaginitis, vaginal strictures, autoimmune thrombocytopenia, and/or anemia.

The method is not limited by the nature of the donor or recipient. Transplantation can cross species lines. In preferred embodiments, the donor and recipient are the same species, *e.g.,* are both human. The transplant recipient can be fully- or partially-allogeneic to the donor. The transplantation can be autologous. Transplant recipients or donors can be less than five years of age, from 1 to 10 years of age, from 5 to 15 years of age, from 10 to 20 years of age, from 15 to 25 years of age, from 20 to 30 years of age, from 25 to 35 years of age, from 30 to 40 years of age, from 35 to 45 years of age, from 40 to 50 years of age, from 45 to 55 years of age, from 50 to 60 years of age, from 55 to 65 years of age, from 60 to 70 years of age, or 70 years of age or older.

GVHD is generally graded by severity of symptoms. For example, in one embodiment, symptoms of GVHD are staged, and GVHD is graded from 0 (no GVHD) - IV (life-threatening GVHD) according to skin, liver, and/or intestinal symptoms, as shown in Tables 1 and 2:

**Table 1. Staging of Acute Graft-Versus-Host Disease**

| **Stage** | **Skin** | **Liver (Bilirubin Level, mg/dL)** | **Intestine** |
|---|---|---|---|
| + | Maculopapular rash on <25% of body surface | 2-3 | Diarrhea 500-1000 mL/d or persistent nausea |
| ++ | Maculopapular rash on 25-50% of body surface | 3-6 | Diarrhea 1000-1500 mL/d |
| +++ | Generalized erythroderma | 6-15 | Diarrhea >1500 mL/d |
| ++++ | Desquamation and bullae | >15 | Pain with or without ileus |

**Table 2. Grading of Acute GVHD**

| **Overall Grade** | **Stage** | | | |
|---|---|---|---|---|
| | **Skin** | **Liver** | **Gut** | **Functional Impairment** |
| 0 (None) | 0 | 0 | 0 | 0 |
| I (Mild) | + to ++ | 0 | 0 | 0 |
| II (Moderate) | + to +++ | + | + | + |
| III (Severe) | ++ to +++ | ++ to +++ | ++ to +++ | ++ |
| IV (Life threatening) | ++ to ++++ | ++ to ++++ | ++ to ++++ | +++ |

Thus, in another embodiment of the method, the therapeutically effective amount of amnion derived adherent cells is an amount sufficient to cause an improvement in one or more symptoms of graft-versus-host disease, *e.g.,* in an individual, *e.g.,* an individual that has received a transplant (transplant recipient), such that said graft-versus-host disease is reduced in grade by at least one step. In specific embodiments, said graft-versus-host disease is reduced from grade IV to grade III; from grade IV to grade II; from grade IV to grade I; from grade IV to grade 0; from grade III to grade II; from grade III to grade I; from grade III to grade 0; from grade II to grade I; from grade II to grade 0; or from grade I to grade 0. In another embodiment of the method, the therapeutically effective amount of amnion derived adherent cells is an amount such that graft-versus-host disease in said individual does not develop past grade 0, grade I, grade II or grade II within 10, 20, 30, 40, 50, 60, 70, 80, 90, or 100 days post-transplantation.

In various specific embodiments, the individual having, or who is at risk for developing, GVHD are individuals receiving allogeneic hematopoietic cell transplants (e.g., individuals receiving no GVHD prophylaxis; older individuals; recipients of HLA-nonidentical hematopoietic stem cells; recipients of grafts from allosensitized donors; recipients of grafts from unrelated donors); individuals receiving solid organ transplants, particularly transplants of organs comprising lymphoid tissue, e.g., small bowel transplants; and individuals receiving unirradiated blood products (e.g., neonates and fetuses, individuals having congenital immunodeficiency syndromes, individuals receiving immunosuppressive chemotherapy, individuals receiving directed blood donations from partially HLA-identical, HLA-homologous donors), individuals receiving composite tissue allografts (that is, allografts having more than one tissue type); and the like. GVHD can also occur after autologous or syngenic hematopoietic cell transplantation. In another specific embodiment, the individual has received radiation (e.g., has been irradiated) at a sub-lethal or lethal dose as an adjunct to transplantation.

In specific embodiments, the amnion derived adherent cells are administered to the individual within 14, 13, 12, 11, 10, 9, 8, 7, 6, 5, 4, 3, 2 or 1 day prior to transplantation, *e.g.,* of the tissue that causes or initiates graft-versus-host disease. In another specific embodiment, the amnion derived adherent cells are administered concurrently with transplantation. In another specific embodiment, the amnion derived adherent cells are administered within 1, 2, 3, 4, 5, 6, 7, 8, 9, 10, 11, 12, 13 or 14 days of transplantation. Administration of the amnion derived adherent cells can be performed multiple times, *e.g.,* multiple times before, with or after transplantation, or any combination thereof. In another embodiment, amnion derived adherent cells are administered at any time post-transplantation when graft-versus-host disease of Grade II or worse is manifested in the individual (transplant recipient).

In another embodiment of the method, the individual, *e.g.*, transplant recipient or an individual who will receive a transplant, is administered amnion derived adherent cells and additionally one or more second therapeutic agents. In a specific embodiment, the therapeutic agent is athymocyte globulin, mycophenolate mofetil, sirolimus, Campath-1H, keratinocyte growth factor (KGF), suberoylanilide hydroxamic acid (SAHA), cortisone, hydrocortisone, predisone, or methylprednisone. In another specific embodiment, the therapeutic agent is an immunosuppressive agent or immunomodulatory agent. Immunosuppressive agents and immunomodulatory agents applicable to GVHD are known in the art and include, but are not limited to, methothrexate, leflunomide, cyclophosphamide, cyclosporine A, macrolide antibiotics (*e.g.*, FK506 (tacrolimus)), methylprednisolone (MP), corticosteroids, steroids, mycophenolate mofetil, rapamycin (sirolimus), mizoribine, deoxyspergualin, brequinar, malononitriloamindes (*e.g*., leflunamide), T cell receptor modulators, and cytokine receptor modulators. peptide mimetics, and antibodies (*e.g.*, human, humanized, chimeric, monoclonal, polyclonal, Fvs, ScFvs, Fab or F(ab)₂ fragments or epitope binding fragments), nucleic acid molecules (*e.g*., antisense nucleic acid molecules and triple helices), small molecules, organic compounds, and inorganic compounds. In particular, immunomodulatory agents include, but are not limited to, methothrexate, leflunomide, cyclophosphamide, cytoxan, Immuran, cyclosporine A, minocycline, azathioprine, antibiotics *(e.g.,* FK506 (tacrolimus)), methylprednisolone (MP), corticosteroids, steroids, mycophenolate mofetil, rapamycin (sirolimus), mizoribine, deoxyspergualin, brequinar, malononitriloamindes (*e.g.*, leflunamide), T cell receptor modulators, and cytokine receptor modulators. Examples of T cell receptor modulators include, but are not limited to, anti-T cell receptor antibodies (*e.g.,* anti-CD4 antibodies *(e.g.,* cM-T412 (Boeringer), IDEC-CE9.Is (IDEC and SKB), mAB 4162W94, ORTHOCLONE® and OKTcdr4a (Janssen-Cilag)), anti-CD3 antibodies (*e.g.*, NUVION® (Product Design Labs), OKT3 (Johnson & Johnson), or Rituxan (IDEC)), anti-CD5 antibodies (*e.g.*, an anti-CD5 ricin-linked immunoconjugate), anti-CD7 antibodies (*e.g.*, CHH-380 (Novartis)), anti-CD8 antibodies, anti-CD40 ligand monoclonal antibodies *(e.g.,* IDEC-131 (IDEC)), anti-CD52 antibodies (*e.g.,* CAMPATH® 1H (Ilex)), anti-CD2 antibodies, anti-CD1a antibodies (*e.g.*, Xanelim (Genentech)), and anti-B7 antibodies (*e.g.*, IDEC-114) (IDEC))), CTLA4-immunoglobulin, thalidomide, or one of the compounds in Section 5.6.6, above. In a specific embodiment, a T cell receptor modulator is a CD2 antagonist. In other embodiments, a T cell receptor modulator is not a CD2 antagonist. In another specific embodiment, the agent is antibody MEDI-501 (T10B9). In another specific embodiment, a T cell receptor modulator is a CD2 binding molecule, preferably MEDI-507. In other embodiments, a T cell receptor modulator is not a CD2 binding molecule. Any combination of the above therapeutic agents, suitable for treatment of GVHD or a symptom of GVHD, can be administered. Such therapeutic agents can be administered in any combination with the amnion derived adherent cells, at the same time or as a separate course of treatment.

### 5.2.4 Treatment of Rheumatoid Arthritis

In another embodiment, provided herein is a method of treating an individual that has, or is experiencing a symptom of, or is at risk for developing, rheumatoid arthritis (RA), comprising administering to the individual a therapeutically effective amount of amnion derived adherent cells (that is, a number of cells), or culture medium conditioned by amnion derived adherent cells, wherein the therapeutically effective amount is an amount sufficient to cause a detectable improvement in one or more symptoms of RA, or sufficient to detectably reduce the onset of one or more symptoms of RA. Rheumatoid arthritis is a chronic, inflammatory autoimmune condition in which the body's immune system attacks the joints, and, typically, other tissues of the body.

In a specific embodiment, the administration is sufficient to cause a detectable improvement in one or more symptoms of RA, or sufficient to detectably reduce the onset of one or more symptoms of RA, in at least one joint in the individual with RA. In another specific embodiment, the administration is sufficient to cause a detectable improvement in one or more symptoms of RA, or sufficient to detectably reduce the onset of one or more symptoms of RA, in at least one non-joint tissue in the individual with RA. Examples of non-joint tissue that can be affected by RA include, but are not limited to, skin (dermis), lungs, autoimmune system or blood, renal tissue, cardiovascular tissue, ocular tissue, or neurological tissue.

In specific embodiments, the symptom of RA is, without limitation, morning stiffness (*e.g.*, over an hour in duration), soft-tissue swelling of one or more joints or joint groups, joint pain, subcutaneous nodules, rheumatoid factor present at above 95^{th} percentile, or radiological changes suggestive of joint erosion.

In a specific embodiment, the administration is sufficient to cause a detectable improvement in one or more conditions adjunct to RA, or sufficient to detectably reduce the onset of one or more conditions adjunct to RA. Examples of such conditions include, but are not limited to, pyoderma gangrenosum, neutrophilic dermatosis, Sweet's syndrome, viral infection, erythema nodosum, lobular panniculitis, atrophy of digital skin, palmar erythema, diffuse thinning (rice paper skin), skin fragility, subcutaneous nodules on an exterior surface, *e.g.,* on the elbows, fibrosis of the lungs *(e.g.,* as a consequence of methotrexate therapy), Caplan's nodules, vasculitic disorders, nail fold infarcts, neuropathy, nephropathy, amyloidosis, muscular pseudohypertrophy, endoscarditis, left ventricular failure, valulitis, scleromalacia, mononeuritis multiplex, atlanto-axial subluxation, and the like.

In another embodiment of the method, the individual having RA is administered amnion derived adherent cells and additionally at least one other therapeutic agent. In specific embodiments, the therapeutic agent is, *e.g.,* an analgesic, or an anti-inflammatory agent. In another specific embodiment, the therapeutic agent is a disease-modifying antirheumatic drug (DMARD). In a more specific embodiment, the DMARD is one or more of a xenobiotic (*e.g.*, azathioprine, cyclosporine A, D-pennicillamine, gold salts, hydroxychloroquine, leflunomide, methotrexate, minocycline or sulfasalazine) or a biological agent (*e.g.*, tumor necrosis factor alpha (TNF-α) blockers, such as etanercept (ENBREL®), infliximab (REMICADE®), adalimumab (HUMIRA®), or one of the compounds disclosed in Section 5.6.8, above; interleukin-1 blockers; anti-B cell (CD20) antibody (e.g., rituximab or RITUXAN®); or blockers of T cell activation (*e.g.*, abatacept or ORENCIA®). In another more specific embodiment, the analgesic or anti-inflammatory agent is a glucocorticoid, a non-steroidal anti-inflammatory drug, acetaminophen, ibuprofen, aspirin, an opiate, or lidocaine (topical). Any combination of the above therapeutic agents, suitable for treatment of GVHD or a symptom of GVHD, can be administered. Such therapeutic agents can be administered in any combination with the amnion derived adherent cells, at the same time or as a separate course of treatment.

In a specific embodiment, a plurality of the amnion derived adherent cells administered to an individual having RA have been genetically engineered to express a polypeptide therapeutic for RA. In a more specific embodiment, the polypeptide therapeutic for RA is IL-1Ra (interleukin-1 receptor antagonist). In another more specific embodiment, the polypeptide therapeutic for RA is a fusion protein comprising IL-1Ra and DHFR (dihydrofolate reductase). In a more specific embodiment, the amnion derived adherent cells are transformed with a nucleic acid encoding IL-1Ra-DHFR fusion protein, wherein expression of the fusion protein is enhanced by an antifolate, e.g., methotrexate. In another specific embodiment, a plurality of a second type of stem cell is additionally administered to the individual having RA, wherein the second type of stem cells, or at least a plurality of the second type of stem cells, have been genetically engineered to express a polypeptide therapeutic for RA, *e.g.,* any of the polypeptides disclosed above. In an even more specific embodiment, the nucleic acid encodes IL-lRa-DHFR-IRES-Luc, where IRES is an internal ribosomal entry site, and Luc is luciferase. In another specific embodiment, said nucleic acid comprises a nucleotide sequence that enables control of expression of the IL-1Ra or IL-1Ra-DHFR fusion polypeptide.

Genetically engineered amnion derived adherent cells, or another kind of stem cell, used to treat RA, can be administered to an individual with RA in any combination with such stem cells that have not been genetically modified.

### 5.2.5 Treatment of Scleroderma

In another embodiment, provided herein is a method of treating an individual that has, or is experiencing a symptom of, or is at risk for developing, scleroderma, comprising administering to the individual a therapeutically effective amount of amnion derived adherent cells (that is, a number of cells), or culture medium conditioned by amnion derived adherent cells, wherein the therapeutically effective amount is an amount sufficient to cause a detectable improvement in one or more symptoms of scleroderma, or sufficient to detectably reduce the onset of one or more symptoms of scleroderma.

Scleroderma is a chronic disease characterized by excessive deposits of collagen in the skin or other organs. Scleroderma can be localized or generalized. The localized form of the disease, while disabling, tends not to be fatal. The generalized form of the disease, manifesting as diffuse scleroderma or systemic sclerosis, can be fatal as a result of heart, kidney, lung or intestinal damage. The three types of scleroderma are diffuse scleroderma and limited (CREST syndrome) scleroderma, which are systemic, and morphea/linear scleroderma, which is limited to the skin. Diffuse scleroderma is the most severe form, with victims experiencing rapid onset, widespread skin hardening, and significant internal organ damage, particularly to the lungs and gastrointestinal tract.

The limited form of scleroderma is much milder, exhibiting a slower onset and progression. Skin hardening is usually confined to the hands and face, internal organ involvement is less severe than in the diffuse form. Typically, Raynaud's phenomenon may precede scleroderma by several years. Raynaud's phenomenon is due to vasoconstriction of the small arteries of exposed peripheries - particularly the hands and feet - in the cold, and is classically characterized by a triphasic color change - first white, then blue and finally red on rewarming. The limited form is often referred to as CREST syndrome, where "CREST" is an acronym for the five main features, calcinosis (calcium deposits in soft tissue, *e.g.,* the skin), Raynaud's syndrome, esophageal dysmotility, sclerodactyly (scleroderma of the fingers), and telangiectasia (spider veins).

Development of scleroderma has been correlated with the presence of autoantibodies, particularly anti-centromere and anti-scl70/anti-topoisomerase antibodies. Up to 90% of affected individuals have a detectable anti-nuclear antibody. Anti-centromere antibody is more common in the limited form (80-90%) than in the systemic form (10%), and anti-scl70 is more common in the diffuse form (30-40%) and in African-American patients.

Thus, in the method of treatment provided herein, the administration of amnion derived adherent cells inhibits the development of, reduces the severity of, or reduces the progression of, one or more symptoms of scleroderma. In one embodiment, the scleroderma is limited scleroderma. In another embodiment, the scleroderma is diffuse scleroderma. In another embodiment, the scleroderma is morphea. In another specific embodiment, the symptom is one or more of hardening of the skin of the face, hardening of the skin of the fingers, Reynaud's syndrome, inappropriate vasoconstriction in an extremity, calcinosis, telangiectasia, or esophageal dysmotility. In another specific embodiment, the administration of amnion derived adherent cells detectably reduces the amount or concentration in a milliliter of blood from the individual of one or more anti-nuclear antibodies, e.g., an anti-centromere antibody or an anti-topoisomerase antibody.

In another embodiment, the method of treatment provided herein comprises the administration of a second therapeutic agent, wherein the second therapeutic agent is an anti-inflammatory drug, *e.g.,* a steroidal anti-inflammatory drug, or a non-steroidal anti-inflammatory drug (NSAID), acetaminophen, naproxen, ibuprofen, acetylsalicylic acid, and the like. In a more specific embodiment in which an NSAID is administered, a proton pump inhibitor (PPI), *e.g.*, omeprazole may also administered. In another embodiment, the second therapeutic agent is an immunosuppressant compound such as mycophenolate mofetil, cyclophosphamide or methotrexate. In another embodiment, where the affected individual has digital ulcerations and pulmonary hypertension, a vasodilator such as prostacyclin (iloprost) may be administered.

In another embodiment, the second therapeutic agent is a second type of cell. In certain embodiments, the second type of cell is hematopoietic stem cells, *e.g.,* CD34⁺ hematopoietic stem cells, *e.g.,* in one or more doses of from about 10⁵ cells/kg to about 10⁹ cells/kg. In another specific embodiment, said second type of stem cell is a mesenchymal stem cell, *e.g.,* a bone marrow-derived mesenchymal stem cell. In another specific embodiment, the second type of stem cell is an adipose-derived stem cell. The second type of stem cell, e.g., hematopoietic stem cell, mesenchymal stem cell, adipose stem cell, or the like, can be administered with the amnion derived adherent cells in any ratio, e.g., about 100:1, 75:1, 50:1, 25:1, 20:1, 15:1, 10:1, 5:1, 1:1, 1:5, 1:10, 1:15, 1:20, 1:25, 1:50, 1:75 or 1:100. Stem cells, such as bone marrow-derived mesenchymal stem cells or adipose derived stem cells, can be obtained commercially or from an original source, e.g., bone marrow, bone marrow aspirate, adipose tissue, and the like.

Any combination of the above therapeutic agents, suitable for treatment of scleroderma or a symptom of scleroderma, can be administered. Such therapeutic agents can be administered in any combination with the amnion derived adherent cells, at the same time or as a separate course of treatment.

The amnion derived adherent cells described herein can be administered to the individual suffering scleroderma in the form of a pharmaceutical composition, e.g., a pharmaceutical composition suitable for, e.g., intravenous, intramuscular or intraperitoneal injection.

### 5.2.6 Treatment of Psoriasis

In another embodiment, provided herein is a method of treating an individual that has, or is experiencing a symptom of, or is at risk for developing, psoriasis, comprising administering to the individual a therapeutically effective amount of amnion derived adherent cells (that is, a number of cells), or culture medium conditioned by amnion derived adherent cells, wherein the therapeutically effective amount is an amount sufficient to cause a detectable improvement in one or more symptoms of psoriasis, sufficient to detectably reduce the onset of one or more symptoms of psoriasis, or sufficient to reduce the progress of psoriasis.

Psoriasis is a disease affecting the skin and joints, which commonly causes red scaly patches, called psoriatic plaques, to appear on the skin. Psoriatic patches are areas of inflammation and excessive skin production. Skin rapidly accumulates at these sites and takes a silvery-white appearance. Plaques frequently occur on the skin of the elbows and knees, but can affect any area including the scalp and genitals. Psoriasis is hypothesized to be immune-mediated.

Several different types of psoriasis have been identified. Plaque psoriasis (psoriasis vulgaris), the most common form of psoriasis, typically appears as raised areas of inflamed skin covered with silvery white scaly skin, called plaques. Flexural psoriasis (inverse psoriasis) appears as smooth inflamed patches of skin occurring in skin folds, for example, around the genitals (between the thigh and groin), the armpits, under an overweight stomach, and under the breasts. Guttate psoriasis manifests as numerous small oval (teardrop-shaped) spots that appear over large areas of the body, such as the trunk, limbs, and scalp. Guttate psoriasis is associated with streptococcal throat infection. Pustular psoriasis appears as raised bumps that are filled with non-infectious pus (pustules). Pustular psoriasis can be localized, commonly to the hands and feet (palmoplantar pustulosis), or generalized with widespread patches occurring randomly on any part of the body. Nail psoriasis produces a variety of changes in the appearance of finger and toe nails, including discoloration under the nail plate, pitting of the nails, lines going across the nails, thickening of the skin under the nail, and the loosening (onycholysis) and crumbling of the nail. Psoriatic arthritis involves joint and connective tissue inflammation, *e.g.*, in the joints of the fingers and toes, which can result in a sausage-shaped swelling of the fingers and toes known as dactylitis. Psoriatic arthritis can also affect the hips, knees and spine (spondylitis). Erythrodermic psoriasis manifests as the widespread inflammation and exfoliation of the skin over most of the body surface. It may be accompanied by severe itching, swelling and pain. It is often the result of an exacerbation of unstable plaque psoriasis, particularly following the abrupt withdrawal of systemic treatment. This form of psoriasis can be fatal, as the extreme inflammation and exfoliation disrupt the body's ability to regulate temperature and for the skin to perform barrier functions.

In one embodiment, therefore, the invention provides a method of treatment of an individual having psoriasis, where the psoriasis is one of the psoriasis types listed above, comprising administering to the individual a therapeutically effective dose of amnion derived adherent cells to the individual, wherein said effective dose is an amount of amnion derived adherent (that is, a number of cells) sufficient, *e.g.*, to cause a detectable improvement in, reduce the severity of, or reduce the progression of, one or more of the symptoms of psoriasis listed above.

The severity of psoriasis can be evaluated, *e.g.*, by the Psoriasis Area Severity Index (PASI). PASI combines the assessment of the severity of lesions and the area affected into a single score in the range 0 (no disease) to 72 (maximal disease).

To calculate the PASI, the body is divided into four sections: legs, the body (trunk area (stomach, chest, back, etc.); arms; and head. Each of these areas is scored by itself, and then the four scores are combined into the final PASI. For each section, the percent of area of skin afflicted with psoriasis is estimated and then transformed into a grade from 0 to 6, as follows:

| Percent Area Involved | Grade |
|---|---|
| 0 | 0 |
| <10 | 1 |
| 10-29% | 2 |
| 30-49 | 3 |
| 50-69 | 4 |
| 70-89 | 5 |
| 90-100 | 6 |

The severity is estimated by four different parameters, graded from 0 to 4: itching, erythema (redness), scaling and thickness. The sum of all four severity parameters is then calculated for each section of skin, multiplied by the area score for that area and multiplied by weight of respective section (0.1 for head, 0.2 for arms, 0.3 for body and 0.4 for legs). Example: (I_{bod}y+E_{body}+S_{body}+T_{body}) x A_{body} x 0.3 = Total_{body}. At the end the total PASI is calculated as a sum of PASIs for all four skin sections.

The degree of severity can also be assessed by photographing an individual afflicted with psoriasis, and calculating, by computer, the percent body area covered by psoriatic lesions.

Thus, in specific embodiments of the method of treatment provided herein, the psoriasis is plaque psoriasis (psoriasis vulgaris), flexural psoriasis (inverse psoriasis), guttate psoriasis, pustular psoriasis, nail psoriasis, psoriatic arthritis, or erythodermic psoriasis. In a specific embodiment of the method, the therapeutically effective amount of amnion derived adherent cells, or culture medium conditioned by amnion derived adherent cells is an amount sufficient to cause an improvement in, a delay in onset of, or a lessening of the progression of one or more symptoms of psoriasis, where said one or more symptoms are scaling of the skin, redness of the skin, thickening of the skin, formation of plaques, discoloration under the nail plate, pitting of the nails, lines going across the nails, thickening of the skin under the nail, onycholysis, development of pustules, joint or connective tissue inflammation, inflammation of the skin, or exfoliation of the skin. In another embodiment, the therapeutically effective amount of amnion derived adherent cells, or culture medium conditioned by amnion derived adherent cells is an amount sufficient to cause a 5, 10, 15, 20, 25, 30, 35, 40 or more point reduction in the Psoriasis Area Severity Index.

In another embodiment, the therapeutically effective amount of amnion derived adherent cells, or culture medium conditioned by amnion derived adherent cells, is administered in conjunction with a second therapy. The second therapy can be topical, *e.g.,* creams or ointments comprising one or more of a corticosteroid (e.g., desoximetasine), a vitamin D₃ analog (e.g., calcipotriol), anthralin, argan oil, a retinoid, or coal tar. In another specific embodiment, the second therapy comprises one or more exposures, e.g., for about 1, 2, 3, 4, 5, 6, 7, 8, 9, 10, 12, 14, 16, 18 or 20 minutes, to ultraviolet light, e.g., UVB of a wavelength between about 280 nm to about 315 nm, particularly about 311 nm to about 312 nm. In another specific embodiment, the second therapy comprises topical administration of psoralen in combination with exposure to UVA light. In another specific embodiment, the second therapy comprises one or more systemic administrations of one or more of, *e.g.,* methotrexate, cyclosporine, a retinoid, tioguanine, hydroxyurea, sulfasalazine, mycophenolate mofetil, azathioprine, oral tacrolimus and/or a fumaric acid ester.

### 5.2.7 Treatment of Lupus Erythematosus

In another embodiment, provided herein is a method of treating an individual that has, or is experiencing a symptom of, or is at risk for developing, lupus erythematosus (LE), comprising administering to the individual a therapeutically effective amount of amnion derived adherent cells, or culture medium conditioned by amnion derived adherent cells, wherein the therapeutically effective amount is an amount sufficient to cause a detectable improvement in one or more symptoms of LE, sufficient to detectably reduce the onset of one or more symptoms of LE, or sufficient to reduce the progress of LE.

Symptoms of LE are numerous, and the disease may progress differently in different individuals. Symptoms can include dermatological manifestations (e.g., malar rash (also called butterfly rash), discoid lupus (thick, red scaly patches on the skin), alopecia, mouth, nasal, and vaginal ulcers, and/or lesions on the skin); musculoskeletal manifestations *(e.g.,* joint pain); hematological manifestations (*e.g.,* anemia and iron deficiency, lower than normal platelet and white blood cell counts, antiphospholipid antibody syndrome (a thrombotic disorder in which autoantibodies to phospholipids are present in the patient's serum), and/or presence of anticardiolipin antibody in the blood); cardiac manifestations (*e.g.*, pericarditis, myocarditis, and/or endocarditis); pulmonary manifestations (*e.g.*, lung and/or pleura inflammation, pleuritis, pleural effusion, lupus pneumonitis, chronic diffuse interstitial lung disease, pulmonary hypertension, pulmonary emboli, and/or pulmonary hemorrhage); hepatic manifestations (*e.g.*, autoimmune hepatitis; jaundice; presence of antinuclear antibody (ANA), smooth muscle antibody (SMA), liver/kidney microsomal antibody (LKM-1) and/or anti-mitochondrial antibody (AMA) in the bloodstream); renal manifestations (*e.g.*, painless hematuria or proteinuria, lupus nephritis, renal failure, and/or development of membranous glomerulonephritis with "wire loop" abnormalities); neurological manifestations (*e.g.*, seizures, psychosis, abnormalities in the cerebrospinal fluid); T-cell abnormalities (*e.g.*, deficiency in CD45 phosphatase and/or increased expression of CD40 ligand); and/or nonspecific manifestations (*e.g.*, lupus gastroenteritis, lupus pancreatitis, lupus cystitis, autoimmune inner ear disease, parasympathetic dysfunction, retinal vasculitis, systemic vasculitis, increased expression of FcεRIγ, increased and sustained calcium levels in T cells, increase of inositol triphosphate in the blood, reduction in protein kinase C phosphorylation, reduction in Ras-MAP kinase signaling, and/or a deficiency in protein kinase A I activity.

Thus, in a specific embodiment, said therapeutically effective amount of amnion derived adherent cells, or culture medium conditioned by amnion derived adherent cells is an amount effective to cause a detectable improvement in one or more symptoms of LE, sufficient to detectably reduce the onset of one or more symptoms of LE, or sufficient to reduce the worsening of one or more symptoms of LE, wherein said one or more symptoms comprises one or more dermatological, hematological, musculoskeletal, neurological, renal, hepatic, or T-cell manifestations of LE. In another specific embodiment, said therapeutically effective amount is an amount sufficient to cause a detectable improvement in one or more symptoms of LE, sufficient to detectably reduce the onset of one or more symptoms of LE, or sufficient to reduce the worsening of one or more symptoms of LE, wherein said one or more symptoms comprise malar rash, butterfly rash, discoid lupus, alopecia, mouth, nasal, and vaginal ulcers, lesions on the skin, joint pain anemia and/or iron deficiency, lower than normal platelet and white blood cell counts, antiphospholipid antibody syndrome, presence of anticardiolipin antibody in the blood, pericarditis, myocarditis, endocarditis, lung and/or pleural inflammation, pleuritis, pleural effusion, lupus pneumonitis, chronic diffuse interstitial lung disease, pulmonary hypertension, pulmonary emboli, pulmonary hemorrhage, autoimmune hepatitis; jaundice; presence of antinuclear antibody (ANA), smooth muscle antibody (SMA), liver/kidney microsomal antibody (LKM-1) and/or anti-mitochondrial antibody (AMA) in the bloodstream, painless hematuria or proteinuria, lupus nephritis, renal failure, and/or development of membranous glomerulonephritis with "wire loop" abnormalities); neurological manifestations (*e.g.*, seizures, psychosis, abnormalities in the cerebrospinal fluid); T-cell abnormalities (*e.g.*, deficiency in CD45 phosphatase and/or increased expression of CD40 ligand); and/or nonspecific manifestations (*e.g.*, lupus gastroenteritis, lupus pancreatitis, lupus cystitis, autoimmune inner ear disease, parasympathetic dysfunction, retinal vasculitis, systemic vasculitis, increased expression of FcεRIγ, increased and sustained calcium levels in T cells, increase of inositol triphosphate in the blood, reduction in protein kinase C phosphorylation, reduction in Ras-MAP kinase signaling, and/or a deficiency in protein kinase A I activity.

Amnion derived adherent cells can be administered to the individual suffering scleroderma in the form of a pharmaceutical composition, e.g., a pharmaceutical composition suitable for, *e.g.,* intravenous, intramuscular or intraperitoneal injection.

### 5.2.8 Treatment of Mycosis Fungoides

In another embodiment, provided herein is a method of treating an individual that has, or is experiencing a symptom of, or is at risk for developing, mycosis fungoides, comprising administering to the individual a therapeutically effective amount of amnion derived adherent cells (that is, a number of cells), or culture medium conditioned by amnion derived adherent cells, wherein the therapeutically effective amount is an amount sufficient to cause a detectable improvement in one or more symptoms of mycosis fungoides, sufficient to detectably reduce the onset of one or more symptoms of mycosis fungoides, or sufficient to reduce the progress of mycosis fungoides.

Mycosis fungoides is the most common of the cutaneous T-cell lymphomas, a group of rare cancers that grow in the skin. Sezary syndrome, a more rare form in which the T cells affect the peripheral blood as well as the skin, occurs in about 5% of all cases of mycosis fungoides. Mycosis fungoides generally progresses in stages defined by the skin symptoms: (1) patch phase, in which the skin has flat, red patches, or, in dark-skinned individuals very light or very dark patches, that are very itchy, and may be raised and hard (plaques); (2) skin tumors phase, in which red-violet raised lumps (nodules) appear, which may be dome-shaped (like a mushroom) or ulcerated; (3) skin redness (erythroderma) stage, in which the individual's skin develops large red areas that are very itchy and scaly, and in which skin of the palms and soles may thicken and crack; and (4) lymph node stage, in which mycosis fungoides begins to move to other parts of the body via the lymph nodes, which become inflamed, and often cancerous, and may spread to the liver, lungs, or bone marrow.

Thus in a specific embodiment, the therapeutically effective amount of amnion derived adherent cells, or culture medium conditioned by amnion derived adherent cells is an amount effective to cause a detectable improvement in one or more symptoms of mycosis fungoides, sufficient to detectably reduce the onset of one or more symptoms of mycosis fungoides, or sufficient to reduce the worsening of one or more symptoms of LE, wherein said one or more symptoms comprises one or more dermatological, hematological, musculoskeletal, neurological, renal, hepatic, or T-cell manifestations of mycosis fungoides. In another specific embodiment, said therapeutically effective amount is an amount sufficient to cause a detectable improvement in one or more symptoms of mycosis fungoides, sufficient to detectably reduce the onset of one or more symptoms of mycosis fungoides, or sufficient to reduce the worsening of one or more symptoms of mycosis fungoides, wherein said one or more symptoms comprise itchy light or dark patches on the skin, skin plaques, development of skin tumors, raised bumps on the skin, development of skin areas that are red, scaly and itchy, thickening of the skin of the soles or palms, cracking of the skin of the soles or palms, or inflammation of the lymph nodes.

In another embodiment, the therapeutically effective amount of amnion derived adherent cells, or culture medium conditioned by amnion derived adherent cells, is administered in conjunction with a second therapy or second therapeutic agent. In more specific embodiments, the second therapy or therapeutic agent is one or more of exposure of an affected area of said individual to sunlight or ultraviolet light, topical steroids, local superficial radiotherapy, total skin electron beam radiation, application of organic (Manuka) honey to skin affected by erythorderma, or biological therapies. In a more specific embodiment, said biological therapies comprise administration to the individual of one or more of an interferon, a retinoid, a rexinoid, vorinostat (*e*.g., ZOLINZA®).

### 5.2.9 Treatment of Diabetes

In another embodiment, provided herein is a method of treating an individual that has, or is experiencing a symptom of, or is at risk for developing, diabetes, comprising administering to the individual a therapeutically effective amount of amnion derived adherent cells (that is, a number of cells), or culture medium conditioned by amnion derived adherent cells, wherein the therapeutically effective amount is an amount sufficient to cause a detectable improvement in one or more symptoms of diabetes, sufficient to detectably reduce the onset of one or more symptoms of diabetes, or sufficient to reduce the progress of diabetes. In a specific embodiment, the diabetes is diabetes mellitus Type 1, also known as Type 1 diabetes, Type I diabetes, T1D, or insulin-dependent diabetes mellitus (IDDM).

The amnion derived adherent cells can be administered one or more times during the course of the disease. Preferably, the stem cells are administered within 1, 2, 3, 4, 5, or 6 days, or 1 week, of first diagnosis. In one embodiment, said therapeutically effective amount of the amnion derived adherent cells is an amount sufficient to reverse, reduce the severity of, or otherwise ameliorate a symptom of diabetes mellitus Type 1, including abnormally high blood sugar, lack of insulin resistance as determined by a glucose tolerance test, fatigue, or loss of consciousness.

The amnion derived adherent cells can be administered in conjunction with a second therapy, *e.g.*, transplanted pancreatic tissue and/or islet cells; autologous or allogeneic stem cell therapy, or the like.

### 5.2.10 Treatment of Other Immune-Related Diseases or Disorders

Further provided herein are methods of treating other immune-related diseases or conditions using AMDACs. In other embodiments, for example, provided herein is a method of treating an individual having a disease or disorder, wherein the disease or disorder is caused by, or is associated with, an inappropriate or undesirable immune response, *e.g.,* a disease, disorder or condition that can be treated beneficially by immunosuppression, comprising administering to the individual the amnion derived adherent cells described herein. In various specific embodiments, said immune-related disease is one or more of Addison's disease, alopecia areata, ankylosing spondylitis, antiphospholipid antibody syndrome, antiphospholipid syndrome (primary or secondary), asthma, autoimmune gastritis, autoimmune hemolytic anemia, autoimmune hepatitis, autoimmune inner ear disease, autoimmune lymphoproliferative disease, autoimmune thrombocytopenic purpura, Balo disease, Behcet's disease, bullous pemphigoid, cardiomyopathy, celiac disease, Chagas disease, chronic inflammatory demyelinating polyneuropathy, cicatrical pemphigoid (e.g., mucous membrane pemphigoid), cold agglutinin disease, degos disease, dermatitis hepatiformis, essential mixed cryoglobulinemia, Goodpasture's syndrome, Graves' disease, Guillain-Barre syndrome, Hashimoto's thyroiditis (Hashimoto's disease; autoimmune thyroditis), idiopathic pulmonary fibrosis, idiopathic thrombocytopenia purpura, IgA nephropathy, juvenile arthritis, lichen planus, Ménière disease, mixed connective tissue disease, morephea, myasthenia gravis, narcolepsy, neuromyotonia, pediatric autoimmune neuropsychiatric disorders (PANDAs), pemphigus vulgaris, pernicious anemia, polyarteritis nodosa, polychondritis, polymyalgia rheumatica, primary agammaglobulinemia, primary biliary cirrhosis, Raynaud disease (Raynaud phenomenon), Reiter's syndrome, relapsing polychondritis, rheumatic fever, Sjogren's syndrome, stiff-person syndrome (Moersch-Woltmann syndrome), Takayasu's arteritis, temporal arteritis (giant cell arteritis), uveitis, vasculitis (e.g., vasculitis not associated with lupus erythematosus), vitiligo, and/or Wegener's granulomatosis.

In various other specific embodiments, said autoimmune disease is one or more of inflammatory myositis, dermatomyositis, dermatomyositis (juvenile), juvenile myositis, inclusion body myositis, and/or polymyositis (*e*.g., with dermatomyositis).

### 5.2.11 Second Therapeutic Compositions and Second Therapies

In any of the above methods of treatment, the method can comprise the administration of a second therapeutic composition or second therapy. The recitation of specific second therapeutic compounds or second therapies in the methods of treating specific diseases, above, are not intended to be exclusive. For example, any of the diseases, disorders or conditions discussed herein can be treated with any of the anti-inflammatory compounds or immunosuppressive compounds described herein. In embodiments in which amnion derived adherent cells are administered with a second therapeutic agent, or with a second type of stem cell, the amnion derived adherent cells and second therapeutic agent and/or second type of stem cell can be administered at the same time or different times, *e.g.,* the administrations can take place within 1, 2, 3, 4, 5, 6, 7, 8, 9 10, 20, 30, 40, or 50 minutes of each other, or 1, 2, 3, 4, 5, 6, 7, 8, 9, 10, 12, 14, 16, 18, 20, or 22 hours of each other, or within 1, 2, 3, 4, 5, 6, 7 8, 9 or 10 or more days of each other.

In a specific embodiment, treatment of a disease, disorder or condition related to or caused by an inappropriate, deleterious or harmful immune response comprises administration of a second type of stem cell, or population of a second type of stem cell, in addition to the amnion derived adherent cells. In a specific embodiment, said second type of stem cell is a mesenchymal stem cell, *e.g.,* a bone marrow-derived mesenchymal stem cell. In another embodiment, the second type of stem cell is an adipose-derived stem cell. In other embodiments, the second type of stem cell is a multipotent stem cell, a pluripotent stem cell, a progenitor cell, a hematopoietic stem cell, *e.g.,* a CD34⁺ hematopoietic stem cell, an adult stem cell, an embryonic stem cell or an embryonic germ cell. The second type of stem cell, e.g., mesenchymal stem cell or adipose-derived stem cell, can be administered with the amnion derived adherent cells in any ratio, *e.g.,* a ratio of about 100:1, 75:1, 50:1, 25:1, 20:1, 15:1, 10:1, 5:1, 1:1, 1:5, 1:10, 1:15, 1:20, 1:25, 1:50, 1:75 or 1:100. Mesenchymal stem cells can be obtained commercially or from an original source, e.g., bone marrow, bone marrow aspirate, adipose tissue, and the like.

In another specific embodiment, said second therapy comprises an immunomodulatory compound, wherein the immunomodulatory compound is 3-(4-amino-1-oxo-1,3-dihydroisoindol-2-yl)-piperidine-2,6-dione; 3-(4'aminoisolindoline-1'-onw)-1-piperidine-2,6-dione; 4-(Amino)-2-(2,6-dioxo(3-piperidyl))-isoindoline-1,3-dione; or α-(3-aminophthalimido) glutarimide. In a more specific embodiment, said immunomodulatory compound is a compound having the structure 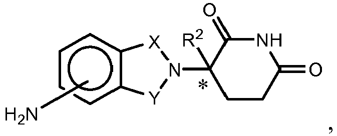 wherein one of X and Y is C=O, the other of X and Y is C=O or CH₂, and R² is hydrogen or lower alkyl, or a pharmaceutically acceptable salt, hydrate, solvate, clathrate, enantiomer, diastereomer, racemate, or mixture of stereoisomers thereof. In another more specific embodiment, said immunomodulatory compound is a compound having the structure 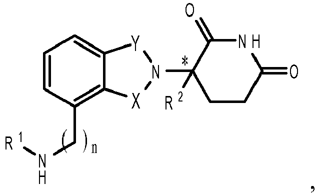 wherein one of X and Y is C=O and the other is CH₂ or C=O;
R¹ is H, (C₁-C₈ )alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(S)R³, C(O)OR⁴, (C₁-C₈)alkyl-N(R⁶)₂, (C₁-C₈)alkyl-OR⁵, (C₁-C₈)alkyl-C(O)OR⁵, C(O)NHR³, C(S)NHR³, C(O)NR³R^{3'}, C(S)NR³R^{3'} or (C₁-C₈)alkyl-O(CO)R⁵;
R² is H, F, benzyl, (C₁-C₈)alkyl, (C₂-C₈)alkenyl, or (C₂-C₈)alkynyl;
R³ and R³ are independently (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, (C₀-C₈)alkyl-N(R⁶)₂, (C₁-C₈)alkyl-OR⁵, (C₁-C₈)alkyl-C(O)OR⁵, (C₁-C₈)alkyl-O(CO)R⁵, or C(O)OR⁵;
R⁴ is (C₁-C₈)alkyl, (C₂-C₈)alkenyl, (C₂-C₈)alkynyl, (C₁-C₄)alkyl-OR⁵, benzyl, aryl, (C₀-C₄)alkyl-(C₁-C₆)heterocycloalkyl, or (C₀-C₄)alkyl-(C₂-C₅)heteroaryl;
R⁵ is (C₁-C₈)alkyl, (C₂-C₈)alkenyl, (C₂-C₈)alkynyl, benzyl, aryl, or (C₂-C₅)heteroaryl;
each occurrence of R⁶ is independently H, (C₁-C₈)alkyl, (C₂-C₈)alkenyl, (C₂-C₈)alkynyl, benzyl, aryl, (C₂-C₅)heteroaryl, or (C₀-C₈)alkyl-C(O)O-R⁵ or the R⁶ groups can join to form a heterocycloalkyl group;
n is 0 or 1; and
* represents a chiral-carbon center;
or a pharmaceutically acceptable salt, hydrate, solvate, clathrate, enantiomer, diastereomer, racemate, or mixture of stereoisomers thereof. In another more specific embodiment, said immunomodulatory compound is a compound having the structure 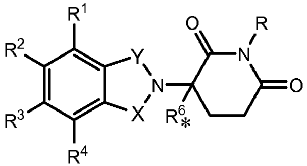 wherein:
one of X and Y is C=O and the other is CH₂ or C=O;
R is H or CH₂OCOR';
   (i) each of R¹, R², R³, or R⁴, independently of the others, is halo, alkyl of 1 to 4 carbon atoms, or alkoxy of 1 to 4 carbon atoms or (ii) one of R¹, R², R³, or R⁴ is nitro or -NHR⁵ and the remaining of R¹, R², R³, or R⁴ are hydrogen;
R⁵ is hydrogen or alkyl of 1 to 8 carbons
R⁶ hydrogen, alkyl of 1 to 8 carbon atoms, benzo, chloro, or fluoro;
R' is R⁷-CHR¹⁰-N(R⁸R⁹);
R⁷ is m-phenylene or p-phenylene or -(CₙH₂ₙ)- in which n has a value of 0 to 4;
each of R⁸ and R⁹ taken independently of the other is hydrogen or alkyl of 1 to 8 carbon atoms, or R⁸ and R⁹ taken together are tetramethylene, pentamethylene, hexamethylene, or -CH₂CH₂X₁CH₂CH₂- in which X₁ is -O-, -S-, or -NH-;
R¹⁰ is hydrogen, alkyl of to 8 carbon atoms, or phenyl; and
* represents a chiral-carbon center;
or a pharmaceutically acceptable salt, hydrate, solvate, clathrate, enantiomer, diastereomer, racemate, or mixture of stereoisomers thereof.

Any combination of the above therapeutic agents can be administered. Such therapeutic agents can be administered in any combination with the amnion derived adherent cells, at the same time or as a separate course of treatment.

Amnion derived adherent cells can be administered, to the individual suffering from an immune-related disease, in the form of a pharmaceutical composition, e.g., a pharmaceutical composition suitable for intravenous, intramuscular or intraperitoneal injection. Amnion derived adherent cells can be administered in a single dose, or in multiple doses. Where amnion derived adherent cells are administered in multiple doses, the doses can be part of a therapeutic regimen designed to relieve one or more acute symptoms of an immune-related disease or disorder, *e.g.,* IBD, *e.g.,* Crohn's disease, of can be part of a long-term therapeutic regimen designed to prevent, or lessen the severity, of, *e.g.,* a chronic course of the disease. In embodiments in which amnion derived adherent cells are administered with a second therapeutic agent, or with a second type of stem cell, the amnion derived adherent cells and second therapeutic agent and/or second type of stem cell can be administered at the same time or different times, *e.g.,* the administrations can take place within 1, 2, 3, 4, 5, 6, 7, 8, 9 10, 20, 30, 40, or 50 minutes of each other, or 1, 2, 3, 4, 5, 6, 7, 8, 9, 10, 12, 14, 16, 18, 20, or 22 hours of each other, or within 1, 2, 3, 4, 5, 6, 7 8, 9 or 10 or more days of each other.

### 5.3 AMNION DERIVED ADHERENT CELLS

The methods provided herein use tissue culture plastic adherent, amnion derived cells, and populations of such cells, referred to herein as "amnion derived adherent cells" or AMDACs. Generally, amnion derived adherent cells superficially resemble fibroblasts or mesenchymal cells in appearance, having a generally fibroblastoid shape. Such cells adhere to a cell culture surface, *e.g.,* to tissue culture plastic. In certain embodiments of any of the AMDACs disclosed herein, the cells are human cells.

AMDACs provided herein display cellular markers that distinguish them from other amnion-derived, or placenta-derived, cells. In certain embodiments of each of the embodiments of AMDACs described herein, the AMDACs are isolated.

In one embodiment, amnion derived adherent cells are OCT-4⁻(octamer binding protein 4), as determinable by RT-PCR. In another specific embodiment, OCT-4⁻ amnion derived adherent cells are CD49f⁺, as determinable, *e.g.,* by immunolocalization, *e.g.,* flow cytometry. In another specific embodiment, said OCT-4⁻ cells are HLA-G⁻, as determinable by RT-PCR. In another specific embodiment, the OCT-4- cells are VEGFR1/Flt-1⁺ (vascular endothelial growth factor receptor 1) and/or VEGFR2/KDR⁺ (vascular endothelial growth factor receptor 2), as determinable by immunolocalization, *e.g**.,*** flow cytometry. In a specific embodiment, OCT-4- amnion derived adherent cells express at least 2 log less PCR-amplified mRNA for OCT-4 at, *e.g.,* 20 cycles, than an equivalent number of NTERA-2 cells and RNA amplification cycles. In another specific embodiment, said OCT-4- cells are CD90⁺, CD105⁺, or CD117⁻ as determinable, *e.g.,* by immunolocalization, *e.g.,* flow cytometry. In a more specific embodiment, said OCT-4- cells are CD90⁺, CD105⁺, and CD117⁻ as determinable, *e.g.,* by immunolocalization, *e.g.,* flow cytometry. In a more specific embodiment, the cells are OCT-4⁻ or HLA-G⁻, and is additionally CD49f⁺, CD90⁺, CD105⁺, and CD117⁻ as determinable, *e.g.,* by immunolocalization, *e.g.,* flow cytometry. In a more specific embodiment, the cells are OCT-4⁻, HLA-G⁻, CD49f⁺, CD90⁺, CD105⁺, and CD117⁻ as determinable, *e.g.,* by immunolocalization, *e.g.,* flow cytometry. In another specific embodiment, the OCT-4- cells do not express SOX2, *e.g.,* as determinable by RT-PCR for 30 cycles. In a specific embodiment, therefore, the amnion derived adherent cells are OCT-4⁻, CD49f⁺, CD90⁺, CD105⁺, and CD117⁻, as determinable by immunolocalization, *e.g.,* flow cytometry, and SOX2⁻, as determinable by RT-PCR, *e.g.,* for 30 cycles.

In a specific embodiment, the AMDACs described herein are GFAP⁺ as determinable by, *e.g.,* a short-term neural differentiation assay (See, *e.g.,* Section 6.3.3, below). In another specific embodiment, the AMDACs described herein are beta-tubulin III (Tuj1)⁺ as determinable by, *e.g.,* a short-term neural differentiation assay. In another specific embodiment, the AMDACs are OCT-4⁻, GFAP⁺, and beta-tubulin III (Tuj 1)⁺. In another specific embodiment, the AMDACs are OCT-4⁻, CD200⁺, CD105⁺, and CD49f⁺. In another specific embodiment, the AMDACs are CD200⁺, CD105⁺, CD90⁺, and CD73⁺. In another specific embodiment, the AMDACs described herein are CD117⁻ and are not selected using an antibody to CD117. In another specific embodiment, the AMDACs described herein are CD 146- and are not selected using an antibody to CD 146. In another specific embodiment, the AMDACs described herein OCT-4⁻, as determinable by RT-PCR and/or immunolocalization, *e.g.,* flow cytometry, and do not express CD34 following induction with VEGF, *e.g.,* as determinable by RT-PCR and/or immunolocalization, *e.g.,* flow cytometry. In another specific embodiment, the AMDACs used in the methods described herein are neurogenic, as determinable by a short-term neural differentiation assay (see, *e.g.,* Section 6.3.3, below). In another specific embodiment, the AMDACs used in the methods described herein are non-chondrogenic as determinable by an in vitro chondrogenic potential assay (see, *e.g.,* Section 6.3.2, below). In another specific embodiment, the AMDACs used in the methods described herein are non-osteogenic as determinable by an osteogenic phenotype assay (see, *e.g.,* Section 6.3.1, below). In another specific embodiment, the AMDACs described herein are non-osteogenic after being cultured for up to 6 weeks *(e.g.,* for 2 weeks, for 4 weeks, or for 6 weeks) in DMEM at pH 7.4 (High glucose) supplemented with 100 nM dexamethasone, 10 mM β-glycerol phosphate, 50 µM L-ascorbic acid-2-phosphate, wherein osteogenesis is assessable using von Kossa staining; alizarin red staining; or detectable by the presence of osteopontin, osteocalcin, osteonectin, and/or bone sialoprotein by, *e.g.,* RT-PCR.

In another embodiment, said OCT-4⁻ cells are one or more of CD29⁺, CD73⁺, ABC-p⁺, and CD38⁻, *e.g.,* as determinable by immunolocalization, *e.g.,* flow cytometry.

In another specific embodiment, for example, OCT-4⁻ AMDACs can additionally be one or more of CD9⁺, CD10⁺, CD44⁺, CD54⁺, CD98⁺, TEM-7⁺ (tumor endothelial marker 7), CD31⁻, CD34⁻, CD45⁻, CD133⁻, CD143⁻ (angiotensin-I-converting enzyme, ACE), CD146- (melanoma cell adhesion molecule), or CXCR4⁻ (chemokine (C-X-C motif) receptor 4), *e.g.,* as determinable by immunolocalization, *e.g.,* flow cytometry, or HLA-G⁻ as determinable by RT-PCR. In a more specific embodiment, said cells are CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻, and CXCR4⁻, *e.g.,* as determinable by immunolocalization, *e.g.,* flow cytometry, and HLA-G⁻ as determinable by RT-PCR. In another embodiment, the amnion derived adherent cells are one or more of CD31⁻, CD34⁻, CD45⁻, and/or CD133⁻, as determinable, *e.g.,* by immunolocalization, *e.g.,* flow cytometry. In a specific embodiment, the amnion derived adherent cells are OCT-4⁻, as determinable by RT-PCR; VEGFR1/Flt-1⁺ and/or VEGFR2/KDR⁺, as determinable by immunolocalization, *e.g.,* flow cytometry; and one or more, or all, of CD31⁻, CD34⁻, CD45⁻, and/or CD133⁻ as determinable, *e.g.,* by immunolocalization, *e.g.,* flow cytometry.

In another specific embodiment, said AMDACs are additionally VE-cadherin⁻ as determinable by immunolocalization, *e.g.,* flow cytometry. In another specific embodiment, said OCT-4- cells are, either alone or in combination with other markers, additionally positive for CD105⁺ and CD200⁺ as determinable by immunolocalization, *e.g.,* flow cytometry. In another specific embodiment, said cells do not express CD34 as detected by immunolocalization, *e.g.,* flow cytometry, after exposure to 1 to 100 ng/mL VEGF for 4 to 21 days. In more specific embodiments, said cells do not express CD34 as detected by immunolocalization, *e.g.,* flow cytometry, after exposure to 25 to 75 ng/mL VEGF for 4 to 21 days, or to 50 ng/mL VEGF for 4 to 21 days. In even more specific embodiments, said cells do not express CD34 as detected by immunolocalization, *e.g.,* flow cytometry, after exposure to 1, 2.5, 5, 10, 25, 50, 75 or 100 ng/mL VEGF for 4 to 21 days. In yet more specific embodiments, said cells do not express CD34 as detected by immunolocalization, *e.g.,* flow cytometry, after exposure to 1 to 100 ng/mL VEGF for 7 to 14, *e.g.,* 7, days.

In specific embodiments, the amnion derived adherent cells are OCT-4⁻, as determinable by RT-PCR, and one or more of VE-cadherin⁻, VEGFR2/KDR⁺, CD9⁺, CD54⁺, CD105⁺, and/or CD200⁺ as determinable by immunolocalization, *e.g.,* flow cytometry. In a specific embodiment, the amnion derived adherent cells are OCT-4⁻, as determinable by RT-PCR, and VE-cadherin⁻, VEGFR2/KDR⁺, CD9⁺, CD54⁺, CD105⁺, and CD200⁺ as determinable by immunolocalization, *e.g.,* flow cytometry. In another specific embodiment, said cells do not express CD34, as detected by immunolocalization, *e.g.,* flow cytometry, *e.g.,* after exposure to 1 to 100 ng/mL VEGF for 4 to 21 days.

In another embodiment, the amnion derived adherent cells are OCT-4⁻, CD49f⁺, HLA-G⁻, CD90⁺, CD105⁺, and CD117⁻. In a more specific embodiment, said cells are one or more of CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻, or CXCR4⁻, as determinable by immunolocalization, *e.g.,* flow cytometry. In a more specific embodiment, said cells CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻, and CXCR4⁻ as determinable by immunolocalization, *e.g.,* flow cytometry. In another specific embodiment, said cells are additionally VEGFR1/Flt-1⁺ and/or VEGFR2/KDR⁺, as determinable by immunolocalization, *e.g.,* flow cytometry; and one or more of CD31⁻, CD34⁻, CD45⁻, CD133⁻, and/or Tie-2⁻ as determinable by immunolocalization, *e.g.,* flow cytometry. In another specific embodiment, said cells are additionally VEGFR1/Flt-1⁺, VEGFR2/KDR⁺, CD31⁻, CD34⁻, CD45⁻, CD133⁻, and Tie-2⁻ as determinable by immunolocalization, e.g., flow cytometry.

In another embodiment, the OCT-4- amnion derived adherent cells are additionally one or more, or all, of CD9⁺, CD10⁺, CD44⁺, CD49f⁺, CD54⁺, CD90⁺, CD98⁺, CD105⁺, CD200⁺, Tie-2⁺, TEM-7⁺, VEGFR1/Flt-1⁺, and/or VEGFR2/KDR⁺ (CD309⁺), as determinable by immunolocalization, *e.g.,* flow cytometry; or additionally one or more, or all, of CD31⁻, CD34⁻, CD38⁻, CD45⁻, CD117⁻, CD133⁻, CD143⁻, CD144⁻, CD146⁻, CD271⁻, CXCR4⁻, HLA-G⁻, and/or VE-cadherin⁻, as determinable by immunolocalization, *e.g.,* flow cytometry, or SOX2⁻, as determinable by RT-PCR.

In certain embodiments, the isolated tissue culture plastic-adherent amnion derived adherent cells are CD49f⁺. In a specific embodiment, said CD49f⁺ cells are additionally one or more, or all, of CD9⁺, CD10⁺, CD44⁺, CD54⁺, CD90⁺, CD98⁺, CD105⁺, CD200⁺, Tie-2⁺, TEM-7⁺, VEGFR1/Flt-1⁺, and/or VEGFR2/KDR⁺ (CD309⁺), as determinable by immunolocalization, *e.g.,* flow cytometry; or additionally one or more, or all, of CD31⁻, CD34⁻, CD38⁻, CD45⁻, CD117⁻, CD133⁻, CD143⁻, CD144⁻, CD146⁻, CD271⁻, CXCR4⁻, HLA-G⁻, OCT-4⁻ and/or VE-cadherin⁻, as determinable by immunolocalization, *e.g.,* flow cytometry, or SOX2⁻, as determinable by RT-PCR.

In certain other embodiments, the isolated tissue culture plastic-adherent amnion derived adherent cells are HLA-G⁻, CD90⁺, and CD117⁻. In a specific embodiment, said HLA-G⁻, CD90⁺, and CD117⁻ cells are additionally one or more, or all, of CD9⁺, CD10⁺, CD44⁺, CD49f⁺, CD54⁺, CD98⁺, CD105⁺, CD200⁺, Tie-2⁺, TEM-7⁺, VEGFR1/Flt-1⁺, and/or VEGFR2/KDR⁺ (CD309⁺), as determinable by immunolocalization, *e.g.,* flow cytometry; or additionally one or more, or all, of CD31⁻, CD34⁻, CD38⁻, CD45⁻,CD133⁻, CD143⁻, CD144⁻, CD146⁻, CD271⁻, CXCR4⁻, OCT-4⁻ and/or VE-cadherin⁻, as determinable by immunolocalization, *e.g.,* flow cytometry, or SOX2⁻, as determinable by RT-PCR.

In another embodiment, the isolated amnion derived adherent cells do not constitutively express mRNA for angiopoietin 4 (ANGPT4), angiopoietin-like 3 (ANGPTL3), cadherin 5, type 2 (CDH5), bone gamma-carboxyglutamate (gla) protein (BGLAP), CD31, CD34, chemokine (C-X-C motif) ligand 10 (CXCL10), distal-less homeobox 5 (DLX5), fibrinogen α chain (FGA), fibroblast growth factor 4 (FGF4), FMS-like tyrosine kinase 3 (FLT3), HLA-G, interferon γ (IFNG), leukocyte cell derived chemotaxin 1 (LECT1), leptin (LEP), matrix metalloprotease 13 (MMP-13), NANOG, nestin, plasminogen (PLG), POU5F1 (OCT-4), prolactin (PRL), prokineticin 1 (PROK1), (sex determining region Y)-box 2 (SOX2), telomerase reverse transcriptase (TERT), tenomodulin (TNMD), and/or extracellular link domain containing 1 (XLKD1), as determinable by RT-PCR, *e.g.,* for 30 cycles under standard culture conditions.

In other embodiments, isolated amnion derived adherent cells, or population of amnion derived adherent cells, express mRNA for (ARNT2), nerve growth factor (NGF), brain-derived neurotrophic factor (BDNF), glial-derived neurotrophic factor (GDNF), neurotrophin 3 (NT-3), NT-5, hypoxia-Inducible Factor 1α (HIF1A), hypoxia-inducible protein 2 (HIG2), heme oxygenase (decycling) 1 (HMOX1), Extracellular superoxide dismutase [Cu-Zn] (SOD3), catalase (CAT), transforming growth factor β1 (TGFB1), transforming growth factor β1 receptor (TGFB1R), and hepatoycte growth factor receptor (HGFR/c-met)

In another aspect, provided herein are isolated populations of cells, *e.g.,* isolated populations of amnion cells or placental cells, or substantially isolated populations of AMDACs, comprising the amnion derived adherent cells described herein. The populations of cells can be homogeneous populations, *e.g.,* a population of cells, at least about 90%, 95%, 98% or 99% of which are amnion derived adherent cells. The populations of cells can be heterogeneous, *e.g.,* a population of cells wherein at most about 10%, 20%, 30%, 40%, 50%, 60%, 70% or 80% of the cells in the population are amnion derived adherent cells. The isolated populations of cells are not, however, tissue, *i.e.,* amniotic membrane.

In one embodiment, provided herein is an isolated population of cells comprising AMDACs, *e.g.,* a population of cells substantially homogeneous for AMDACs, or a population of cells heterogeneous with respect to the AMDACs, wherein said AMDACs are adherent to tissue culture plastic, and wherein said AMDACs are OCT-4⁻, as determinable by RT-PCR. In a specific embodiment, the AMDACs are CD49f⁺ or HLA-G⁺, *e.g.,* as determinable by immunolocalization, *e.g.,* flow cytometry, or RT-PCR. In another specific embodiment, said AMDACs in said population of cells are VEGFR1/Flt-1⁺ and/or VEGFR2/KDR⁺ as determinable by immunolocalization, *e.g.,* flow cytometry, wherein said isolated population of cells is not an amnion or amniotic membrane or other tissue. In a more specific embodiment, the AMDACs in said population of cells are OCT-4⁻, and/or HLA-G⁻ as determinable by RT-PCR, and VEGFR1/Flt-1⁺ and/or VEGFR2/KDR⁺ as determinable by immunolocalization, *e.g.,* flow cytometry. In another specific embodiment, said AMDACs are CD90⁺, CD105⁺, or CD117⁻. In a more specific embodiment, said AMDACs are CD90⁺, CD105⁺, and CD117⁻. In a more specific embodiment, the AMDACs are OCT-4⁻, CD49f⁺, CD90⁺, CD105⁺, and CD117⁻. In another specific embodiment, the AMDACs do not express SOX2, *e.g.,* as determinable by RT-PCR for 30 cycles. In an even more specific embodiment, the population comprises AMDACs, wherein said AMDACs are OCT-4⁻, HLA-G⁻, CD49f⁺, CD90⁺, CD105⁺, and CD117⁻, as determinable by immunolocalization, *e.g.,* flow cytometry, and SOX2⁻, *e.g.,* as determinable by RT-PCR for 30 cycles.

In another specific embodiment, said AMDACs in said population of cells are CD90⁺, CD105⁺, or CD117⁻, as determinable by immunolocalization, *e.g.,* flow cytometry. In a more specific embodiment, the AMDACs are CD90⁺, CD105⁺, and CD117⁻, as determinable by immunolocalization, *e.g.,* flow cytometry. In a more specific embodiment, the AMDACs are OCT-4- or HLA-G⁻, *e.g.,* as determinable by RT-PCR, and are additionally CD49f⁺, CD90⁺, CD105⁺, and CD117⁻ as determinable by immunolocalization, *e.g.,* flow cytometry. In a more specific embodiment, the AMDACs in said population of cells are OCT-4⁻, HLA-G⁻, CD49f⁺, CD90⁺, CD105⁺, and CD117⁻. In another specific embodiment, the AMDACs do not express SOX2, *e.g.,* as determinable by RT-PCR for 30 cycles. In a more specific embodiment, therefore, the AMDACs are OCT-4⁻, CD49f⁺, CD90⁺, CD105⁺, and CD117⁻, as determinable by immunolocalization, *e.g.,* flow cytometry, and SOX2⁻, as determinable by RT-PCR, *e.g.,* for 30 cycles. In an even more specific embodiment, the AMDACs are OCT-4⁻ or HLA-G⁻, and are additionally CD49f⁺, CD90⁺, CD105⁺, and CD117⁻. In a more specific embodiment, the AMDACs are OCT-4⁻, HLA-G⁻, CD49f⁺, CD90⁺, CD105⁺, and CD117⁻.

In another embodiment, the amnion derived adherent cells in said population of cells are adherent to tissue culture plastic, OCT-4- as determinable by RT-PCR, and VEGFR1/Flt-1⁺ and/or VEGFR2/KDR⁺ as determinable by immunolocalization, *e.g.,* flow cytometry, and are additionally one or more of CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻, or CXCR4⁻, as determinable by immunolocalization, *e.g.,* flow cytometry, or HLA-G⁻ as determinable by RT-PCR, and wherein said isolated population of cells is not an amnion. In another embodiment, provided herein is an isolated population of cells comprising amnion derived adherent cells, wherein said cells are adherent to tissue culture plastic, wherein said cells are OCT-4- as determinable by RT-PCR, and VEGFR1/Flt-1⁺ and/or VEGFR2/KDR⁺ as determinable by immunolocalization, *e.g.,* flow cytometry, wherein said cells do not express CD34 as detected by immunolocalization, *e.g.,* flow cytometry, after exposure to 1 to 100 ng/mL VEGF for 4 to 21 days, and wherein said isolated population of cells is not an amnion.

In a specific embodiment of any of the above embodiments, at least about 50%, 60%, 70%, 80%, 90%, 95%, 98% or 99% of cells in said population are said amnion derived adherent cells, as described or characterizable by any of the cellular marker combinations described above.

In another embodiment, any of the above populations of cells comprising amnion derived adherent cells forms sprouts or tube-like structures when cultured in the presence of an extracellular matrix protein, *e.g.,* like collagen type I and IV, or an angiogenic factor, *e.g.,* like vascular endothelial growth factor (VEGF), epithelial growth factor (EGF), platelet derived growth factor (PDGF) or basic fibroblast growth factor (bFGF), *e.g.,* in or on a substrate such as placental collagen, *e.g.,* or MATRIGEL™ for at least 4 days and up to 14 days.

In certain embodiments, provided herein is a cell that expresses, or a population of cells, wherein at least about 50%, 60%, 70%, 80%, 90%, 95% or 98% of cells in said isolated population of cells are amnion derived adherent cells that express RNA for one or more of, or all of, ACTA2 (actin, alpha 2, smooth muscle, aorta), ACTC1 (Actin, alpha cardiac muscle 1), ADAMTS1 (ADAM metallopeptidase with thrombospondin type 1 motif, 1), AMOT (angiomotin), ANG (angiogenin), ANGPT1 (angiopoietin 1), ANGPT2, ANGPTL1 (angiopoietin-like 1), ANGPTL2, ANGPTL4, BAI1 (brain-specific angiogenesis inhibitor 1), c-myc, CD44, CD140a, CD140b, CD200, CD202b, CD304, CD309, CEACAM1 (carcinoembryonic antigen-related cell adhesion molecule 1), CHGA (chromogranin A), COL15A1 (collagen, type XV, alpha 1), COL18A1 (collagen, type XVIII, alpha 1), COL4A1 (collagen, type IV, alpha 1), COL4A2 (collagen, type IV, alpha 2), COL4A3 (collagen, type IV, alpha 3), connexin-43, CSF3 (colony stimulating factor 3 (granulocyte), CTGF (connective tissue growth factor), CXCL 12 (chemokine (CXC motif) ligand 12 (stromal cell-derived factor 1)), CXCL2, DNMT3B (DNA (cytosine-5-)-methyltransferase 3 beta), ECGF1 (thymidine phosphorylase), EDG1 (endothelial cell differentiation gene 1), EDIL3 (EGF-like repeats and discoidin I-like domains 3), ENPP2 (ectonucleotide pyrophosphatase/ phosphodiesterase 2), EPHB2 (EPH receptor B2), FBLN5 (FIBULIN 5), F2 (coagulation factor II (thrombin)), FGF1 (acidic fibroblast growth factor), FGF2 (basic fibroblast growth factor), FIGF (c-fos induced growth factor (vascular endothelial growth factor D)), FLT4 (fms-related tyrosine kinase 4), FN1 (fibronectin 1), FST (follistatin), FOXC2 (forkhead box C2 (MFH-1, mesenchyme forkhead 1)), follistatin, Galectin-1, GRN (granulin), HGF (hepatocyte growth factor), HEY1 (hairy/enhancer-of-split related with YRPW motif 1), HSPG2 (heparan sulfate proteoglycan 2), IFNB1 (interferon, beta 1, fibroblast), IL8 (interleukin 8), IL12A, ITGA4 (integrin, alpha 4; CD49d), ITGAV (integrin, alpha V), ITGB3 (integrin, beta 3), KLF4 (Kruppel-like factor 4), MDK (midkine), MMP2 (matrix metalloprotease 2), MYOZ2 (myozenin 2), NRP2 (neuropilin 2), PDGFB (platelet-derived growth factor β, PF4 (platelet factor 4), PGK1 (phosphoglycerate kinase 1), PROX1 (prospero homeobox 1), PTN (pleiotrophin), SEMA3F (semophorin 3F), SERPINB5 (serpin peptidase inhibitor, clade B (ovalbumin), member 5), SERPINC1, SERPINF1, TIMP2 (tissue inhibitor of metalloproteinases 2), TIMP3, TGFA (transforming growth factor, alpha), TGFB1, THBS1 (thrombospondin 1), THBS2, TIE1 (tyrosine kinase with immunoglobulin-like and EGF-like domains 1), TNF (tumor necrosis factor), TNNC1 (troponin C, type 1), TNNT2, TNFSF15 (tumor necrosis factor (ligand) superfamily, member 15), VASH1 (vasohibin 1), VEGF (vascular endothelial growth factor), VEGFB, VEGFC, and/or VEGFR1/FLT1 (vascular endothelial growth factor receptor 1).

When human cells are used, the gene designations throughout refer to human sequences, and, as is well known to persons of skill in the art, representative sequences can be found in literature, or in GenBank. Probes to the sequences can be determined by sequences that are publicly-available, or through commercial sources, e.g., specific TAQMAN® probes or TAQMAN® Angiogenesis Array (Applied Biosystems, part no. 4378710).

In certain embodiments, provided herein is a cell that expresses, or a population of cells, wherein at least about 50%, 60%, 70%, 80%, 90%, 95% or 98% of cells in said isolated population of cells are amnion derived adherent cells that express CD49d, Connexin-43, HLA-ABC, Beta 2-microglobulin, CD349, CD318, PDL1, CD106, Galectin-1, ADAM 17 precursor (A disintegrin and metalloproteinase domain 17) (TNF-alpha converting enzyme) (TNF-alpha convertase), Angiotensinogen precursor, Filamin A (Alpha-filamin) (Filamin 1) (Endothelial actin-binding protein) (ABP-280) (Nonmuscle filamin), Alpha-actinin 1 (Alpha-actinin cytoskeletal isoform) (Non-muscle alpha-actinin 1) (F-actin cross linking protein), Low-density lipoprotein receptor-related protein 2 precursor (Megalin) (Glycoprotein 330) (gp330), Macrophage scavenger receptor types I and II (Macrophage acetylated LDL receptor I and II), Activin receptor type IIB precursor (ACTR-IIB), Wnt-9 protein, Glial fibrillary acidic protein, astrocyte (GFAP), Myosin-binding protein C, cardiac-type (Cardiac MyBP-C) (C-protein, cardiac muscle isoform), and/or Myosin heavy chain, nonmuscle type A (Cellular myosin heavy chain, type A) (Nonmuscle myosin heavy chain-A) (NMMHC-A), as detectable by immunolocalization.

In certain embodiments, the amnion derived adherent cells, or population of cells comprising amnion derived adherent cells, *e.g.,* wherein at least about 50%, 60%, 70%, 80%, 90%, 95% or 98% of cells in said isolated population of cells are amnion derived adherent cells, secrete one or more, or all, of VEGF, HGF, IL-8, MCP-3, FGF2, Follistatin, G-CSF, EGF, ENA-78, GRO, IL-6, MCP-1, PDGF-BB, TIMP-2, uPAR, Galectin-1, *e.g.,* into culture medium in which the cell, or cells, are grown.

In another embodiment, provided herein is a population of cells, *e.g.,* a population of amnion derived adherent cells, or a population of cells wherein at least about 50%, 60%, 70%, 80%, 90%, 95% or 98% of cells in said isolated population of cells are amnion derived adherent cells that express micro RNAs (miRNAs) at a higher level than bone marrow-derived mesenchymal stem cells, wherein said miRNAs comprise one or more, or all of, miR-17-3p, miR-18a, miR-18b, miR-19b, miR-92, and/or miR-296. In another embodiment, provided herein is a population of cells, *e.g.,* a population of amnion derived adherent cells, or a population of cells wherein at least about 50%, 60%, 70%, 80%, 90%, 95% or 98% of cells in said isolated population of cells are amnion derived adherent cells that express one or more of, or all of, micro RNAs (miRNAs) at a lower level than bone marrow-derived mesenchymal stem cells, wherein said miRNAs comprise one or more, or all of, miR-20a, miR-20b, miR-221, miR-222, miR-15b, and/or miR-16. In certain embodiments, AMDACs, or populations of AMDACs, express one or more, or all, of the angiogenic miRNAs miR-17-3p, miR-18a, miR-18b, miR-19b, miR-92, miR-20a, miR-20b, (members of the of the angiogenic miRNA cluster 17-92), miR-296, miR-221, miR-222, miR-15b, and/or miR-16.

In one embodiment, provided herein are isolated amnion derived adherent cells, wherein said cells are adherent to tissue culture plastic, wherein said cells are OCT-4⁻, as determinable by RT-PCR, and CD49f⁺, HLA-G⁻, CD90⁺, CD105⁺, and CD117⁻, as determinable by immunolocalization, *e.g.,* flow cytometry, and wherein said cells: (a) express one or more of CD9, CD10, CD44, CD54, CD98, CD200, Tie-2, TEM-7, VEGFR1/Flt-1, or VEGFR2/KDR (CD309), as determinable by immunolocalization, *e.g.,* flow cytometry; (b) lack expression of CD31, CD34, CD38, CD45, CD133, CD143, CD144, CD146, CD271, CXCR4, HLA-G, or VE-cadherin, as determinable by immunolocalization, *e.g.,* flow cytometry; (c) lack expression of SOX2, as determinable by RT-PCR; (d) express mRNA for ACTA2, ADAMTS1, AMOT, ANG, ANGPT1, ANGPT2, ANGPTL1, ANGPTL2, ANGPTL4, BAI1, c-myc, CD44, CD140a, CD140b, CD200, CD202b, CD304, CD309, CEACAM1, CHGA, COL15A1, COL18A1, COL4A1, COL4A2, COL4A3, Connexin-3, CSF3, CTGF, CXCL12, CXCL2, DNMT3B, ECGF1, EDG1, EDIL3, ENPP2, EPHB2, FBLN5, F2, FGF1, FGF2, FIGF, FLT4, FN1, FST, FOXC2, Galectin-1, GRN, HGF, HEY1, HSPG2, IFNB1, IL8, IL12A, ITGA4, ITGAV, ITGB3, KLF-4, MDK, MMP2, MYOZ2, NRP2, PDGFB, PF4, PGK1, PROX1, PTN, SEMA3F, SERPINB5, SERPINC1, SERPINF1, TGFA, TGFB1, THBS1, THBS2, TIE1, TIMP2, TIMP3, TNF, TNNC1, TNNT2, TNFSF15, VASH1, VEGF, VEGFB, VEGFC, or VEGFR1/FLT1; (e) express one or more of the proteins CD49d, Connexin-43, HLA-ABC, Beta 2-microglobulin, CD349, CD318, PDL1, CD106, Galectin-1, ADAM 17, angiotensinogen precursor, filamin A, alpha-actinin 1, megalin, macrophage acetylated LDL receptor I and II, activin receptor type IIB precursor, Wnt-9 protein, glial fibrillary acidic protein, astrocyte, myosin-binding protein C, or myosin heavy chain, nonmuscle type A; (f) secret VEGF, HGF, IL-8, MCP-3, FGF2, Follistatin, G-CSF, EGF, ENA-78, GRO, IL-6, MCP-1, PDGF-BB, TIMP-2, uPAR, or galectin-1 into culture medium in which the cell grows; (g) express micro RNAs miR-17-3p, miR-18a, miR-18b, miR-19b, miR-92, or miR-296 at a higher level than an equivalent number of bone marrow-derived mesenchymal stem cells; (h) express micro RNAs miR-20a, miR-20b, miR-221, miR-222, miR-15b, or miR-16 at a lower level than an equivalent number of bone marrow-derived mesenchymal stem cells; (i) express miRNAs miR-17-3p, miR-18a, miR-18b, miR-19b, miR-92, miR-20a, miR-20b, miR-296, miR-221, miR-222, miR-15b, or miR-16; and/or (j) express increased levels of CD202b, IL-8 or VEGF when cultured in less than about 5% O₂, compared to expression of CD202b, IL-8 or VEGF under 21% O₂. In a specific embodiment, the isolated amnion derived adherent cells are OCT-4⁻, as determinable by RT-PCR, and CD49f⁺, HLA-G⁻, CD90⁺, CD105⁺, and CD117⁻, as determinable by immunolocalization, *e.g.,* flow cytometry, and (a) express CD9, CD10, CD44, CD54, CD90, CD98, CD200, Tie-2, TEM-7, VEGFR1/Flt-1, and VEGFR2/KDR (CD309), as determinable by immunolocalization, *e.g.,* flow cytometry; (b) lack expression of CD31, CD34, CD38, CD45, CD133, CD143, CD144, CD146, CD271, CXCR4, HLA-G, and VE-cadherin, as determinable by immunolocalization, *e.g.,* flow cytometry; (c) lack expression of SOX2, as determinable by RT-PCR; (d) express mRNA for ACTA2, ADAMTS1, AMOT, ANG, ANGPT1, ANGPT2, ANGPTL1, ANGPTL2, ANGPTL4, BAI1, c-myc, CD44, CD140a, CD140b, CD200, CD202b, CD304, CD309, CEACAM1, CHGA, COL15A1, COL18A1, COL4A1, COL4A2, COL4A3, Connexin-3, CSF3, CTGF, CXCL12, CXCL2, DNMT3B, ECGF1, EDG1, EDIL3, ENPP2, EPHB2, FBLN5, F2, FGF1, FGF2, FIGF, FLT4, FN1, FST, FOXC2, Galectin-1, GRN, HGF, HEY1, HSPG2, IFNB1, IL8, IL12A, ITGA4, ITGAV, ITGB3, KLF-4, MDK, MMP2, MYOZ2, NRP2, PDGFB, PF4, PGK1, PROX1, PTN, SEMA3F, SERPINB5, SERPINC1, SERPINF1, TGFA, TGFB1, THBS1, THBS2, TIE1, TIMP2, TIMP3, TNF, TNNC1, TNNT2, TNFSF15, VASH1, VEGF, VEGFB, VEGFC, and VEGFR1/FLT1; (e) express one or more of CD49d, Connexin-43, HLA-ABC, Beta 2-microglobulin, CD349, CD318, PDL1, CD106, Galectin-1, ADAM 17, angiotensinogen precursor, filamin A, alpha-actinin 1, megalin, macrophage acetylated LDL receptor I and II, activin receptor type IIB precursor, Wnt-9 protein, glial fibrillary acidic protein, astrocyte, myosin-binding protein C, and/or myosin heavy chain, nonmuscle type A; (f) secrete VEGF, HGF, IL-8, MCP-3, FGF2, Follistatin, G-CSF, EGF, ENA-78, GRO, IL-6, MCP-1, PDGF-BB, TIMP-2, uPAR, and/or Galectin-1, *e.g.,* into culture medium in which the cells grow; (g) express micro RNAs miR-17-3p, miR-18a, miR-18b, miR-19b, miR-92, and miR-296 at a higher level than an equivalent number of bone marrow-derived mesenchymal stem cells; (h) express micro RNAs miR-20a, miR-20b, miR-221, miR-222, miR-15b, and miR-16 at a lower level than an equivalent number of bone marrow-derived mesenchymal stem cells; (i) express miRNAs miR-17-3p, miR-18a, miR-18b, miR-19b, miR-92, miR-20a, miR-20b, miR-296, miR-221, miR-222, miR-15b, and miR-16; and/or (i) expresses increased levels of CD202b, IL-8 and VEGF when cultured in less than about 5% O₂, compared to expression of CD202b, IL-8 and/or VEGF when said cells are cultured under 21 % O₂. Further provided herein are populations of cells comprising AMDACs, *e.g.* populations of AMDACs, having one or more of the above-recited characteristics.

In other specific embodiments, the population of cells comprising amnion-derived angiogenic cells secretes one or more angiogenic factors and thereby induces human endothelial cells to migrate in an *in vitro* wound healing assay. In other specific embodiments, the population of cells comprising amnion derived adherent cells induces maturation, differentiation or proliferation of human endothelial cells, endothelial progenitors, myocytes or myoblasts.

In another embodiment, any of the above amnion derived adherent cells, or populations of cells comprising amnion derived adherent cells, take up acetylated low density lipoprotein (LDL) when cultured in the presence of extracellular matrix proteins, *e.g.,* collagen type I or IV, and/or one or more angiogenic factors, *e.g.,* VEGF, EGF, PDGF, or bFGF, *e.g.,* on a substrate such as placental collagen or MATRIGEL™.

In another embodiment, the AMDACs are comprised within a population of cells. In specific embodiments of such embodiments, the amnion derived adherent cells are adherent to tissue culture plastic, are OCT-4⁻, as determinable by RT-PCR, and VEGFR2/KDR⁺, CD9⁺, CD54⁺, CD105⁺, CD200⁺, or VE-cadherin⁻, as determinable by immunolocalization, *e.g.,* flow cytometry. In specific embodiments, at least 10%, 20%, 30%, 40%, 50%, 60%, 70%, 80%, 90%, 95%, 98% or 99% of the cells in said population of cells are amnion derived cells that are OCT-4⁻, as determinable by RT-PCR, and VEGFR2/KDR⁺, CD9⁺, CD54⁺, CD105⁺, CD200⁺, or VE-cadherin⁻, as determinable by immunolocalization, *e.g.,* flow cytometry. In another specific embodiment, at least 10%, 20%, 30%, 40%, 50%, 60%, 70%, 80%, 90%, 95%, 98% or 99% of the cells in said population are amnion derived cells that are OCT-4⁻, as determinable by RT-PCR, and VEGFR2/KDR⁺, CD9⁺, CD54⁺, CD105⁺, CD200⁺, and VE-cadherin⁻, as determinable by immunolocalization, *e.g.,* flow cytometry. In another specific embodiment, said cells that are OCT-4⁻, as determinable by RT-PCR, and VEGFR2/KDR⁺, CD9⁺, CD54⁺, CD105⁺, CD200⁺, or VE-cadherin⁻, as determinable by immunolocalization, *e.g.,* flow cytometry, do not express CD34, as determinable by immunolocalization, *e.g.,* flow cytometry, after exposure to 1 to 100 ng/mL VEGF for 4 to 21 days. In another specific embodiment, said cells are also VE-cadherin⁻.

In a specific embodiment, said amnion derived cells that are OCT-4⁻, as determinable by RT-PCR, and VEGFR2/KDR⁺, CD9⁺, CD54⁺, CD105⁺, CD200⁺, or VE-cadherin⁻, as determinable by immunolocalization, *e.g.,* flow cytometry, form sprouts or tube-like structures when said population of cells is cultured in the presence of vascular endothelial growth factor (VEGF).

The amnion derived adherent cells described herein display the above characteristics, *e.g.,* combinations of cell surface markers and/or gene expression profiles, in primary culture, or during proliferation in medium suitable for the culture of stem cells. Such medium includes, for example, medium comprising 1 to 100% DMEM-LG (Gibco), 1 to 100% MCDB-201 (Sigma), 1 to 10% fetal calf serum (FCS) (Hyclone Laboratories), 0.1 to 5x insulin-transferrin-selenium (ITS, Sigma), 0.1 to 5x linolenic-acid-bovine-serum-albumin (LA-BSA, Sigma), 10⁻⁵ to 10⁻¹⁵ M dexamethasone (Sigma), 10⁻² to 10⁻¹⁰ M ascorbic acid 2-phosphate (Sigma), 1 to 50 ng/mL epidermal growth factor (EGF), (R&D Systems), 1 to 50 ng/mL platelet derived-growth factor (PDGF-BB) (R&D Systems), and 100U penicillin/1000U streptomycin. In a specific embodiment, the medium comprises 60% DMEM-LG (Gibco), 40% MCDB-201(Sigma), 2% fetal calf serum (FCS) (Hyclone Laboratories), 1x insulin-transferrin-selenium (ITS), 1x linolenic-acid-bovine-serum-albumin (LA-BSA), 10⁻⁹ M dexamethasone (Sigma), 10⁻⁴M ascorbic acid 2-phosphate (Sigma), epidermal growth factor (EGF)10 ng/ml (R&D Systems), platelet derived-growth factor (PDGF-BB) 10 ng/ml (R&D Systems), and 100U penicillin/1000U streptomycin Other suitable media are described below.

The isolated populations of amnion derived adherent cells provided herein can comprise about, at least about, or no more than about, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more amnion derived adherent cells, *e.g.,* in a container. In various embodiments, at least 10%, 20%, 30%, 40%, 50%, 60%, 70%, 80%, 90%, 95%, or 99% of the cells in the isolated cell populations provided herein are amnion derived adherent cells. That is, a population of isolated amnion derived adherent cells can comprise, *e.g.,* as much as 1%, 5%, 10%, 20%, 30%, 40%, 50%, 60%, 70%, 80%, 90% non-AMDAC cells. In other specific embodiments, at least 25%, 35%, 45%, 50%, 60%, 75%, 85% or more of the cells in the isolated population of cells comprising amnion derived adherent cells are not OCT-4⁺.

The amnion derived adherent cells provided herein can be cultured on a substrate. In various embodiments, the substrate can be any surface on which culture and/or selection of amnion derived adherent cells, can be accomplished. Typically, the substrate is plastic, *e.g.,* tissue culture dish or multiwell plate plastic. Tissue culture plastic can be treated, coated or imprinted with a biomolecule or synthetic mimetic agent, *e.g.,* CELLSTART™, MESENCULT™ ACF-substrate, ornithine, or polylysine, or an extracellular matrix protein, *e.g.,* collagen, laminin, fibronectin, vitronectin, or the like.

The amnion derived adherent cells provided herein, and populations of such cells, can be isolated from one or more placentas. Isolated amnion derived cells can be cultured and expanded to produce populations of such cells. Populations of cells comprising amnion derived adherent cells can also be cultured and expanded to produce populations of amnion derived adherent cells.

In certain embodiments, AMDACs displaying any of the above marker and/or gene expression characteristics have been passaged at least 1, 2, 3, 4, 5, 6, 7, 8, 9, 10, 11, 12, 13, 14, 15, 16, 17, 18, 19 or 20 times, or more. In certain other embodiments, AMDACs displaying any of the above marker and/or gene expression characteristics have been doubled in culture at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or at least 50 times, or more.

In a specific embodiment, the AMDACs described herein are negative for telomerase, as measured by RT-PCR and/or TRAP assays. In another specific embodiment, the AMDACs described herein do not express mRNA for telomerase reverse transcriptase (TERT) as determinable by RT-PCR, *e.g.,* for 30 cycles. In another specific embodiment, the AMDACs described herein are NANOG⁻, as measured by RT-PCR. In another specific embodiment, the AMDACs described herein do not express mRNA for NANOG as determinable by RT-PCR, *e.g.,* for 30 cycles. In a specific embodiment, the AMDACs described herein are negative for (sex determining region Y)-box 2 (SOX2). In another specific embodiment, the AMDACs described herein do not express mRNA for SOX2 as determinable by RT-PCR, *e.g.,* for 30 cycles. In another specific embodiment, the AMDACs described herein are not osteogenic as measured by an osteogenic phenotype assay (see, *e.g.,* Section 6.3.1, below). In another specific embodiment, the AMDACs described herein are not chondrogenic as measured by a chondrogenic potential assay (see, *e.g.,* Section 6.3.2, below). In another specific embodiment, the AMDACs described herein are not osteogenic as measured by an osteogenic phenotype assay (see, *e.g.,* Section 6.3.1, below) and are not chondrogenic as measured by a chondrogenic potential assay (see, *e.g.,* Section 6.3.1, below).

AMDACs can exhibit one or more of the characteristics described herein as determined by RT-PCR, as demonstrated in Table 3. For example, AMDACs can exhibit one or more of such characteristics when isolated and cultured as described in Section 5.6, below.

**Table 3**

| AMDAC Marker | Positive | Negative |
|---|---|---|
| ACTA2 | X | |
| ACTC1 | X | |
| ADAMTS1 | X | |
| AMOT | X | |
| ANG | X | |
| ANGPT1 | X | |
| ANGPT2 | X | |
| ANGPT4 | | X |
| ANGPTL1 | X | |
| ANGPTL2 | X | |
| ANGPTL3 | | X |
| ANGPTL4 | X | |
| BAI1 | X | |
| BGLAP | | X |
| c-myc | X | |
| CD31 | | X |
| CD34 | | X |
| CD44 | X | |
| CD140a | X | |
| CD140b | X | |
| CD200 | X | |
| CD202b | X | |
| CD304 | X | |
| CD309 (VEGFR2/KDR) | X | |
| CDH5 | | X |
| CEACAM1 | X | |
| CHGA | X | |
| COL15A1 | X | |
| COL18A1 | X | |
| COL4A1 | X | |
| COL4A2 | x | |
| COL4A3 | X | |
| Connexin-43 | X | |
| CSF3 | X | |
| CTGF | X | |
| CXCL10 | | X |
| CXCL12 | X | |
| CXCL2 | X | |
| DLX5 | | X |
| DNMT3B | X | |
| ECGF1 | X | |
| EDG1 | X | |
| EDIL3 | X | |
| ENPP2 | X | |
| EPHB2 | X | |
| F2 | X | |
| FBLN5 | X | |
| FGA | | X |
| FGF1 | X | |
| FGF2 | X | |
| FGF4 | | X |
| FIGF | X | |
| FLT3 | | X |
| FLT4 | X | |
| FN1 | X | |
| FOXC2 | X | |
| Follistatin | X | |
| Galectin-1 | X | |
| GRN | X | |
| HEY1 | X | |
| HGF | X | |
| HLA-G | | X |
| HSPG2 | X | |
| IFNB1 | X | |
| IFNG | | X |
| IL-8 | X | |
| IL-12A | X | |
| ITGA4 | X | |
| ITGAV | X | |
| ITGB3 | X | |
| KLF-4 | X | |
| LECT1 | | X |
| LEP | | X |
| MDK | X | |
| MMP-13 | | X |
| MMP-2 | X | |
| MYOZ2 | X | |
| NANOG | | X |
| NESTIN | | X |
| NRP2 | X | |
| PDGFB | X | |
| PF4 | X | |
| PGK1 | X | |
| PLG | | X |
| POU5F1 (OCT-4) | | X |
| PRL | | X |
| PROK1 | | X |
| PROX1 | X | |
| PTN | X | |
| SEMA3F | X | |
| SERPINB5 | X | |
| SERPINC1 | X | |
| SERPINF1 | X | |
| SOX2 | | X |
| TERT | | X |
| TGFA | X | |
| TGFB1 | X | |
| THBS1 | X | |
| THBS2 | X | |
| TIE1 | X | |
| TIMP2 | X | |
| TIMP3 | X | |
| TNF | X | |
| TNFSF15 | X | |
| TNMD | | X |
| TNNC1 | X | |
| TNNT2 | X | |
| VASH1 | X | |
| VEGF | X | |
| VEGFB | X | |
| VEGFC | X | |
| VEGFR1/FLT-1 | X | |
| XLKD1 | | X |

AMDACs can exhibit one or more of the characteristics described herein as determinable by immunolocalization, *e.g.,* flow cytometry, as demonstrated in Table 4. For example, AMDACs can exhibit one or more of such characteristics when isolated and cultured as described in Section 5.6, below.

**Table 4**

| AMDAC Marker | Positive | Negative |
|---|---|---|
| CD6 | | X |
| CD9 | X | |
| CD10 | X | |
| CD31 | | X |
| CD34 | | X |
| CD44 | X | |
| CD45 | | X |
| CD49b | X | |
| CD49c | X | |
| CD49d | X | |
| CD49f | X | |
| CD54 | X | |
| CD68 | X | |
| CD90 | X | |
| CD98 | X | |
| CD105 | X | |
| CD117 | | X |
| CD133 | | X |
| CD143 | | X |
| CD 144 (VE-cadherin) | | X |
| CD 146 | | X |
| CD166 | X | |
| CD184 | | X |
| CD200 | X | |
| CD202b | X | |
| CD271 | | X |
| CD304 | X | |
| CD309 (VEGFR2/KDR) | X | |
| CD318 | X | |
| CD349 | X | |
| CytoK | X | |
| HLA-ABC+ B2 Micro+ | X | |
| Invariant Chain+ HLA-DR-DP-DQ⁺ | | X |
| PDL-1 | X | |
| VEGFR1/FLT-1 | X | |

AMDACs can exhibit one or more of the characteristics described herein as determinable by immunolocalization, *e.g.,* immunofluorescence and/or immunohistochemistry, as demonstrated in Table 5. For example, AMDACs can exhibit one or more of such characteristics when isolated and cultured as described in Section 5.6, below.

**Table 5**

| AMDAC Marker | Positive | Negative |
|---|---|---|
| CD31 | | X |
| CD34 | | X |
| VEGFR2/KDR | X | |
| Connexin-43 | X | |
| Galectin-1 | X | |
| TEM-7 | X | |

AMDACs can exhibit one or more of the characteristics described herein as determinable by immunolocalization, *e.g.,* membrane proteomics, as demonstrated in Table 6. For example, AMDACs can exhibit one or more of such characteristics when isolated and cultured as described in Section 5.6, below.

**Table 6**

| AMDAC Marker | Positive | Negative |
|---|---|---|
| Activin receptor type IIB | X | |
| ADAM 17 | X | |
| Alpha-actinin 1 | X | |
| Angiotensinogen | X | |
| Filamin A | X | |
| Macrophage acetylated LDL receptor I and II | X | |
| Megalin | X | |
| Myosin heavy chain non muscle type A | X | |
| Myosin-binding protein C cardiac type | X | |
| Wnt-9 | X | |

AMDACs can exhibit one or more of the characteristics described herein as determinable by secretome analysis, e.g., ELISA, as demonstrated in Table 7. For example, AMDACs can exhibit one or more of such characteristics when isolated and cultured as described in Section 5.6, below.

**Table 7**

| AMDAC Marker | Positive | Negative |
|---|---|---|
| ANG | X | |
| EGF | X | |
| ENA-78 | X | |
| FGF2 | X | |
| Follistatin | X | |
| G-CSF | X | |
| GRO | X | |
| HGF | X | |
| IL-6 | X | |
| IL-8 | X | |
| Leptin | X | |
| MCP-1 | X | |
| MCP-3 | X | |
| PDGFB | X | |
| PLGF | X | |
| Rantes | X | |
| TGFB1 | X | |
| Thrombopoietin | X | |
| TIMP1 | X | |
| TIMP2 | X | |
| uPAR | X | |
| VEGF | X | |
| VEGFD | X | |

### 5.4 POPULATIONS OF AMNION DERIVED ADHERENT CELLS COMPRISING OTHER CELL TYPES

The isolated cell populations comprising amnion derived adherent cells described herein can comprise a second type of cell, *e.g.,* placental cells that are not amnion derived adherent cells, or, *e.g.,* cells that are not placental cells. For example, an isolated population of amnion derived adherent cells can comprise, *e.g.,* can be combined with, a population of a second type of cells, wherein said second type of cell are, *e.g.,* embryonic stem cells, blood cells (*e.g.,* placental blood, placental blood cells, umbilical cord blood, umbilical cord blood cells, peripheral blood, peripheral blood cells, nucleated cells from placental blood, umbilical cord blood, or peripheral blood, and the like), stem cells isolated from blood (*e.g.,* stem cells isolated from placental blood, umbilical cord blood or peripheral blood), placental stem cells (*e.g.,* the placental stem cells described in U.S. Patent No. 7,468,276, and in U.S. Patent Application Publication No. 2007/0275362, the disclosures of which are incorporated herein by reference in their entireties), nucleated cells from placental perfusate, *e.g.,* total nucleated cells from placental perfusate, the cells described and claimed in U.S. Patent No. 7,638,141, the disclosure of which is hereby incorporated by reference in its entirety, umbilical cord stem cells, populations of blood-derived nucleated cells, bone marrow-derived mesenchymal stromal cells, bone marrow-derived mesenchymal stem cells, bone marrow-derived hematopoietic stem cells, crude bone marrow, adult (somatic) stem cells, populations of stem cells contained within tissue, cultured cells, *e.g.,* cultured stem cells, populations of fully-differentiated cells (*e.g*., chondrocytes, fibroblasts, amniotic cells, osteoblasts, muscle cells, cardiac cells, *etc*.), pericytes, and the like. In a specific embodiment, an isolated population of cells comprising amnion derived adherent cells comprises placental stem cells or stem cells from umbilical cord. In certain embodiments in which the second type of cell is blood or blood cells, erythrocytes have been removed from the population of cells.

In a specific embodiment, the second type of cell is a hematopoietic stem cell. Such hematopoietic stem cells can be, for example, contained within unprocessed placental blood, umbilical cord blood or peripheral blood; in total nucleated cells from placental blood, umbilical cord blood or peripheral blood; in an isolated population of CD34⁺ cells from placental blood, umbilical cord blood or peripheral blood; in unprocessed bone marrow; in total nucleated cells from bone marrow; in an isolated population of CD34⁺ cells from bone marrow, or the like.

In a another embodiment, the second cell type is a non-embryonic cell type manipulated in culture in order to express markers of pluripotency and functions associated with embryonic stem cells

In specific embodiments of the above isolated populations of amnion derived adherent cells, either or both of the amnion derived adherent cells and cells of a second type are autologous, or are allogeneic, to an intended recipient of the cells.

Further provided herein is a composition comprising amnion derived adherent cells, and a plurality of stem cells other than the amnion derived adherent cells. In a specific embodiment, the composition comprises a stem cell that is obtained from a placenta, *i.e.,* a placental stem cell, *e.g.,* placental stem cells as described in U.S. Patent Nos. 7,045,148; 7,255,879; and 7,311,905, and in U.S. Patent Application Publication No. 2007/0275362, the disclosures of each of which are incorporated herein by reference in their entireties. In a specific embodiment, the placental stem cells are CD34⁻, CD10⁺ and CD105⁺. In a more specific embodiment, the placental stem cells are CD34⁻, CD10⁺, CD105⁺ and CD200⁺. In a more specific embodiment, the placental stem cells are CD34⁻, CD45⁻, CD10⁺, CD90⁺, CD105⁺ and CD200⁺. In a more specific embodiment, the placental stem cells are CD34⁻, CD45⁻, CD80⁻, CD86⁻, CD10⁺, CD90⁺, CD105⁺ and CD200⁺. In other specific embodiments, said placental stem cells are CD200⁺ and HLA-G⁺; CD73⁺, CD105⁺, and CD200⁺; CD200⁺ and OCT-4⁺; CD73⁺, CD105⁺ and HLA-G⁺; CD73⁺ and CD105⁺ and facilitate the formation of one or more embryoid-like bodies in a population of placental cells comprising said stem cell when said population is cultured under conditions that allow the formation of an embryoid-like body; or OCT-4⁺ and facilitate the formation of one or more embryoid-like bodies in a population of placental cells comprising the stem cell when said population is cultured under conditions that allow formation of embryoid-like bodies; or any combination thereof. In a more specific embodiment, said CD200⁺, HLA-G⁺ stem cells are CD34⁻, CD38⁻, CD45⁻, CD73⁺ and CD105⁺. In another more specific embodiment, said CD73⁺, CD105⁺, and CD200⁺ stem cells are CD34⁻, CD38⁻, CD45⁻, and HLA-G⁺. In another more specific embodiment, said CD200⁺, OCT-4⁺ stem cells are CD34⁻, CD38⁻, CD45⁻, CD73⁺, CD105⁺ and HLA-G⁺. In another more specific embodiment, said CD73⁺, CD105⁺ and HLA-G⁺ stem cells are CD34⁻, CD45⁻, OCT-4⁺ and CD200⁺. In another more specific embodiment, said CD73⁺ and CD105⁺ stem cells are OCT-4⁺, CD34⁻, CD38⁻ and CD45⁻. In another more specific embodiment, said OCT-4⁺ stem cells are CD73⁺, CD105⁺, CD200⁺, CD34⁻, CD38⁻, and CD45⁻. In another more specific embodiment, the placental stem cells are maternal in origin (that is, have the maternal genotype). In another more specific embodiment, the placental stem cells are fetal in origin (that is, have the fetal genotype).

In another specific embodiment, the composition comprises amnion derived adherent cells, and embryonic stem cells. In another specific embodiment, the composition comprises amnion derived adherent cells and mesenchymal stromal or stem cells, *e.g.,* bone marrow-derived mesenchymal stromal or stem cells. In another specific embodiment, the composition comprises bone marrow-derived hematopoietic stem cells. In another specific embodiment, the composition comprises amnion derived adherent cells and hematopoietic progenitor cells, *e.g.,* hematopoietic progenitor cells from bone marrow, fetal blood, umbilical cord blood, placental blood, and/or peripheral blood. In another specific embodiment, the composition comprises amnion derived adherent cells and somatic stem cells. In a more specific embodiment, said somatic stem cell is a neural stem cell, a hepatic stem cell, a pancreatic stem cell, an endothelial stem cell, a cardiac stem cell, or a muscle stem cell.

In other specific embodiments, the second type of cells comprise about, at least, or no more than, 10%, 15%, 20%, 25%, 30%, 35%, 40%, 45%, or 50% of cells in said population. In other specific embodiments, the AMDACs in said composition comprise at least 50%, 55%, 60%, 65%, 70%, 75%, 80%, 85% or 90% of cells in said composition. In other specific embodiments, the amnion derived adherent cells comprise about, at least, or no more than, 10%, 15%, 20%, 25%, 30%, 35%, 40%, or 45% of cells in said population. In other specific embodiments, at least 25%, 35%, 45%, 50%, 60%, 75%, 85% or more of the cells in said population are not OCT-4⁺.

Cells in an isolated population of amnion derived adherent cells can be combined with a plurality of cells of another type, *e.g.,* with a population of stem cells, in a ratio of about 100,000,000:1, 50,000,000:1, 20,000,000:1, 10,000,000:1, 5,000,000:1, 2,000,000:1, 1,000,000:1, 500,000:1, 200,000:1, 100,000:1, 50,000:1, 20,000:1, 10,000:1, 5,000:1, 2,000:1, 1,000:1, 500:1, 200:1, 100:1, 50:1, 20:1, 10:1, 5:1, 2:1, 1:1; 1:2; 1:5; 1:10; 1:100; 1:200; 1:500; 1:1,000; 1:2,000; 1:5,000; 1:10,000; 1:20,000; 1:50,000; 1:100,000; 1:500,000; 1:1,000,000; 1:2,000,000; 1:5,000,000; 1:10,000,000; 1:20,000,000; 1:50,000,000; or about 1:100,000,000, comparing numbers of total nucleated cells in each population. Cells in an isolated population of amnion derived adherent cells can be combined with a plurality of cells of a plurality of cell types, as well.

### 5.5 GROWTH IN CULTURE

The growth of the amnion derived adherent cells described herein, as for any mammalian cell, depends in part upon the particular medium selected for growth. Under optimum conditions, amnion derived adherent cells typically double in number in approximately 24 hours. During culture, the amnion derived adherent cells described herein adhere to a substrate in culture, *e.g.* the surface of a tissue culture container (*e.g.,* tissue culture dish plastic, fibronectin-coated plastic, and the like) and form a monolayer. Typically, the cells establish in culture within 2-7 days after digestion of the amnion. They proliferate at approximately 0.4 to 1.2 population doublings per day and can undergo at least 30 to 50 population doublings. The cells display a mesenchymal/fibroblastic cell-like phenotype during subconfluence and expansion, and a cuboidal/cobblestone-like appearance at confluence, and proliferation in culture is strongly contact-inhibited. Populations of amnion-derived adherent cells can form embryoid bodies during expansion in culture. Amnion-derived adherent cells may be grown under normoxic or hypoxic conditions, *e.g.,* from about 0.1% O₂ to about 21% O₂, *e.g.,* at about 1, 2, 3, 4, 5, 6, 7, 8, 9, 10,.11, 12, 13, 14, 15, 16, 17, 18, 19, 20 or 21% O₂.

### 5.6 METHODS OF OBTAINING AMNION-DERIVED ADHERENT CELLS

The amnion derived adherent cells, and populations of cells comprising the amnion derived adherent cells, can be produced, *e.g.,* isolated from other cells or cell populations, for example, through particular methods of digestion of amnion tissue, optionally followed by assessment of the resulting cells or cell population for the presence or absence of markers, or combinations of markers, characteristics of amnion derived adherent cells, or by obtaining amnion cells and selecting on the basis of markers characteristic of amnion derived adherent cells.

The amnion derived adherent cells, and isolated populations of cells comprising the amnion derived adherent cells, provided herein can be produced by, *e.g.,* digestion of amnion tissue followed by selection for adherent cells. In one embodiment, for instance, isolated amnion derived adherent cells, or an isolated population of cells comprising amnion derived adherent cells, can be produced by (1) digesting amnion tissue with a first enzyme to dissociate cells from the epithelial layer of the amnion from cells from the mesenchymal layer of the amnion; (2) subsequently digesting the mesenchymal layer of the amnion with a second enzyme to form a single-cell suspension; (3) culturing cells in said single-cell suspension on a tissue culture surface, *e.g.,* tissue culture plastic; and (4) selecting cells that adhere to said surface after a change of medium, thereby producing an isolated population of cells comprising amnion derived adherent cells. In a specific embodiment, said first enzyme is trypsin. In a more specific embodiment, said trypsin is used at a concentration of 0.25% trypsin (w/v), in 5-20, *e.g.,* 10 milliliters solution per gram of amnion tissue to be digested. In another more specific embodiment, said digesting with trypsin is allowed to proceed for about 15 minutes at 37°C and is repeated up to three times. In another specific embodiment, said second enzyme is collagenase. In a more specific embodiment, said collagenase is used at a concentration between 50 and 500 U/L in 5 mL per gram of amnion tissue to be digested. In another more specific embodiment, said digesting with collagenase is allowed to proceed for about 45-60 minutes at 37°C. In another specific embodiment, the single-cell suspension formed after digestion with collagenase is filtered through, *e.g.,* a 75 µM - 150 µM filter between step (2) and step (3). In another specific embodiment, said first enzyme is trypsin, and said second enzyme is collagenase.

An isolated population of cells comprising amnion derived adherent cells can, in another embodiment, be obtained by selecting cells from amnion, *e.g.,* cells obtained by digesting amnion tissue as described elsewhere herein, that display one or more characteristics of an amnion derived adherent cell. In one embodiment, for example, a cell population is produced by a method comprising selecting identifying cells that are (a) negative for OCT-4, as determinable by RT-PCR, and (b) positive for one or more of VEGFR2/KDR, CD9, CD54, CD 105, CD200, as determinable or selectable by immunolocalization, *e.g.,* flow cytometry; and isolating said cells from other cells to form a cell population. In a specific embodiment, said amnion cells are additionally VE-cadherin⁻. In a specific embodiment, a cell population is produced by selecting amnion cells that are (a) negative for OCT-4, as determinable by RT-PCR, and VE-cadherin, as determinable by immunolocalization, *e.g.,* flow cytometry, and (b) positive for each of VEGFR2/KDR, CD9, CD54, CD105, CD200, as determinable by immunolocalization, *e.g.,* flow cytometry; and isolating said cells from other cells to form a cell population. In certain embodiments, selection by immunolocalization, *e.g.,* flow cytometry, is performed before selection by RT-PCR. In another specific embodiment, said selecting comprises selecting cells that do not express cellular marker CD34 after culture for 4 to 21 days in the presence of 1 to 100 ng/mL VEGF.

In another embodiment, for example, a cell population is produced by a method comprising selecting amnion cells that are adherent to tissue culture plastic and are OCT-4⁻, as determinable by RT-PCR, and VEGFR1/Flt-1⁺ and VEGFR2/KDR⁺, as determinable by immunolocalization, *e.g.,* flow cytometry, and isolating said cells from other cells to form a cell population. In a specific embodiment, a cell population is produced by a method comprising selecting amnion cells that are OCT-4⁻, as determinable by RT-PCR, and VEGFR1/Flt-1⁺, VEGFR2/KDR⁺, and HLA-G⁻, as determinable by immunolocalization, *e.g.,* flow cytometry. In another specific embodiment, said cell population is produced by selecting amnion cells that are additionally one or more, or all, of CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻, and/or CXCR4⁻ (chemokine (C-X-C motif) receptor 4) as determinable by immunolocalization, *e.g.,* flow cytometry, and isolating the cells from cells that do not display one or more of these characteristics. In another specific embodiment, said cell population is produced by selecting amnion cells that are additionally VE-cadherin⁺ as determinable by immunolocalization, *e.g.,* flow cytometry, and isolating the cells from cells that are VE-cadherin⁺. In another specific embodiment, said cell population is produced by selecting amnion cells that are additionally CD105⁺ and CD200⁺ as determinable by immunolocalization, *e.g.,* flow cytometry, and isolating the cells from cells that are CD105⁻ or CD200⁻. In another specific embodiment, said cell does not express CD34 as detected by immunolocalization, *e.g.,* flow cytometry, after exposure to 1 to 100 ng/mL VEGF for 4 to 21 days.

In the selection of cells, it is not necessary to test an entire population of cells for characteristics specific to amnion derived adherent cells. Instead, one or more aliquots of cells (*e.g*., about 0.5% - 2%) of a population of cells may be tested for such characteristics, and the results can be attributed to the remaining cells in the population.

Selected cells can be confirmed to be the amnion derived adherent cells provided herein by culturing a sample of the cells (*e.g.,* about 10⁴ to about 10⁵ cells) on a substrate, *e.g.,* MATRIGEL™, for 4 to 14, *e.g.,* 7, days in the presence of VEGF (*e.g.,* about 50 ng/mL), and visually inspecting the cells for the appearance of sprouts and/or cellular networks.

Amnion derived adherent cells can be selected by the above markers using any method known in the art of cell selection. For example, the adherent cells can be selected using an antibody or antibodies to one or more cell surface markers, for example, in immunolocalization, *e.g.,* flow cytometry or FACS. Selection can be accomplished using antibodies in conjunction with magnetic beads. Antibodies that are specific for certain markers are known in the art and are available commercially, *e.g.,* antibodies to CD9 (Abcam); CD54 (Abcam); CD105 (Abcam; BioDesign International, Saco, ME, *etc.*); CD200 (Abcam) cytokeratin (SigmaAldrich). Antibodies to other markers are also available commercially, *e.g.,* CD34, CD38 and CD45 are available from, *e.g.,* StemCell Technologies or BioDesign International. Primers to OCT-4 sequences suitable for RT-PCR can be obtained commercially, *e.g.,* from Millipore or Invitrogen, or can be readily derived from the human sequence in GenBank Accession No. DQ486513.

Detailed methods of obtaining placenta and amnion tissue from placenta, and treating such tissue in order to obtain amnion derived adherent cells, are provided below.

### 5.6.1 Cell Collection Composition

Generally, cells can be obtained from amnion from a mammalian placenta, *e.g.,* a human placenta, using a physiologically-acceptable solution, *e.g.,* a cell collection composition. In certain embodiments, the cell collection composition prevents or suppresses apoptosis, prevents or suppresses cell death, lysis, decomposition and the like. A cell collection composition is described in detail in related U.S. Patent Application Publication No. 2007/0190042, entitled "Improved Medium for Collecting Placental Stem Cells and Preserving Organs," the disclosure of which is incorporated herein by reference in its entirety.

The cell collection composition can comprise any physiologically-acceptable solution suitable for the collection and/or culture of amnion derived adherent cells, for example, a saline solution (*e.g*., phosphate-buffered saline, Kreb's solution, modified Kreb's solution, Eagle's solution, 0.9% NaCl. *etc*.), a culture medium (*e.g.,* DMEM, H.DMEM, *etc*.), and the like, with or without the addition of a buffering component, *e.g.,* 4-(2-hydroxyethyl)-1-piperazineethanesulfonic acid (HEPES).

The cell collection composition can comprise one or more components that tend to preserve cells, *e.g.,* amnion derived adherent cells, that is, prevent the cells from dying, or delay the death of the cells, reduce the number of cells in a population of cells that die, or the like, from the time of collection to the time of culturing. Such components can be, *e.g.,* an apoptosis inhibitor (*e.g.,* a caspase inhibitor or JNK inhibitor); a vasodilator *(e.g.,* magnesium sulfate, an antihypertensive drug, atrial natriuretic peptide (ANP), adrenocorticotropin, corticotropin-releasing hormone, sodium nitroprusside, hydralazine, adenosine triphosphate, adenosine, indomethacin or magnesium sulfate, a phosphodiesterase inhibitor, *etc.*); a necrosis inhibitor (*e.g*., 2-(1H-Indol-3-yl)-3-pentylamino-maleimide, pyrrolidine dithiocarbamate, or clonazepam); a TNF-α inhibitor; and/or an oxygen-carrying perfluorocarbon (*e.g.,* perfluorooctyl bromide, perfluorodecyl bromide, *etc.*).

The cell collection composition can comprise one or more tissue-degrading enzymes, *e.g*., a metalloprotease, a serine protease, a neutral protease, an RNase, or a DNase, or the like. Such enzymes include, but are not limited to, collagenases (*e.g*., collagenase I, II, III or IV, a collagenase from *Clostridium histolyticum, etc.*); dispase, thermolysin, elastase, trypsin, LIBERASE™, hyaluronidase, and the like. The use of cell collection compositions comprising tissue-digesting enzymes is discussed in more detail below.

The cell collection composition can comprise a bacteriocidally or bacteriostatically effective amount of an antibiotic. In certain non-limiting embodiments, the antibiotic is a macrolide (*e.g.,* tobramycin), a cephalosporin (*e.g.,* cephalexin, cephradine, cefuroxime, cefprozil, cefaclor, cefixime or cefadroxil), a clarithromycin, an erythromycin, a penicillin (*e.g.,* penicillin V) or a quinolone (*e.g.,* ofloxacin, ciprofloxacin or norfloxacin), a tetracycline, a streptomycin, *etc.* In a particular embodiment, the antibiotic is active against Gram(+) and/or Gram(-) bacteria, *e.g., Pseudomonas aeruginosa, Staphylococcus aureus,* and the like.

The cell collection composition can also comprise one or more of the following compounds: adenosine (about 1 mM to about 50 mM); D-glucose (about 20 mM to about 100 mM); magnesium ions (about 1 mM to about 50 mM); a macromolecule of molecular weight greater than 20,000 daltons, in one embodiment, present in an amount sufficient to maintain endothelial integrity and cellular viability (*e.g*., a synthetic or naturally occurring colloid, a polysaccharide such as dextran or a polyethylene glycol present at about 25 g/l to about 100 g/l, or about 40 g/l to about 60 g/l); an antioxidant (*e.g*., butylated hydroxyanisole, butylated hydroxytoluene, glutathione, vitamin C or vitamin E present at about 25 µM to about 100 µM); a reducing agent (*e.g*., N-acetylcysteine present at about 0.1 mM to about 5 mM); an agent that prevents calcium entry into cells (*e.g*., verapamil present at about 2 µM to about 25 µM); nitroglycerin (*e.g*., about 0.05 g/L to about 0.2 g/L); an anticoagulant, in one embodiment, present in an amount sufficient to help prevent clotting of residual blood (*e.g.,* heparin or hirudin present at a concentration of about 1000 units/l to about 100,000 units/l); or an amiloride containing compound (*e.g*., amiloride, ethyl isopropyl amiloride, hexamethylene amiloride, dimethyl amiloride or isobutyl amiloride present at about 1.0 µM to about 5 µM).

The amnion derived adherent cells described herein can also be collected, *e.g.,* during and after digestion as described below, into a simple physiologically-acceptable buffer, *e.g.,* phosphate-buffered saline, a 0.9% NaCl solution, cell culture medium, or the like.

### 5.6.2 Collection and Handling of Placenta

Generally, a human placenta is recovered shortly after its expulsion after birth, or after, *e.g.,* Caesarian section. In a preferred embodiment, the placenta is recovered from a patient after informed consent and after a complete medical history of the patient is obtained and is associated with the placenta. Preferably, the medical history continues after delivery. Such a medical history can be used to coordinate subsequent use of the placenta or cells harvested therefrom. For example, human amnion derived adherent cells can be used, in light of the medical history, for personalized medicine for the infant, or a close relative, associated with the placenta, or for parents, siblings, or other relatives of the infant.

Prior to recovery of amnion derived adherent cells, the umbilical cord blood and placental blood are removed. In certain embodiments, after delivery, the cord blood in the placenta is recovered. The placenta can be subjected to a conventional cord blood recovery process. Typically a needle or cannula is used, with the aid of gravity, to exsanguinate the placenta (*see, e.g.,* Anderson, U.S. Patent No. 5,372,581; Hessel et al., U.S. Patent No. 5,415,665). The needle or cannula is usually placed in the umbilical vein and the placenta can be gently massaged to aid in draining cord blood from the placenta. Such cord blood recovery may be performed commercially, *e.g*., LifeBank USA, Cedar Knolls, N.J., ViaCord, Cord Blood Registry and Cryocell. Preferably, the placenta is gravity drained without further manipulation so as to minimize tissue disruption during cord blood recovery.

Typically, a placenta is transported from the delivery or birthing room to another location, *e.g.*, a laboratory, for recovery of cord blood and collection of cells by, *e.g*., tissue dissociation. The placenta is preferably transported in a sterile, thermally insulated transport device (maintaining the temperature of the placenta between 20-28°C), for example, by placing the placenta, with clamped proximal umbilical cord, in a sterile zip-lock plastic bag, which is then placed in an insulated container. In another embodiment, the placenta is transported in a cord blood collection kit substantially as described in United States Patent No. 7,147,626. Preferably, the placenta is delivered to the laboratory four to twenty-four hours following delivery. In certain embodiments, the proximal umbilical cord is clamped, preferably within 4-5 cm (centimeter) of the insertion into the placental disc prior to cord blood recovery. In other embodiments, the proximal umbilical cord is clamped after cord blood recovery but prior to further processing of the placenta.

The placenta, prior to cell collection, can be stored under sterile conditions and at a temperature of, *e.g*., 4 to 25°C (centigrade), *e.g.*, at room temperature. The placenta may be stored for, *e.g*., a period of for zero to twenty-four hours, up to forty-eight hours, or longer than forty eight hours, prior to perfusing the placenta to remove any residual cord blood. In one embodiment, the placenta is harvested from between about zero hours to about two hours post-expulsion. The placenta can be stored in an anticoagulant solution at a temperature of, *e.g*., 4 to 25°C (centigrade). Suitable anticoagulant solutions are well known in the art. For example, a solution of sodium citrate, heparin or warfarin sodium can be used. In a preferred embodiment, the anticoagulant solution comprises a solution of heparin (*e.g*., 1% w/w in 1:1000 solution). The exsanguinated placenta is preferably stored for no more than 36 hours before cells are collected.

See, e.g., U.S. Patent No. 7,638,141, the disclosure of which is hereby incorporated by reference in its entirety, for additional information regarding collection and handling of placenta.

### 5.6.3 Physical Disruption and nzymatic Digestion of Amnion Tissue

In one embodiment, the amnion is separated from the rest of the placenta, *e.g*., by blunt dissection, *e.g*., using the fingers. The amnion can be dissected, *e.g*., into parts or tissue segments, prior to enzymatic digestion and adherent cell recovery. Amnion derived adherent cells can be obtained from a whole amnion, or from a small segment of amnion, *e.g*., a segment of amnion that is about 1, 2, 3, 4, 5, 6, 7, 8, 9, 10, 20, 30, 40, 50, 60, 70, 80, 90, 100, 200, 300, 400, 500, 600, 700, 800, 900 or about 1000 square millimeters in area.

Amnion derived adherent cells can generally be collected from a placental amnion or a portion thereof, at any time within about the first three days post-expulsion, but preferably between about 0 hours and 48 hours after expulsion, or about 8 hours and about 18 hours post-expulsion.

AMDACs can, for example, be isolated using a specific two-step isolation method comprising digestion with trypsin followed by digestion with collagenase. For example, provided herein is a method of isolating amnion derived adherent cells comprising digesting an amniotic membrane or portion thereof with trypsin such that epithelial cells are released from said amniotic membrane; removing the amniotic membrane or portion thereof from said epithelial cells; further digesting the amniotic membrane or portion thereof with collagenase. In a specific embodiment, digestion of the amniotic membrane or portion thereof with trypsin is repeated at least once. In another specific embodiment, digestion of the amniotic membrane or portion thereof with collagenase is repeated at least once. In another specific embodiment, the trypsin is at about 0.1%-1.0% (final concentration). In a more specific embodiment, the trypsin is at about 0.25% (final concentration). In another specific embodiment, the collagenase is at about 50 U/mL to about 1000 U/mL (final concentration). In a more specific embodiment, the collagenase is at about 125 U/mL (final concentration).

In one embodiment, for example, amnion derived adherent cells can be obtained as follows. The amniotic membrane is isolated from the placenta via, *e.g*., dissection, then cut into segments approximately 0.1" x 0.1" to about 5" x 5", *e.g*., 2" x 2", in size. The epithelial monolayer is removed from the fetal side of the amniotic membrane by trypsinization, *e.g*., triple trypsinization as follows. The segments of amniotic membrane are placed into a container with warm (*e.g*., about 20°C to about 37°C) trypsin-EDTA solution (0.25%). The volume of the trypsin solution can range from about 5 mL per gram of amniotic membrane to about 50 mL per gram of amniotic membrane. The container is agitated for about 5 minutes to about 30 minutes, *e.g*., 15 minutes, while maintaining the temperature constant. The segments of amniotic membrane are then separated from the trypsin solution by any appropriate method, such as manually removing the amnion segments, or by filtration. The trypsinization step can be repeated at least one more time. In one embodiment, the trypsinization step is repeated twice (for triple trypsinization) or three times (for quadruple trypsinization).

In one embodiment, upon completion of the final trypsinization, the segments of amniotic membrane are placed back into warm (*e.g*., about 20°C to about 37°C) trypsin neutralization solution (*e.g*., at a volume of about 5 mL per gram of amniotic membrane to about 50 mL per gram of amniotic membrane), such as phosphate-buffered saline (PBS)/10% fetal bovine serum (FBS), PBS/5% FBS or PBS/3% FBS. The container is agitated for about 5 seconds to about 30 minutes, *e.g*., 5, 10 or 15 minutes. The segments of amniotic membrane are then separated from the trypsin neutralization solution by any appropriate method, such as manually removing the amnion segments, or by filtration. The segments of amniotic membrane are placed into the container filled with warm (*e.g*., about 20°C to about 37°C) PBS, pH 7.2 solution (*e.g*., at a volume of about 5 mL per gram of amniotic membrane to about 50 mL per gram of amniotic membrane). The container is agitated for about 5 seconds to about 30 minutes, *e.g*., 5, 10 or 15 minutes. The amniotic membrane segments are then separated from the PBS as described above.

The segments of amniotic membrane are then placed into warm (*e.g*., about 20°C to about 37°C) digestion solution. The volume of digestion solution can range from about 5 mL per gram of amnion to about 50 mL per gram of amnion. Digestion solutions comprise digestion enzymes in an appropriate culture medium, such as DMEM. Typical digestion solutions include collagenase type I (about 50 U/mL to about 500 U/mL). Digestion solutions for this stage of the process do not generally comprise trypsin. Agitation is generally at 37°C until amnion digestion is substantially complete, as evidenced, *e.g*., by complete dissolution of the amniotic membrane yielding a homogeneous suspension (approximately 10 minutes to about 90 minutes). Warm PBS/5% FBS is then added at a ratio of about 1 mL per gram of amniotic tissue to about 50 mL per gram of amniotic tissue and agitated for about 2 minutes to about 5 minutes. The cell suspension is then filtered to remove any un-digested tissue using, *e.g*., a 40 µm to 100 µm filter. The cells are suspended in warm PBS (about 1 mL to about 500 mL), and then centrifuged at 200 x g to about 400 x g for about 5 minutes to about 30 minutes, *e.g*. 300 x g for about 15 minutes at 20°C. After centrifugation, the supernatant is removed and the cells are resuspended in a suitable culture medium. The cell suspension can be filtered (40 µm to 70 µm filter) to remove any remaining undigested tissue, yielding a single cell suspension. The remaining undigested amnion, in this embodiment, can be discarded.

In this embodiment, cells in suspension are collected and cultured as described elsewhere herein to produce isolated amnion derived adherent cells, and populations of such cells. For example, in one embodiment, the cells in suspension can be cultured and amnion derived adherent cells can be separated from non-adherent cells in said culture to produce an enriched population of amnion derived adherent cells. In more specific embodiments, at least 50%, 55%, 60%, 65%, 70%, 75%, 80%, 85%, 90%, 95%, 98% or 99% of cells in said enriched population of amnion derived adherent cells are said amnion derived adherent cells.

In any of the digestion protocols herein, the cell suspension obtained by digestion can be filtered, *e.g*., through a filter comprising pores from about 50 µm to about 150 µm, *e.g*., from about 75 µm to about 125 µm. In a more specific embodiment, the cell suspension can be filtered through two or more filters, *e.g*., a 125 µm filter and a 75 µm filter.

In conjunction with any of the methods described herein, AMDACs can be isolated from the cells released during digestion by selecting cells that express one or more characteristics of AMDACs, as described in Section 5.3, above.

In one embodiment, AMDACs can be isolated using, in order, a first enzyme and a second enzyme, wherein the first enzyme used in the method is not collagenase, and wherein the second enzyme used in the method is not trypsin. In another embodiment, the digestion step used to isolate AMDACs does not use a combination of any two or more of collagenase, dispase or hyaluronidase. In another embodiment, the AMDACs are not isolated via explant culturing to allow the cells to be detected by growth, replication, or migration out of the explants.

In another embodiment, deoxyribonuclease (DNase) is not used during the isolation of AMDACs. For example, in certain embodiments, DNase is not used following the collagenase digestion step of the isolation.

### 5.6.4 Isolation, Sorting, and Characterization of Amnion Derived Adherent Cells

Cell pellets can be resuspended in fresh cell collection composition, as described above, or a medium suitable for cell maintenance, *e.g*., Dulbecco's Modified Eagle's Medium (DMEM); Iscove's Modified Dulbecco's Medium (IMDM), *e.g.* IMDM serum-free medium containing 2U/mL heparin and 2 mM EDTA (GibcoBRL, NY); a mixture of buffer (*e.g*. PBS, HBSS) with FBS (*e.g.* 2% v/v); or the like.

Amnion derived adherent cells that have been cultured, *e.g*., on a surface, *e.g*., on tissue culture plastic, with or without additional extracellular matrix coating such as fibronectin, can be passaged or isolated by differential adherence. For example, a cell suspension obtained as described in Section 5.6.3, above, can be cultured, *e.g*., for 3-7 days in culture medium on tissue culture plastic. During culture, a plurality of cells in the suspension adhere to the culture surface, and nonadherent cells are removed during medium exchange.

The number and type of cells collected from amnion can be monitored, for example, by measuring changes in morphology and cell surface markers using standard cell detection techniques such as immunolocalization, *e.g*., flow cytometry, cell sorting, immunocytochemistry (*e.g*., staining with tissue specific or cell-marker specific antibodies) fluorescence activated cell sorting (FACS), magnetic activated cell sorting (MACS), by examination of the morphology of cells using light or confocal microscopy, and/or by measuring changes in gene expression using techniques well known in the art, such as PCR and gene expression profiling. These techniques can be used, too, to identify cells that are positive for one or more particular markers. For example, using one or more antibodies to CD34, one can determine, using the techniques above, whether a cell comprises a detectable amount of CD34; if so, the cell is CD34⁺.

Amnion derived adherent cells can be isolated by Ficoll separation, *e.g*., Ficoll gradient centrifugation. Such centrifugation can follow any standard protocol for centrifugation speed, etc. In one embodiment, for example, cells recovered after digestion of the amnion are separated using a Ficoll gradient by centrifugation at 5000 x g for 15 minutes at room temperature and cell layers of interest are collected for further processing.

Amnion-derived cells, *e.g*., cells that have been isolated by Ficoll separation, differential adherence, or a combination of both can be sorted using a fluorescence activated cell sorter (FACS). Fluorescence activated cell sorting (FACS) is a well-known method for separating particles, including cells, based on the fluorescent properties of the particles *(see, e.g*., Kamarch, 1987, Methods Enzymol, 151:150-165). Laser excitation of fluorescent moieties in the individual particles results in a small electrical charge allowing electromagnetic separation of positive and negative particles from a mixture. In one embodiment, cell surface marker-specific antibodies or ligands are labeled with distinct fluorescent labels. Cells are processed through the cell sorter, allowing separation of cells based on their ability to bind to the antibodies used. FACS sorted particles may be directly deposited into individual wells of 96-well or 384-well plates to facilitate separation and cloning.

In one sorting scheme, cells from placenta, *e.g*., amnion derived adherent cells, can be sorted on the basis of expression of the markers CD49f, VEGFR2/KDR, and/or Flt-1/VEGFR1. Preferably the cells are identified as being OCT-4⁻, *e.g*., by determining the expression of OCT-4 by RT-PCR in a sample of the cells, wherein the cells are OCT-4⁻ if the cells in the sample fail to show detectable production of mRNA for OCT-4 after 30 cycles. For example, cells from amnion that are VEGFR2/KDR⁺ and VEGFR1/Flt-1⁺ can be sorted from cells that are one or more of VEGFR2/KDR⁻, and VEGFR1/Flt-1⁺, CD9⁺, CD54⁺, CD105⁺, CD200⁺, and/or VE-cadherin⁻. In a specific embodiment, amnion-derived, tissue culture plastic-adherent cells that are one or more of CD49f⁺, VEGFR2/KDR⁺, CD9⁺, CD54⁺, CD105⁺, CD200⁺, and/or VE-cadherin⁻, or cells that are VEGFR2/KDR⁺, CD9⁺, CD54⁺, CD105⁺, CD200⁺, and VE-cadherin⁻, are sorted away from cells not expressing one or more of such marker(s), and selected. In another specific embodiment, CD49f⁺, VEGFR2/KDR⁺, VEGFR1/Flt-1⁺ cells that are additionally one or more, or all, of CD31⁺, CD34⁺, CD45⁺, CD133⁻, and/or Tie-2⁺ are sorted from cells that do not display one or more, or any, of such characteristics. In another specific embodiment, VEGFR2/KDR⁺, VEGFR1/Flt-1⁺ cells that are additionally one or more, or all, of CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻, and/or CXCR4⁻, are sorted from cells that do not display one or more, or any, of such characteristics.

Selection for amnion derived adherent cells can be performed on a cell suspension resulting from digestion, or on isolated cells collected from digestate, *e.g*., by centrifugation or separation using flow cytometry. Selection by expressed markers can be accomplished alone or, *e.g*., in connection with procedures to select cells on the basis of their adherence properties in culture. For example, an adherence selection can be accomplished before or after sorting on the basis of marker expression.

With respect to antibody-mediated detection and sorting of amnion cells, any antibody, specific for a particular marker, can be used, in combination with any fluorophore or other label suitable for the detection and sorting of cells (*e.g*., fluorescence-activated cell sorting). Antibody/fluorophore combinations to specific markers include, but are not limited to, fluorescein isothiocyanate (FITC) conjugated monoclonal antibodies against CD105 (available from R&D Systems Inc., Minneapolis, Minnesota); phycoerythrin (PE) conjugated monoclonal antibodies against CD200 (BD Biosciences Pharmingen); VEGFR2/KDR-Biotin (CD309, Abcam), and the like. Antibodies to any of the markers disclosed herein can be labeled with any standard label for antibodies that facilitates detection of the antibodies, including, *e.g*., horseradish peroxidase, alkaline phosphatase, β-galactosidase, acetylcholinesterase streptavidin/biotin, avidin/biotin, umbelliferone, fluorescein, fluorescein isothiocyanate (FITC), rhodamine, dichlorotriazinylamine fluorescein, dansyl chloride or phycoerythrin (PE), luminol, luciferase, luciferin, and aequorin, and examples of suitable radioactive material include ¹²⁵I, ¹³¹I ³⁵S or ³H.

Amnion derived adherent cells can be labeled with an antibody to a single marker and detected and/sorted based on the single marker, or can be simultaneously labeled with multiple antibodies to a plurality of different markers and sorted based on the plurality of markers.

In another embodiment, magnetic beads can be used to separate cells, *e.g*., to separate the amnion derived adherent cells described herein from other amnion cells. The cells may be sorted using a magnetic activated cell sorting (MACS) technique, a method for separating particles based on their ability to bind magnetic beads (0.5-100 µm diameter). A variety of useful modifications can be performed on the magnetic microspheres, including covalent addition of antibody that specifically recognizes a particular cell surface molecule or hapten. The beads are then mixed with the cells to allow binding. Cells are then passed through a magnetic field to separate out cells having the specific cell surface marker. In one embodiment, these cells can then isolated and re-mixed with magnetic beads coupled to an antibody against additional cell surface markers. The cells are again passed through a magnetic field, isolating cells that bound both the antibodies. Such cells can then be diluted into separate dishes, such as microtiter dishes for clonal isolation.

Amnion derived adherent cells can be assessed for viability, proliferation potential, and longevity using standard techniques known in the art, such as trypan blue exclusion assay, fluorescein diacetate uptake assay, propidium iodide uptake assay (to assess viability); and thymidine uptake assay or MTT cell proliferation assay (to assess proliferation). Longevity may be determined by methods well known in the art, such as by determining the maximum number of population doubling in an extended culture.

Amnion derived adherent cells, can also be separated from other placental or amnion cells using other techniques known in the art, *e.g*., selective growth of desired cells (positive selection), selective destruction of unwanted cells (negative selection); separation based upon differential cell agglutinability in the mixed population as, for example, with soybean agglutinin; freeze-thaw procedures; filtration; conventional and zonal centrifugation; centrifugal elutriation (counter-streaming centrifugation); unit gravity separation; countercurrent distribution; electrophoresis; and the like.

### 5.7 CULTURE OF AMNION DERIVED ADHERENT CELLS

### 5.7.1 Culture Media

Isolated amnion derived adherent cells, or populations of such cells, can be used to initiate, or seed, cell cultures. Cells are generally transferred to sterile tissue culture vessels either uncoated or coated with extracellular matrix or biomolecules such as laminin, collagen (*e.g*., native or denatured), gelatin, fibronectin, ornithine, vitronectin, and extracellular membrane protein (*e.g*., MATRIGEL™ (BD Discovery Labware, Bedford, Mass.)).

AMDACs can, for example, be established in media suitable for the culture of stem cells. Establishment media can, for example, include EGM-2 medium (Lonza), DMEM + 10% FBS, or medium comprising 60% DMEM-LG (Gibco), 40% MCDB-201(Sigma), 2% fetal calf serum (FCS) (Hyclone Laboratories), 1X insulin-transferrin-selenium (ITS), 1X lenoleic-acid-bovine-serum-albumin (LA-BSA), 10⁻⁹ M dexamethasone (Sigma), 10⁻⁴M ascorbic acid 2-phosphate (Sigma), epidermal growth factor (EGF) 10 ng/ml (R&D Systems), platelet derived-growth factor (PDGF-BB) 10 ng/ml (R&D Systems), and 100 U penicillin/1000 U streptomycin (referred to herein as "standard medium").

Amnion derived adherent cells can be cultured in any medium, and under any conditions, recognized in the art as acceptable for the culture of cells, *e.g*., adherent placental stem cells. Preferably, the culture medium comprises serum. In various embodiments, media for the culture or subculture of AMDACs includes STEMPRO® (Invitrogen), MSCM-sf (ScienCell, Carlsbad, CA), MESENCULT®-ACF medium (StemCell Technologies, Vancouver, Canada), standard medium, standard medium lacking EGF, standard medium lacking PDGF, DMEM + 10% FBS, EGM-2 (Lonza), EGM-2MV (Lonza), 2%, 10% and 20% ES media, ES-SSR medium, or α-MEM-20%FBS. Medium acceptable for the culture of amnion derived adherent cells includes, *e.g*., DMEM, IMDM, DMEM (high or low glucose), Eagle's basal medium, Ham's F10 medium (F10), Ham's F-12 medium (F12), Iscove's modified Dulbecco's medium, Mesenchymal Stem Cell Growth Medium (MSCGM Lonza), ADVANCESTEM™ Medium (Hyclone), KNOCKOUT™ DMEM (Invitrogen), Leibovitz's L-15 medium, MCDB, DMEM/F12, RPMI 1640, advanced DMEM (Gibco), DMEM/MCDB201 (Sigma), and CELL-GRO FREE, or the like. In various embodiments, for example, DMEM-LG (Dulbecco's Modified Essential Medium, low glucose)/MCDB 201 (chick fibroblast basal medium) containing ITS (insulin-transferrin-selenium), LA+BSA (linoleic acid-bovine serum albumin), dextrose, L-ascorbic acid, PDGF, EGF, IGF-1, and penicillin/streptomycin; DMEM-HG (high glucose) comprising about 2 to about 20%, *e.g*., about 10%, fetal bovine serum (FBS; *e.g.* defined fetal bovine serum, Hyclone, Logan Utah); DMEM-HG comprising about 2 to about 20%, *e.g*., about 15%, FBS; IMDM (Iscove's modified Dulbecco's medium) comprising about 2 to about 20%, *e.g*., about 10%, FBS, about 2 to about 20%, *e.g*., about 10%, horse serum, and hydrocortisone; M199 comprising about 2 to about 20%, *e.g*., about 10%, FBS, EGF, and heparin; α-MEM (minimal essential medium) comprising about 2 to about 20%, *e.g*., about 10%, FBS, GLUTAMAX™ and gentamicin; DMEM comprising 10% FBS, GLUTAMAX™ and gentamicin; DMEM-LG comprising about 2 to about 20%, *e.g*., about 15%, (v/v) fetal bovine serum (*e.g*., defined fetal bovine serum, Hyclone, Logan Utah), antibiotics/antimycotics (*e.g*., penicillin at about 100 Units/milliliter, streptomycin at 100 micrograms/milliliter, and/or amphotericin B at 0.25 micrograms/milliliter (Invitrogen, Carlsbad, Calif.)), and 0.001% (v/v) β-mercaptoethanol (Sigma, St. Louis Mo.); KNOCKOUT™-DMEM basal medium supplemented with 2 to 20% FBS, non-essential amino acid (Invitrogen), beta-mercaptoethanol, KNOCKOUT™ basal medium supplemented with KNOCKOUT™ Serum Replacement, alpha-MEM comprising 2 to 20% FBS, EBM2™ basal medium supplemented with EGF, VEGF, bFGF, R3-IGF-1, hydrocortisone, heparin, ascorbic acid, FBS, gentamicin), or the like.

The culture medium can be supplemented with one or more components including, for example, serum (*e.g.*, FCS or FBS, *e.g*., about 2-20% (v/v); equine (horse) serum (ES); human serum (HS)); beta-mercaptoethanol (BME), preferably about 0.001% (v/v); one or more growth factors, for example, platelet-derived growth factor (PDGF), epidermal growth factor (EGF), basic fibroblast growth factor (bFGF), insulin-like growth factor-1 (IGF-1), leukemia inhibitory factor (LIF), vascular endothelial growth factor (VEGF), and erythropoietin (EPO); amino acids, including L-valine; and one or more antibiotic and/or antimycotic agents to control microbial contamination, such as, for example, penicillin G, streptomycin sulfate, amphotericin B, gentamicin, and nystatin, either alone or in combination.

Amnion derived adherent cells (AMDACs) can be cultured in standard tissue culture conditions, *e.g*., in tissue culture dishes or multiwell plates. The cells can also be cultured using a hanging drop method. In this method, the cells are suspended at about 1 x 10⁴ cells per mL in about 5 mL of medium, and one or more drops of the medium are placed on the inside of the lid of a tissue culture container, *e.g*., a 100 mL Petri dish. The drops can be, *e.g*., single drops, or multiple drops from, *e.g*., a multichannel pipetter. The lid is carefully inverted and placed on top of the bottom of the dish, which contains a volume of liquid, *e.g*., sterile PBS sufficient to maintain the moisture content in the dish atmosphere, and the cells are cultured. AMDACs can also be cultured in standard or high-volume or high-throughput culture systems, such as T-flasks, Corning HYPERFLASK®, Cell Factories (Nunc), 1-, 2-, 4-, 10 or 40-Tray Cell stacks, and the like.

In one embodiment, amnion derived adherent cells are cultured in the presence of a compound that acts to maintain an undifferentiated phenotype in the cells. In a specific embodiment, the compound is a substituted 3,4-dihydropyridimol[4,5-d]pyrimidine. In a more specific embodiment, the compound is a compound having the following chemical structure: 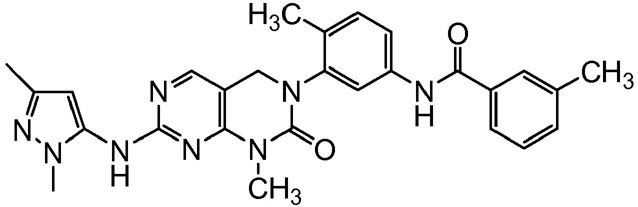 The compound can be contacted with an amnion derived adherent cell, or population of such cells, at a concentration of, for example, between about 1 µM to about 10 µM.

### 5.7.2 Expansion and Proliferation of Amnion Derived Adherent Cells

Once an isolated amnion derived adherent cell, or isolated population of such cells (*e.g*., amnion derived adherent cells, or population of such cells separated from at least 50% of the amnion cells with which the cell or population of cells is normally associated *in vivo*), the cells can be proliferated and expanded *in vitro*. For example, a population of adherent cells or amnion derived adherent cells can be cultured in tissue culture containers, *e.g*., dishes, flasks, multiwell plates, or the like, for a sufficient time for the cells to proliferate to 40-70% confluence, that is, until the cells and their progeny occupy 40-70% of the culturing surface area of the tissue culture container.

Amnion derived adherent cells can be seeded in culture vessels at a density that allows cell growth. For example, the cells may be seeded at low density (*e.g*., about 400 to about 6,000 cells/cm²) to high density (*e.g*., about 20,000 or more cells/cm²). In a preferred embodiment, the cells are cultured at about 0% to about 5% by volume CO₂ in air. In some preferred embodiments, the cells are cultured at about 0.1% to about 25% O₂ in air, preferably about 5% to about 20% O₂ in air. The cells are preferably cultured at about 25°C to about 40°C, preferably at about 37°C.

The cells are preferably cultured in an incubator. During culture, the culture medium can be static or can be agitated, for example, during culture using a bioreactor. Amnion derived adherent cells preferably are grown under low oxidative stress (*e.g*., with addition of glutathione, ascorbic acid, catalase, tocopherol, N-acetylcysteine, or the like).

Although the amnion-derived adherent cells may be grown to confluence, the cells are preferably not grown to confluence. For example, once 40%-70% confluence is obtained, the cells may be passaged. For example, the cells can be enzymatically treated, *e.g*., trypsinized, using techniques well-known in the art, to separate them from the tissue culture surface. After removing the cells by pipetting and counting the cells, about 20,000-100,000 cells, preferably about 50,000 cells, or about 400 to about 6,000 cells/cm², can be passaged to a new culture container containing fresh culture medium. Typically, the new medium is the same type of medium from which the cells were removed. The amnion derived adherent cells can be passaged at least 1, 2, 3, 4, 5, 6, 7, 8, 9, 10, 11, 12, 13, 14, 15, 16, 17, 18, 19 or 20 times, or more. AMDACs can be doubled in culture at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or at least 50 times, or more.

### 5.8 COMPOSITIONS COMPRISING AMNION DERIVED ADHERENT CELLS

### 5.8.1 Pharmaceutical Compositions Comprising Amnion Derived Adherent Cells

In certain embodiments, amnion derived adherent cells are contained within, or are components of, a pharmaceutical composition. The cells can be prepared in a form that is easily administrable to an individual, *e.g*., amnion derived adherent cells that are contained within a container that is suitable for medical use. Such a container can be, for example, a syringe, sterile plastic bag, flask, vial, jar, or other container from which the amnion derived adherent cell population can be easily dispensed. For example, the container can be a blood bag or other plastic, medically-acceptable bag suitable for the intravenous administration of a liquid to a recipient. The container, in certain embodiments, is one that allows for cryopreservation of the cells. The cells in the compositions, *e.g*., pharmaceutical compositions, provided herein, can comprise amnion derived adherent cells derived from a single donor, or from multiple donors. The cells can be completely HLA-matched to an intended recipient, or partially or completely HLA-mismatched.

Thus, in one embodiment, amnion derived adherent cells in the compositions provided herein are administered to an individual in need thereof in the form of a composition comprising amnion derived adherent cells in a container. In another specific embodiment, the container is a bag, flask, vial or jar. In more specific embodiment, said bag is a sterile plastic bag. In a more specific embodiment, said bag is suitable for, allows or facilitates intravenous administration of said adherent cells, *e.g*., by intravenous infusion, bolus injection, or the like. The bag can comprise multiple lumens or compartments that are interconnected to allow mixing of the cells and one or more other solutions, *e.g*., a drug, prior to, or during, administration. In another specific embodiment, prior to cryopreservation, the solution comprising the amnion derived adherent cells comprises one or more compounds that facilitate cryopreservation of the cells. In another specific embodiment, said amnion derived adherent cells are contained within a physiologically-acceptable aqueous solution. In a more specific embodiment, said physiologically-acceptable aqueous solution is a 0.9% NaCl solution. In another specific embodiment, said amnion derived adherent cells comprise cells that are HLA-matched to a recipient of said cells. In another specific embodiment, said amnion derived adherent cells comprise cells that are at least partially HLA-mismatched to a recipient of said cells. In another specific embodiment, said amnion derived adherent cells are derived from a plurality of donors. In various specific embodiments, said container comprises about, at least, or at most 1 x 10⁶ said cells, 5 x 10⁶ said cells, 1 x 10⁷ said stem cells, 5 x 10⁷ said cells, 1 x 10⁸ said cells, 5 x 10⁸ said cells, 1 x 10⁹ said cells, 5 x 10⁹ said cells, 1 x 10¹⁰, or 1 x 10¹¹ said cells. In other specific embodiments of any of the foregoing cryopreserved populations, said cells have been passaged about, at least, or no more than 5 times, no more than 10 times, no more than 15 times, or no more than 20 times. In another specific embodiment of any of the foregoing cryopreserved cells, said cells have been expanded within said container. In specific embodiments, a single unit dose of amnion derived adherent cells can comprise, in various embodiments, about, at least, or no more than 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more amnion derived adherent cells.

In certain embodiments, the pharmaceutical compositions provided herein comprises populations of amnion derived adherent cells, that comprise 50% viable cells or more (that is, at least 50% of the cells in the population are functional or living). Preferably, at least 60% of the cells in the population are viable. More preferably, at least 70%, 80%, 90%, 95%, or 99% of the cells in the population in the pharmaceutical composition are viable.

### 5.8.2 Matrices Comprising Amnion Derived Adherent Cells

Further provided herein are compositions comprising matrices, hydrogels, scaffolds, and the like. Such compositions can be used in the place of, or in addition to, such cells in liquid suspension.

The matrix can be, *e.g*., a permanent or degradable decellularized tissue, *e.g*., a decellularized amniotic membrane, or a synthetic matrix. The matrix can be a three-dimensional scaffold. In a more specific embodiment, said matrix comprises collagen, gelatin, laminin, fibronectin, pectin, ornithine, or vitronectin. In another more specific embodiment, the matrix is an amniotic membrane or an amniotic membrane-derived biomaterial. In another more specific embodiment, said matrix comprises an extracellular membrane protein. In another more specific embodiment, said matrix comprises a synthetic compound. In another more specific embodiment, said matrix comprises a bioactive compound. In another more specific embodiment, said bioactive compound is a growth factor, a cytokine, an antibody, or an organic molecule of less than 5,000 daltons.

The amnion derived adherent cells described herein can be seeded onto a natural matrix, *e.g*., a placental biomaterial such as an amniotic membrane material. Such an amniotic membrane material can be, *e.g*., amniotic membrane dissected directly from a mammalian placenta; fixed or heat-treated amniotic membrane, substantially dry (*i.e.*, <20% H₂O) amniotic membrane, chorionic membrane, substantially dry chorionic membrane, substantially dry amniotic and chorionic membrane, and the like. Preferred placental biomaterials on which the amnion derived adherent cells provided herein can be seeded are described in Hariri, U.S. Application Publication No. 2004/0048796, the disclosure of which is incorporated by reference herein in its entirety.

In another specific embodiment, the matrix is a composition comprising an extracellular matrix. In a more specific embodiment, said composition is MATRIGEL™ (BD Biosciences).

The isolated amnion derived adherent cells described herein can be suspended in a hydrogel solution suitable for, *e.g*., injection. The hydrogel is, *e.g*., an organic polymer (natural or synthetic) that is cross-linked via covalent, ionic, or hydrogen bonds to create a three-dimensional open-lattice structure that entraps water molecules to form a gel. Suitable hydrogels for such compositions include self-assembling peptides, such as RAD16. In one embodiment, a hydrogel solution comprising the cells can be allowed to harden, for instance in a mold, to form a matrix having cells dispersed therein for implantation. The amnion derived adherent cells in such a matrix can also be cultured so that the cells are mitotically expanded, *e.g*., prior to implantation. Hydrogel-forming materials include polysaccharides such as alginate and salts thereof, peptides, polyphosphazines, and polyacrylates, which are crosslinked ionically, or block polymers such as polyethylene oxide-polypropylene glycol block copolymers which are crosslinked by temperature or pH, respectively. In some embodiments, the hydrogel or matrix is biodegradable.

In certain embodiments, the compositions comprising cells, provided herein, comprise an *in situ* polymerizable gel (*see.*, *e.g.*, U.S. Patent Application Publication 2002/0022676; Anseth et al., J. Control Release, 78(1-3):199-209 (2002); Wang et al., Biomaterials, 24(22):3969-80 (2003). In some embodiments, the polymers are at least partially soluble in aqueous solutions, such as water, buffered salt solutions, or aqueous alcohol solutions, that have charged side groups, or a monovalent ionic salt thereof. Examples of polymers having acidic side groups that can be reacted with cations are poly(phosphazenes), poly(acrylic acids), poly(methacrylic acids), copolymers of acrylic acid and methacrylic acid, poly(vinyl acetate), and sulfonated polymers, such as sulfonated polystyrene. Copolymers having acidic side groups formed by reaction of acrylic or methacrylic acid and vinyl ether monomers or polymers can also be used. Examples of acidic groups are carboxylic acid groups, sulfonic acid groups, halogenated (preferably fluorinated) alcohol groups, phenolic OH groups, and acidic OH groups.

In a specific embodiment, the matrix is a felt, which can be composed of a multifilament yarn made from a bioabsorbable material, *e.g*., PGA, PLA, PCL copolymers or blends, or hyaluronic acid. The yarn is made into a felt using standard textile processing techniques consisting of crimping, cutting, carding and needling. In another preferred embodiment the cells of the invention are seeded onto foam scaffolds that may be composite structures. In addition, the three-dimensional framework may be molded into a useful shape, such as a specific structure in the body to be repaired, replaced, or augmented. Other examples of scaffolds that can be used include nonwoven mats, porous foams, or self assembling peptides. Nonwoven mats can be formed using fibers comprised of a synthetic absorbable copolymer of glycolic and lactic acids (*e.g*., PGA/PLA) (VICRYL, Ethicon, Inc., Somerville, N.J.). Foams, composed of, *e.g*., poly(ε-caprolactone)/poly(glycolic acid) (PCL/PGA) copolymer, formed by processes such as freeze-drying, or lyophilization (*see, e.g*., U.S. Pat. No. 6,355,699), can also be used as scaffolds.

The amnion derived adherent cells described herein can be seeded onto a three-dimensional framework or scaffold and implanted *in vivo.* Such a framework can be implanted in combination with any one or more growth factors, cells, drugs or other components that, *e.g.*, stimulate tissue formation, *e.g.*, bone formation or formation of vasculature.

The amnion derived adherent cells provided herein can, in another embodiment, be seeded onto foam scaffolds that may be composite structures. Such foam scaffolds can be molded into a useful shape, such as that of a portion of a specific structure in the body to be repaired, replaced or augmented. In some embodiments, the framework is treated, *e.g*., with 0.1M acetic acid followed by incubation in polylysine, PBS, and/or collagen, prior to inoculation of the cells in order to enhance cell attachment. External surfaces of a matrix may be modified to improve the attachment or growth of cells and differentiation of tissue, such as by plasma-coating the matrix, or addition of one or more proteins (*e.g*., collagens, elastic fibers, reticular fibers), glycoproteins, glycosaminoglycans (*e.g*., heparin sulfate, chondroitin-4-sulfate, chondroitin-6-sulfate, dermatan sulfate, keratin sulfate, *etc*.), a cellular matrix, and/or other materials such as, but not limited to, gelatin, alginates, agar, agarose, and plant gums, and the like.

In some embodiments, the matrix comprises, or is treated with, materials that render it non-thrombogenic. These treatments and materials may also promote and sustain endothelial growth, migration, and extracellular matrix deposition. Examples of these materials and treatments include but are not limited to natural materials such as basement membrane proteins such as laminin and Type IV collagen, synthetic materials such as EPTFE, and segmented polyurethaneurea silicones, such as PURSPAN™ (The Polymer Technology Group, Inc., Berkeley, Calif.). The matrix can also comprise anti-thrombotic agents such as heparin; the scaffolds can also be treated to alter the surface charge (*e.g*., coating with plasma) prior to seeding with the adherent cells provided herein.

The framework may be treated prior to inoculation of the amnion derived adherent cells provided herein in order to enhance cell attachment. For example, prior to inoculation with the cells of the invention, nylon matrices could be treated with 0.1 molar acetic acid and incubated in polylysine, PBS, and/or collagen to coat the nylon. Polystyrene can be similarly treated using sulfuric acid.

In addition, the external surfaces of the three-dimensional framework may be modified to improve the attachment or growth of cells and differentiation of tissue, such as by plasma coating the framework or addition of one or more proteins (*e.g*., collagens, elastic fibers, reticular fibers), glycoproteins, glycosaminoglycans (*e.g*., heparin sulfate, chondroitin-4-sulfate, chondroitin-6-sulfate, dermatan sulfate, keratin sulfate), a cellular matrix, and/or other materials such as, but not limited to, gelatin, alginates, agar, agarose, or plant gums.

In some embodiments, the matrix comprises or is treated with materials that render the matrix non-thrombogenic, *e.g*., natural materials such as basement membrane proteins such as laminin and Type IV collagen, and synthetic materials such as ePTFE or segmented polyurethaneurea silicones, such as PURSPAN (The Polymer Technology Group, Inc., Berkeley, Calif.). Such materials can be further treated to render the scaffold non-thrombogenic, *e.g*., with heparin, and treatments that alter the surface charge of the material, such as plasma coating.

The therapeutic cell compositions comprising amnion derived adherent cells can also be provided in the form of a matrix-cell complex. Matrices can include biocompatible scaffolds, lattices, self-assembling structures and the like, whether bioabsorbable or not, liquid, gel, or solid. Such matrices are known in the arts of therapeutic cell treatment, surgical repair, tissue engineering, and wound healing. In certain embodiments, the cells adhere to the matrix. In other embodiments, the cells are entrapped or contained within matrix spaces. Most preferred are those matrix-cell complexes in which the cells grow in close association with the matrix and when used therapeutically, stimulate and support ingrowth of a recipient's cells. The matrix-cell compositions can be introduced into an individual's body in any way known in the art, including but not limited to implantation, injection, surgical attachment, transplantation with other tissue, injection, and the like. In some embodiments, the matrices form *in vivo*, or *in situ.* For example, *in situ* polymerizable gels can be used in accordance with the invention. Examples of such gels are known in the art.

In some embodiments, the cells provided herein are seeded onto such three-dimensional matrices, such as scaffolds and implanted in vivo, where the seeded cells may proliferate on or in the framework or help establish replacement tissue in vivo with or without cooperation of other cells. Growth of the amnion derived adherent cells or co-cultures thereof on the three-dimensional framework preferably results in the formation of a three-dimensional tissue, or foundation thereof, which can be utilized in vivo, for example for repair of damaged or diseased tissue. For example, the three-dimensional scaffolds can be used to form tubular structures, for example for use in repair of blood vessels; or aspects of the circulatory system or coronary structures. In accordance with one aspect of the invention, amnion derived adherent cells, or co-cultures thereof, are inoculated, or seeded on a three-dimensional framework or matrix, such as a scaffold, a foam or hydrogel. The framework may be configured into various shapes such as generally flat, generally cylindrical or tubular, or can be completely free-form as may be required or desired for the corrective structure under consideration. In some embodiments, the amnion derived adherent cells grow on the three dimensional structure, while in other embodiments, the cells only survive, or even die, but stimulate or promote ingrowth of new tissue or vascularization in a recipient.

The cells of the invention can be grown freely in culture, removed from the culture and inoculated onto a three-dimensional framework. Inoculation of the three-dimensional framework with a concentration of cells, *e.g*., approximately 10⁶ to 5 x 10⁷ cells per milliliter, preferably results in the establishment of the three-dimensional support in relatively shorter periods of time. Moreover in some application it may be preferably to use a greater or lesser number of cells depending on the result desired.

In a specific embodiment, the matrix can be cut into a strip (*e.g*., rectangular in shape) of which the width is approximately equal to the inner circumference of a tubular organ into which it will ultimately be inserted. The amnion derived adherent cells can be inoculated onto the scaffold and incubated by floating or suspending in liquid media. At the appropriate stage of confluence, the scaffold can be rolled up into a tube by joining the long edges together. The seam can then be closed by suturing the two edges together using fibers of a suitable material of an appropriate diameter. In order to prevent cells from occluding the lumen, one of the open ends of the tubular framework can be affixed to a nozzle. Liquid media can be forced through the nozzle from a source chamber connected to the incubation chamber to create a current through the interior of the tubular framework. The other open end can be affixed to an outflow aperture which leads into a collection chamber from which the media can be recirculated through the source chamber. The tube can be detached from the nozzle and outflow aperture when incubation is complete. *See, e.g*., International Application No. WO 94/25584.

In general, two three-dimensional frameworks can be combined into a tube in accordance with the invention using any of the following methods. Two or more flat frameworks can be laid atop another and sutured together. The resulting two-layer sheet can then be rolled up, and, as described above, joined together and secured. In certain embodiments, one tubular scaffold that is to serve as the inner layer can be inoculated with amnion derived adherent cells and incubated. A second scaffold can be grown as a flat strip with width slightly larger than the outer circumference of the tubular framework. After appropriate growth is attained, the flat framework is wrapped around the outside of the tubular scaffold followed by closure of the seam of the two edges of the flat framework and securing the flat framework to the inner tube. In another embodiment, two or more tubular meshes of slightly differing diameters can be grown separately. The framework with the smaller diameter can be inserted inside the larger one and secured. For each of these methods, more layers can be added by reapplying the method to the double-layered tube. The scaffolds can be combined at any stage of growth of the amnion derived adherent cells, and incubation of the combined scaffolds can be continued when desirable.

In conjunction with the above, the cells and therapeutic compositions provided herein can be used in conjunction with implantable devices. For example the amnion derived adherent cells can be coadminstered with, for example, stents, artificial valves, ventricular assist devices, Guglielmi detachable coils and the like. As the devices may constitute the dominant therapy provided to an individual in need of such therapy, the cells and the like may be used as supportive or secondary therapy to assist in, stimulate, or promote proper healing in the area of the implanted device. The cells and therapeutic compositions of the invention may also be used to pretreat certain implantable devices, to minimize problems when they are used *in vivo.* Such pretreated devices, including coated devices, may be better tolerated by patients receiving them, with decrease risk of local or systemic infection, or for example, restenosis or further occlusion of blood vessels.

### 5.8.3 Media Conditioned by Amnion Derived Adherent Cells

Further provided herein is medium that has been conditioned by amnion derived adherent cells, that is, medium comprising one or more biomolecules secreted or excreted by the adherent cells. In various embodiments, the conditioned medium comprises medium in which the cells have grown for at least 1, 2, 3, 4, 5, 6, 7, 8, 9, 10, 11, 12, 13, 14 or more days, or for at least 1, 2, 3, 4, 5, 6, 7, 8, 9, 10, 11, 12, 13, 14, 15, 16, 17, 18, 19, or 20 population doublings, or more. In other embodiments, the conditioned medium comprises medium in which amnion derived adherent cells have grown to at least 30%, 40%, 50%, 60%, 70%, 80%, 90% confluence, or up to 100% confluence. Such conditioned medium can be used to support the culture of a population of cells, *e.g*., stem cells, *e.g*., placental stem cells, embryonic stem cells, embryonic germ cells, adult stem cells, or the like. In another embodiment, the conditioned medium comprises medium in which amnion derived adherent cells, and cells that are not amnion derived adherent cells, have been cultured together.

The conditioned medium can comprise the adherent cells provided herein. Thus, provided herein is a cell culture comprising amnion derived adherent cells. In a specific embodiment, the conditioned medium comprises a plurality, *e.g.*, a population, of amnion derived adherent cells.

The conditioned medium can be collected from the cell culture and filtered and/or sterilized using methods known in the art, *e.g*., the conditioned medium can be sterilized to neutralize the activity of any potential contaminants of filtered through a small pore filter (*e.g.,* a 0.22 µM filter) to remove contaminants. In some embodiments, the conditioned medium can be used immediately after collection and sterilization/filtration in a method of treatment provided herein. In other embodiments, the conditioned medium can be frozen and stored for subsequent use in a method of treatment provided herein.

### 5.9 PRESERVATION OF AMNION DERIVED ADHERENT CELLS

Amnion derived adherent cells can be preserved, that is, placed under conditions that allow for long-term storage, or conditions that inhibit cell death by, *e.g*., apoptosis or necrosis, *e.g*., during collection or prior to production of the compositions described herein, *e.g*., using the methods described herein.

Amnion derived adherent cells can be preserved using, *e.g.,* a composition comprising an apoptosis inhibitor, necrosis inhibitor and/or an oxygen-carrying perfluorocarbon, as described in U.S. Application Publication No. 2007/0190042, the disclosure of which is hereby incorporated by reference in its entirety. In one embodiment, a method of preserving such cells, or a population of such cells, comprises contacting said cells or population of cells with a cell collection composition comprising an inhibitor of apoptosis and an oxygen-carrying perfluorocarbon, wherein said inhibitor of apoptosis is present in an amount and for a time sufficient to reduce or prevent apoptosis in the population of cells, as compared to a population of cells not contacted with the inhibitor of apoptosis. In a specific embodiment, said inhibitor of apoptosis is a caspase inhibitor. In another specific embodiment, said inhibitor of apoptosis is a JNK inhibitor. In a more specific embodiment, said JNK inhibitor does not modulate differentiation or proliferation of amnion derived adherent cells. In another embodiment, said cell collection composition comprises said inhibitor of apoptosis and said oxygen-carrying perfluorocarbon in separate phases. In another embodiment, said cell collection composition comprises said inhibitor of apoptosis and said oxygen-carrying perfluorocarbon in an emulsion. In another embodiment, the cell collection composition additionally comprises an emulsifier, *e.g*., lecithin. In another embodiment, said apoptosis inhibitor and said perfluorocarbon are between about 0°C and about 25°C at the time of contacting the cells. In another more specific embodiment, said apoptosis inhibitor and said perfluorocarbon are between about 2°C and 10°C, or between about 2°C and about 5°C, at the time of contacting the cells. In another more specific embodiment, said contacting is performed during transport of said population of cells. In another more specific embodiment, said contacting is performed during freezing and thawing of said population of cells.

Populations of amnion derived adherent cells can be preserved, *e.g*., by a method comprising contacting a population of said cells with an inhibitor of apoptosis and an organ-preserving compound, wherein said inhibitor of apoptosis is present in an amount and for a time sufficient to reduce or prevent apoptosis in the population of cells, as compared to a population of cells not contacted with the inhibitor of apoptosis. In a specific embodiment, the organ-preserving compound is UW solution (described in U.S. Patent No. 4,798,824; also known as ViaSpan; *see also* Southard et al., Transplantation 49(2):251-257 (1990)) or a solution described in Stern et al., U.S. Patent No. 5,552,267. In another embodiment, said organ-preserving compound is hydroxyethyl starch, lactobionic acid, raffinose, or a combination thereof. In another embodiment, the cell collection composition additionally comprises an oxygen-carrying perfluorocarbon, either in two phases or as an emulsion.

In another embodiment of the method, amnion derived adherent cells are contacted with a cell collection composition comprising an apoptosis inhibitor and oxygen-carrying perfluorocarbon, organ-preserving compound, or combination thereof, *e.g*., during a process of tissue disruption, *e.g*., enzymatic digestion of amnion tissue. In another embodiment, amnion derived adherent cells are contacted with said cell collection compound after collection by tissue disruption, *e.g*., enzymatic digestion of amnion tissue.

Typically, during collection of amnion derived adherent cells, enrichment and isolation, it is preferable to minimize or eliminate cell stress due to hypoxia and mechanical stress. In another embodiment of the method, therefore, an amnion derived adherent cell, or population of cells comprising the amnion derived adherent cells, is exposed to a hypoxic condition during collection, enrichment or isolation for less than six hours during said preservation, wherein a hypoxic condition is a concentration of oxygen that is, *e.g.,* less than normal atmospheric oxygen concentration; less than normal blood oxygen concentration; or the like. In a more specific embodiment, said cells or population of said cells is exposed to said hypoxic condition for less than two hours during said preservation. In another more specific embodiment, said cells or population of said cells is exposed to said hypoxic condition for less than one hour, or less than thirty minutes, or is not exposed to a hypoxic condition, during collection, enrichment or isolation. In another specific embodiment, said population of cells is not exposed to shear stress during collection, enrichment or isolation.

Amnion derived adherent cells can be cryopreserved, in general or by the specific methods disclosed herein, *e.g.*, in cryopreservation medium in small containers, *e.g.*, ampoules. Suitable cryopreservation medium includes, but is not limited to, culture medium including, e.g., growth medium, or cell freezing medium, for example commercially available cell freezing medium, *e.g*., cell freezing medium identified by SigmaAldrich catalog numbers C2695, C2639 (Cell Freezing Medium-Serum-free 1X, not containing DMSO) or C6039 (Cell Freezing Medium-Glycoerol 1 X containing Minimum Essential Medium, glycerol, calf serum and bovine serum), Lonza PROFREEZE™ 2x Medium, methylcellulose, dextran, human serum albumin, fetal bovine serum, fetal calf serum, or Plasmalyte. Cryopreservation medium preferably comprises DMSO (dimethylsulfoxide) or glycerol, at a concentration of, *e.g*., about 1% to about 20%, *e.g*., about 5% to 10% (v/v), optionally including fetal bovine serum or human serum. Cryopreservation medium may comprise additional agents, for example, methylcellulose with or without glycerol. Isolated amnion derived adherent cells are preferably cooled at about 1°C/min during cryopreservation. A preferred cryopreservation temperature is about -80°C to about -180°C, preferably about -125°C to about -140°C. Cryopreserved cells can be transferred to vapor phase of liquid nitrogen prior to thawing for use. In some embodiments, for example, once the ampoules have reached about -80°C, they are transferred to a liquid nitrogen storage area. Cryopreservation can also be done using a controlled-rate freezer. Cryopreserved cells preferably are thawed at a temperature of about 25°C to about 40°C, preferably to a temperature of about 37°C.

### 5.10 MODIFIED AMNION DERIVED ADHERENT CELLS

### 5.10.1 Genetically Modified Amnion Derived Adherent Cells

In another aspect, the amnion derived adherent cells described herein can be genetically modified, *e.g.,* to produce a nucleic acid or polypeptide of interest, or to produce a differentiated cell, *e.g.,* an osteogenic cell, myocytic cell, pericytic cell, or angiogenic cell, that produces a nucleic acid or polypeptide of interest. Genetic modification can be accomplished, *e.g*., using virus-based vectors including, but not limited to, non-integrating replicating vectors, e.g., papilloma virus vectors, SV40 vectors, adenoviral vectors; integrating viral vectors, *e.g*., retrovirus vector or adeno-associated viral vectors; or replication-defective viral vectors. Other methods of introducing DNA into cells include the use of liposomes, electroporation, a particle gun, direct DNA injection, or the like.

The adherent cells provided herein can be, *e.g*., transformed or transfected with DNA controlled by or in operative association with, one or more appropriate expression control elements, for example, promoter or enhancer sequences, transcription terminators, polyadenylation sites, internal ribosomal entry sites. Preferably, such a DNA incorporates a selectable marker. Following the introduction of the foreign DNA, engineered adherent cells can be, *e.g*., grown in enriched media and then switched to selective media. In one embodiment, the DNA used to engineer an amnion derived adherent cell comprises a nucleotide sequence encoding a polypeptide of interest, *e.g*., a cytokine, growth factor, differentiation agent, or therapeutic polypeptide.

The DNA used to engineer the adherent cell can comprise any promoter known in the art to drive expression of a nucleotide sequence in mammalian cells, *e.g*., human cells. For example, promoters include, but are not limited to, CMV promoter/enhancer, SV40 promoter, papillomavirus promoter, Epstein-Barr virus promoter, elastin gene promoter, and the like. In a specific embodiment, the promoter is regulatable so that the nucleotide sequence is expressed only when desired. Promoters can be either inducible (*e.g*., those associated with metallothionein and heat shock proteins) or constitutive.

In another specific embodiment, the promoter is tissue-specific or exhibits tissue specificity. Examples of such promoters include but are not limited to myosin light chain-2 gene control region (Shani, 1985, Nature 314:283) (skeletal muscle).

The amnion derived adherent cells disclosed herein may be engineered or otherwise selected to "knock out" or "knock down" expression of one or more genes in such cells. The expression of a gene native to a cell can be diminished by, for example, inhibition of expression by inactivating the gene completely by, *e.g*., homologous recombination. In one embodiment, for example, an exon encoding an important region of the protein, or an exon 5' to that region, is interrupted by a positive selectable marker, *e.g*., neo, preventing the production of normal mRNA from the target gene and resulting in inactivation of the gene. A gene may also be inactivated by creating a deletion in part of a gene or by deleting the entire gene. By using a construct with two regions of homology to the target gene that are far apart in the genome, the sequences intervening the two regions can be deleted (Mombaerts et al., 1991, Proc. Nat. Acad. Sci. U.S.A. 88:3084). Antisense, morpholinos, DNAzymes, small interfering RNA, short hairpin RNA, and ribozyme molecules that inhibit expression of the target gene can also be used to reduce the level of target gene activity in the adherent cells. For example, antisense RNA molecules which inhibit the expression of major histocompatibility gene complexes (HLA) have been shown to be most versatile with respect to immune responses. Triple helix molecules can be utilized in reducing the level of target gene activity. *See, e.g*., L. G. Davis et al. (eds), 1994, BASIC METHODS IN MOLECULAR BIOLOGY, 2nd ed., Appleton & Lange, Norwalk, Conn., which is incorporated herein by reference.

In a specific embodiment, the amnion derived adherent cells disclosed herein can be genetically modified with a nucleic acid molecule comprising a nucleotide sequence encoding a polypeptide of interest, wherein expression of the polypeptide of interest is controllable by an exogenous factor, *e.g*., polypeptide, small organic molecule, or the like. The polypeptide of interest can be a therapeutic polypeptide. In a more specific embodiment, the polypeptide of interest is IL-12 or interleukin-1 receptor antagonist (IL-1Ra). In another more specific embodiment, the polypeptide of interest is a fusion of interleukin-1 receptor antagonist and dihydrofolate reductase (DHFR), and the exogenous factor is an antifolate, *e.g*., methotrexate. Such a construct is useful in the engineering of amnion derived adherent cells that express IL-1Ra, or a fusion of IL-1Ra and DHFR, upon contact with methotrexate. Such a construct can be used, *e.g*., in the treatment of rheumatoid arthritis. In this embodiment, the fusion of IL-1Ra and DHFR is translationally upregulated upon exposure to an antifolate such as methotrexate. Therefore, in another specific embodiment, the nucleic acid used to genetically engineer an amnion derived adherent cell can comprise nucleotide sequences encoding a first polypeptide and a second polypeptide, wherein said first and second polypeptides are expressed as a fusion protein that is translationally upregulated in the presence of an exogenous factor. The polypeptide can be expressed transiently or long-term (*e.g*., over the course of weeks or months). Such a nucleic acid molecule can additionally comprise a nucleotide sequence encoding a polypeptide that allows for positive selection of engineered cells, or allows for visualization of the engineered cells. In another more specific embodiment, the nucleotide sequence encodes a polypeptide that is, *e.g*., fluorescent under appropriate visualization conditions, *e.g*., luciferase (Luc). In a more specific embodiment, such a nucleic acid molecule can comprise IL-1Ra-DHFR-IRES-Luc, where IL-1Ra is interleukin-1 receptor antagonist, IRES is an internal ribosomal entry site, and DHFR is dihydrofolate reductase.

### 5.10.2 Immortalized Amnion Derived Adherent Cell Lines

Mammalian amnion derived adherent cells can be conditionally immortalized by transfection with any suitable vector containing a growth-promoting gene, that is, a gene encoding a protein that, under appropriate conditions, promotes growth of the transfected cell, such that the production and/or activity of the growth-promoting protein is regulatable by an external factor. In a preferred embodiment the growth-promoting gene is an oncogene such as, but not limited to, v-myc, N-myc, c-myc, p53, SV40 large T antigen, polyoma large T antigen, E1a adenovirus or E7 protein of human papillomavirus. In another embodiment, amnion derived adherent cells can be immortalized using cre-lox recombination, as exemplified for a human pancreatic β-cell line by Narushima, M., et al (nature Biotechnology, 2005, 23(10:1274-1282).

External regulation of the growth-promoting protein can be achieved by placing the growth-promoting gene under the control of an externally-regulatable promoter, *e.g*., a promoter the activity of which can be controlled by, for example, modifying the temperature of the transfected cells or the composition of the medium in contact with the cells. in one embodiment, a tetracycline (tet)-controlled gene expression system can be employed *(see* Gossen et al., Proc. Natl. Acad. Sci. USA 89:5547-5551, 1992; Hoshimaru et al., Proc. Natl. Acad. Sci. USA 93:1518-1523, 1996). In the absence of tet, a tet-controlled transactivator (tTA) within this vector strongly activates transcription from ph_{*CMV**-1}, a minimal promoter from human cytomegalovirus fused to tet operator sequences. tTA is a fusion protein of the repressor (tetR) of the transposon-10-derived tet resistance operon of *Escherichia coli* and the acidic domain of VP16 of herpes simplex virus. Low, non-toxic concentrations of tet (*e.g*., 0.01-1.0 µg/mL) almost completely abolish transactivation by tTA.

In one embodiment, the vector further contains a gene encoding a selectable marker, *e.g*., a protein that confers drug resistance. The bacterial neomycin resistance gene (neo*^{R}*) is one such marker that may be employed within the present methods. Cells carrying neo*^{R}* may be selected by means known to those of ordinary skill in the art, such as the addition of, *e.g.,* 100-200 µg/mL G418 to the growth medium.

Transfection can be achieved by any of a variety of means known to those of ordinary skill in the art including, but not limited to, retroviral infection. In general, a cell culture may be transfected by incubation with a mixture of conditioned medium collected from the producer cell line for the vector and DMEM/F12 containing N2 supplements. For example, a placental cell culture prepared as described above may be infected after, *e.g*., five days *in vitro* by incubation for about 20 hours in one volume of conditioned medium and two volumes of DMEM/F12 containing N2 supplements. Transfected cells carrying a selectable marker may then be selected as described above.

Following transfection, cultures are passaged onto a surface that permits proliferation, *e.*g., allows at least 30% of the cells to double in a 24 hour period. Preferably, the substrate is a polyornithine/laminin substrate, consisting of tissue culture plastic coated with polyornithine (10 µg/mL) and/or laminin (10 µg/mL), a polylysine/laminin substrate or a surface treated with fibronectin. Cultures are then fed every 3-4 days with growth medium, which may or may not be supplemented with one or more proliferation-enhancing factors. Proliferation-enhancing factors may be added to the growth medium when cultures are less than 50% confluent.

The conditionally-immortalized amnion derived adherent cell lines can be passaged using standard techniques, such as by trypsinization, when 80-95% confluent. Up to approximately the twentieth passage, it is, in some embodiments, beneficial to maintain selection (by, for example, the addition of G418 for cells containing a neomycin resistance gene). Cells may also be frozen in liquid nitrogen for long-term storage.

Clonal cell lines can be isolated from a conditionally-immortalized adherent cell line prepared as described above. In general, such clonal cell lines may be isolated using standard techniques, such as by limit dilution or using cloning rings, and expanded. Clonal cell lines may generally be fed and passaged as described above.

Conditionally-immortalized human amnion derived adherent cells lines, which may, but need not, be clonal, may generally be induced to differentiate by suppressing the production and/or activity of the growth-promoting protein under culture conditions that facilitate differentiation. For example, if the gene encoding the growth-promoting protein is under the control of an externally-regulatable promoter, the conditions, *e.g*., temperature or composition of medium, may be modified to suppress transcription of the growth-promoting gene. For the tetracycline-controlled gene expression system discussed above, differentiation can be achieved by the addition of tetracycline to suppress transcription of the growth-promoting gene. In general, 1 µg/mL tetracycline for 4-5 days is sufficient to initiate differentiation. To promote further differentiation, additional agents may be included in the growth medium.

### 5.11 DOSAGES AND ROUTES OF ADMINISTRATION

Administration of amnion derived adherent cells (AMDACs) to an individual in need thereof can be by any medically-acceptable route relevant for the immune-related disease or condition to be treated. In another specific embodiment of the methods of treatment described above, said AMDACs are administered by bolus injection. In another specific embodiment, said isolated AMDACs are administered intravenously, *e.g*., by intravenous infusion. In a specific embodiment, said intravenous infusion is intravenous infusion over about 1 to about 8 hours. In another specific embodiment, said isolated AMDACs are administered locally, *e.g*., at a particular site in the body of the individual that is affected by the immune-related disease or condition. In another specific embodiment, said isolated AMDACs are administered intracranially. In another specific embodiment, said isolated AMDACs are administered intramuscularly. In another specific embodiment, said isolated AMDACs are administered intraperitoneally. In another specific embodiment, said isolated AMDACs are administered intra-arterially. In another specific embodiment of the method of treatment, said isolated AMDACs are administered intramuscularly, intradermally, or subcutaneously.. In another specific embodiment, said isolated AMDACs are administered intravenously. In another specific embodiment, said isolated AMDACs are administered intraventricularly. In another specific embodiment, said isolated AMDACs are administered intrasternally. In another specific embodiment, said isolated AMDACs are administered intrasynovially. In another specific embodiment, said isolated AMDACs are administered intraocularly. In another specific embodiment, said isolated AMDACs are administered intravitreally. In another specific embodiment, said isolated AMDACs are administered intracerebrally. In another specific embodiment, said isolated AMDACs are administered intracerebroventricularly. In another specific embodiment, said isolated AMDACs are administered intrathecally. In another specific embodiment, said isolated AMDACs are administered by intraosseous infusion. In another specific embodiment, said isolated AMDACs are administered intravesically. In another specific embodiment, said isolated AMDACs are administered transdermally. In another specific embodiment, said isolated AMDACs are administered intracisternally. In another specific embodiment, said isolated AMDACs are administered epidurally.

In another specific embodiment of the methods of treatment described above, said AMDACs are administered once to said individual. In another specific embodiment, said isolated AMDACs are administered to said individual in two or more separate administrations. In another specific embodiment, said administering comprises administering between about 1 x 10⁴ and 1 x 10⁵ isolated AMDACs, *e.g*., AMDACs per kilogram of said individual. In another specific embodiment, said administering comprises administering between about 1 x 10⁵ and 1 x 10⁶ isolated AMDACs per kilogram of said individual. In another specific embodiment, said administering comprises administering between about 1 x 10⁶ and 1 x 10⁷ isolated AMDACs per kilogram of said individual. In another specific embodiment, said administering comprises administering between about 1 x 10⁷ and 1 x 10⁸ isolated AMDACs per kilogram of said individual. In another specific embodiment, said administering comprises administering between about 1 x 10⁸ and 1 x 10⁹ isolated AMDACs per kilogram of said individual. In another specific embodiment, said administering comprises administering between about 1 x 10⁹ and 1 x 10¹⁰ isolated AMDACs per kilogram of said individual. In another specific embodiment, said administering comprises administering between about 1 x 10¹⁰ and 1 x 10¹¹ isolated AMDACs per kilogram of said individual.

In other specific embodiments, said administering comprises administering between about 1 x 10⁶ and about 2 x 10⁶ isolated AMDACs per kilogram of said individual; between about 2 x 10⁶ and about 3 x 10⁶ isolated AMDACs per kilogram of said individual; between about 3 x 10⁶ and about 4 x 10⁶ isolated AMDACs per kilogram of said individual; between about 4 x 10⁶ and about 5 x 10⁶ isolated AMDACs per kilogram of said individual; between about 5 x 10⁶ and about 6 x 10⁶ isolated AMDACs per kilogram of said individual; between about 6 x 10⁶ and about 7 x 10⁶ isolated AMDACs per kilogram of said individual; between about 7 x 10⁶ and about 8 x 10⁶ isolated AMDACs per kilogram of said individual; between about 8 x 10⁶ and about 9 x 10⁶ isolated AMDACs per kilogram of said individual; or between about 9 x 10⁶ and about 1 x 10⁷ isolated AMDACs per kilogram of said individual. In another specific embodiment, said administering comprises administering between about 1 x 10⁷ and about 2 x 10⁷ isolated AMDACs per kilogram of said individual to said individual. In another specific embodiment, said administering comprises administering between about 1.3 x 10⁷ and about 1.5 x 10⁷ isolated AMDACs per kilogram of said individual to said individual. In another specific embodiment, said administering comprises administering up to about 3 x 10⁷ isolated AMDACs per kilogram of said individual to said individual. In a specific embodiment, said administering comprises administering between about 5 x 10⁶ and about 2 x 10⁷ isolated AMDACs to said individual. In another specific embodiment, said administering comprises administering about 150 x 10⁶ isolated AMDACs in about 20 milliliters of solution to said individual.

In another specific embodiment of the methods of treatment described above, isolated AMDACs are administered to an individual as a single unit dose. In specific embodiments, a single unit dose of AMDACs can comprise, in various embodiments, about, at least, or no more than 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more AMDACs.

In a specific embodiment, said administering comprises administering between about 5 x 10⁶ and about 2 x 10⁷ isolated AMDACs to said individual, wherein said cells are contained in a solution comprising 10% dextran, *e.g*., dextran-40, 5% human serum albumin, and optionally an immunosuppressant. In another specific embodiment, said administering comprises administering between about 5 x 10⁷ and 3 x 10⁹ isolated AMDACs intravenously. In more specific embodiments, said administering comprises administering about 9 x 10⁸ isolated AMDACs or about 1.8 x 10⁹ isolated AMDACs intravenously. In another specific embodiment, said administering comprises administering between about 5 x 10⁷ and 1 x 10⁸ isolated AMDACs intracranially. In a more specific embodiment, said administering comprises administering about 9 x 10⁷ isolated AMDACs intracranially.

Administration of medium conditioned by AMDACs to an individual in need thereof can be by any medically-acceptable route relevant for the disease, disorder or condition associated with CNS injury to be treated including, but not limited to, bolus injection, intravenously (*e.g*., by intravenous infusion), locally (*e.g.*, at a particular site in the body of the individual that is affected by the disease, disorder or condition associated with CNS injury), intracranially, intramuscularly, intraperitoneally, intra-arterially, intramuscularly, intradermally, subcutaneously, intraventricularly, intrasynovially, intraocularly, intravitreally, intracerebrally, intracerebroventricularly, intrathecally, by intraosseous infusion, intravesically, transdermally, intracisternally, or epidurally. In a specific embodiment, the medium conditioned by AMDACs is administered by continuous infusion. In another specific embodiment, the medium conditioned by AMDACs is administered as a single dose.

In some embodiments, administration of medium conditioned by AMDACs to an individual in need thereof comprises administering about 0.01 to about 0.02 ml of medium conditioned by AMDACs per 100 grams of body weight, about 0.01 to about 0.05 ml of medium conditioned by AMDACs per 100 grams of body weight, about 0.01 to about 0.1 ml of medium conditioned by AMDACs per 100 grams of body weight, about 0.01 to about 0.15 ml of medium conditioned by AMDACs per 100 grams of body weight, about 0.01 to about 0.2 ml of medium conditioned by AMDACs per 100 grams of body weight, about 0.01 to about 0.25 ml of medium conditioned by AMDACs per 100 grams of body weight, about 0.01 to about 0.3 ml of medium conditioned by AMDACs per 100 grams of body weight, about 0.01 to about 0.35 ml of medium conditioned by AMDACs per 100 grams of body weight, about 0.01 to about 0.4 ml of medium conditioned by AMDACs per 100 grams of body weight, about 0.01 to about 0.45 ml of medium conditioned by AMDACs per 100 grams of body weight, or about 0.01 to about 0.5 ml of medium conditioned by AMDACs per 100 grams of body weight.

### 5.12 DIFFERENTIATION OF AMNION DERIVED ADHERENT CELLS

The amnion derived adherent cells provided herein can be differentiated. In one embodiment, the cell has been differentiated sufficiently for said cell to exhibit at least one characteristic of an endothelial cell, a myogenic cell, or a pericytic cell, *e.g.*, by contacting the cell with vascular endothelial growth factor (VEGF), or as described in Sections 5.11.2, 6.3.3, or 6.3.4, below. In more specific embodiments, said characteristic of an endothelial cell, myogenic cell or pericytic cell is expression of one or more of CD9, CD31, CD54, CD102, NG2 (neural/glial antigen 2) or alpha smooth muscle actin, which is increased compared to an amniotic cell that is OCT-4⁻, VEGFR2/KDR⁺, CD9⁺, CD54⁺, CD105⁺, CD200⁺, and VE-cadherin⁻. In other more specific embodiments, said characteristic of an endothelial cell, myogenic cell or pericytic cell is expression of one or more of CD9, CD31, CD54, CD102, NG2 (neural/glial antigen 2) or alpha smooth muscle actin, which is increased compared to an amniotic cell that is OCT-4⁻, VEGFR2/KDR⁺, and VEGFR1/Flt-1⁺.

Myogenic (cardiogenic) differentiation of the amnion derived adherent cells provided herein can be accomplished, for example, by placing the cells in cell culture conditions that induce differentiation into cardiomyocytes. A preferred cardiomyocytic medium comprises DMEM/20% CBS supplemented with retinoic acid, 1 µM; basic fibroblast growth factor, 10 ng/mL; and transforming growth factor beta-1, 2 ng/mL; and epidermal growth factor, 100 ng/mL. KnockOut Serum Replacement (Invitrogen, Carlsbad, California) may be used in lieu of CBS. Alternatively, the amnion derived adherent cells are cultured in DMEM/20% CBS supplemented with 1 to 100, *e.g.*, 50 ng/mL Cardiotropin-1 for 24 hours. In another embodiment, amnion derived adherent cells can be cultured 10-14 days in protein-free medium for 5-7 days, then stimulated with human myocardium extract, *e.g*., produced by homogenizing human myocardium in 1% HEPES buffer supplemented with 1% cord blood serum.

Differentiation can be confirmed by demonstration of cardiac actin gene expression, *e.g*., by RT/PCR, or by visible beating of the cell. An adherent cell is considered to have differentiated into a cardiac cell when the cell displays one or more of these characteristics.

### 5.12.1 Differentiation Into Neurogenic Cells

Amnion derived angiogenic cells, when cultured under neurogenic conditions, differentiate into cells displaying neural morphology and neural markers. For example, AMDACs, *e.g*, AMDACs expanded for 4 days in DMEM/F12 medium containing 15% v/v FBS, with basic fibroblast growth factor (bFGF), *e.g.*, at about 20 ng/ml, epidermal growth factor (EGF), *e.g*., at about 20 ng/ml, *e.g*., for four days, followed by culture for four days in induction medium comprising DMEM/F12, serum free, containing 200 mM butylated hydroxyanisole, 10 nM potassium chloride, 5 mgs/mL insulin, 10 nM forskolin, 4 nM valproic acid, and 2 nM hydrocortisone. Under these conditions, AMDACs display expression of human nestin, Tuj1 and GFAP, as assessed by antibody staining.

### 5.12.2 Non-Differentiation Into Osteogenic Cells

Amnion derived adherent cells do not show osteogenic differentiation in standard assays for osteogenesis. For example, in one embodiment, lack of osteogenic differentiation by AMDACs can be shown, *e.g*, by lack of deposition of calcium, as shown by lack of von Kossa staining of AMDACs under osteogenic conditions. For example, AMDACs, *e.g*., freshly-prepared or cryopreserved AMDACs, can be suspended in growth medium, *e.g*., at about 5000 cells/cm² in 24-well plates and 6-well plates in growth medium and incubated overnight, then cultured for 14-35 days, *e.g*., 28, days in osteogenic medium. In certain embodiments, osteogenic medium comprises DMEM-low glucose, 10% v/v fetal bovine serum (FBS), 10 mM beta glycerophosphate, 100 nM dexamethasone, and 100 µM ascorbic acid phosphate salt supplemented with transforming growth factor-beta 1 (TGF-β1), *e.g*., at 1-100 ng/mL, *e.g*., 20 ng/mL, and human recombinant bone morphogenetic protein-2 (BMP-2) at, *e.g*., 1-100 ng/mL, *e.g*., 40 ng/mL. Cells are then stained using von Kossa stain using standard protocols; development of black silver deposits indicates the presence of mineralization. In the case of AMDACs, cultures should be substantially, *e.g*., completely, free of deposits, *e.g*., as compared to bone marrow-mesenchymal stem cells, indicating that the AMDACs do not produce calcium deposits, and therefore do not differentiate down an osteogenic pathway.

### 5.12.3 Non-Differentiation Into Chondrogenic Cells

Amnion derived adherent cells similarly do not show chondrogenic differentiation in standard assays for chondrogenesis. For example, in one embodiment, lack of chondrogenic differentiation by AMDACs can be shown, *e.g*., by lack of development by AMDACs of cell pellets in a chondrogenesis assay in which chondrogenic cells for cell pellets. For example, AMDACs, *e.g*., freshly prepared or cryopreserved, *e.g*., 2.5×10⁵ cells, can be placed in 15 mL conical tubes and centrifuged at 200×g for 5 minutes at room temperature to form a spherical cell pellet. The collected cells are then cultured in chondrogenic induction medium, *e.g*., Lonza Chondrocyte Medium containing TGF beta-3 (*e.g*., at about 10 ng/mL), recombinant human growth/differentiation factor-5 (rhGDF-5) (*e.g*., at about 500 ng/mL), or a combination of TGF beta-3 (10 nanogram/milliliter), and rhGDF-5 (*e.g*., at about 500 ng/mL) for three weeks. At the end of three weeks, the cells are stained with Alcian blue, which stains for mucopolysaccharides and glycosaminoglycans that are produced by chondrogenic cells. Typically, while BM-MSCs or chondrocytes will, when cultured under these conditions, develop cell pellets that stain positively for Alcian blue, AMDACs neither form pellets nor stain with Alcian blue.

### 6. EXAMPLES

### 6.1 EXAMPLE 1: ISOLATION AND EXPANSION OF ADHERENT CELLS FROM AMNIOTIC MEMBRANE

This Example demonstrates the isolation and expansion of amnion derived adherent cells.

### 6.1.1 Isolation

Amnion derived adherent cells were isolated from amniotic membrane as follows. Amnion/chorion were cut from the placenta, and amnion was manually separated from the chorion. The amnion was rinsed with sterile PBS to remove residual blood, blood clots and other material. Sterile gauze was used to remove additional blood, blood clots or other material that was not removed by rinsing, and the amnion was rinsed again with PBS. Excess PBS was removed from the membrane, and the amnion was cut with a scalpel into 2" by 2" segments. For epithelial cell release, a processing vessel was set up by connecting a sterile jacketed glass processing vessel to a circulating 37°C water bath using tubing and connectors, and set on a stir plate. Trypsin (0.25%, 300 mL) was warmed to 37°C in the processing vessel; the amnion segments were added, and the amnion/trypsin suspension was agitated, *e.g*., at 100 RPM-150 RPM at 37° C for 15 minutes. A sterile screening system was assembled by placing a sterile receptacle on a sterile field next to the processing vessel and inserting a sterile 75 µm to 125 µm screen into the receptacle (Millipore, Billerica, MA). After agitating the amnion segments for 15 minutes, the contents of the processing vessel were transferred to the screen, and the amnion segments were transferred, *e.g*., using sterile tweezers back into the processing vessel; the trypsin solution containing the epithelial cells was discarded. The amnion segments were agitated again with 300 mL trypsin solution (0.25%) as described above. The screen was rinsed with approximately 100-150 mL of PBS, and the PBS solution was discarded. After agitating the amnion segments for 15 minutes, the contents of the processing vessel were transferred to the screen. The amnion segments were then transferred back into the processing vessel; the trypsin solution containing the epithelial cells was discarded. The amnion segments were agitated again with 300 mL trypsin solution (0.25%) as described above. The screen was rinsed with approximately 100-150 mL of PBS, and the PBS solution was discarded. After agitating the amnion segments for 15 minutes, the contents of the processing vessel were transferred to the screen. The amnion segments were then transferred back into the processing vessel, and the trypsin solution containing the epithelial cells was discarded. The amnion segments were agitated in PBS/5% FBS (1:1 ratio of amnion to PBS/5% FBS solution by volume) at 37°C for approximately 2-5 minutes to neutralize the trypsin. A fresh sterile screen system was assembled. After neutralizing the trypsin, the contents of the processing vessel were transferred to the new screen, and the amnion segments were transferred back into the processing vessel. Room temperature, sterile PBS (400 mL) was added to the processing vessel, and the contents of the processing vessel were agitated for approximately 2-5 minutes. The screen was rinsed with approximately 100-150 mL of PBS. After agitation, the contents of the processing vessel were transferred to the screen; the processing flask was rinsed with PBS, and the PBS solution was discarded. The processing vessel was then filled with 300 mL of pre-warmed DMEM, and the amnion segments were transferred into the DMEM solution.

For release of the amnion derived adherent cells, the treated amniotic membrane was further treated with collagenase as follows. A sterile collagenase stock solution (500 U/mL) was prepared by dissolving the appropriate amount of collagenase powder (varied with the activity of the collagenase lot received from the supplier) in DMEM. The solution was filtered through a 0.22 µm filter and dispensed into individual sterile containers. CaCl₂ solution (0.5 mL, 600 mM) was added to each 100 mL dose, and the doses were frozen. Collagenase (100 mL) was added to the amnion segments in the processing vessel, and the processing vessel was agitated for 30-50 minutes, or until amnion digestion was complete by visual inspection. After amnion digestion was complete, 100 mL of pre-warmed sterile PBS/5% FBS was added to the processing vessel, and the processing vessel was agitated for an additional 2-3 minutes. Following agitation, the contents of the flask were transferred to a sterile 60 µm screen, and the liquid was collected by vacuum filtration. The processing vessel was rinsed with 400 mL of PBS, and the PBS solution was sterile-filtered. The filtered cell suspension was then centrifuged at 300 x g for 15 minutes at 20°C, and the cell pellets were resuspended in pre-warmed PBS/2% FBS (approximately 10 mL total).

### 6.1.2 Establishment

Freshly isolated angiogenic amniotic cells were added to growth medium containing 60% DMEM-LG (Gibco); 40% MCBD-201 (Sigma); 2% FBS (Hyclone Labs), 1× insulin-transferrin-selenium (ITS); 10 ng/mL linoleic acid-bovine serum albumin (LA-BSA); 1 n-dexamethasone (Sigma); 100 µM ascorbic acid 2-phosphate (Sigma); 10 ng/mL epidermal growth factor (R & D Systems); and 10 ng/mL platelet-derived growth factor (PDGF-BB) (R & D Systems) and were plated in a T-Flask at a seeding density of 10,000 cells per cm². The culture device(s) were then incubated at 37°C, 5% CO₂ with >90% humidity. Cellular attachment, growth, and morphology were monitored daily. Non-adherent cells and debris were removed by medium exchange. Medium exchange was performed twice per week. Adherent cells with typical fibroblastoid/spindle shape morphology appeared at several days after initial plating. When confluency reached 40% - 70% (at 4 - 11 days after initial plating), the cells were harvested by trypsinization (0.25% trypsin - EDTA) for 5 minutes at room temperature (37°C). After neutralization with PBS-5%FBS, the cells were centrifuged at 200 - 400 g for 5-15 minutes at room temperature, and then were resuspended in growth medium. At this point, an AMDAC line was considered to be successfully established at the initial passage. Initial passage amnion derived adherent cells were, in some cases, cryopreserved or expanded (*e.g*., grown in culture such that the number of cells increases).

### 6.1.3 Culture Procedure

Amnion derived adherent cells were cultured in the growth medium described above and seeded at a density of 2000 - 4000 per cm² in an appropriate tissue culture - treated culture device(s). The culture device(s) were then incubated at 37°C, 5% CO₂ with >90% humidity. During culture, AMDACs would adhere and proliferate. Cellular growth, morphology, and confluency were monitored daily. Medium exchange was performed twice a week to replenish fresh nutrients if the culture extended to 5 days or more. When confluency reached 40% - 70% (at 3 - 7 days after seeding), the cells were harvested by trypsinization (0.05% - 0.25% trypsin - EDTA) for 5 minutes at room temperature (37°C). After neutralization with PBS-5%FBS, the cells were centrifuged at 200 - 400 g for 5-15 minutes at room temperature, then were resuspended in growth medium.

AMDACs isolated and cultured in this manner typically produced 33530 +/-15090 colony-forming units (fibroblast) (CFU-F) out of 1 x 10⁶ cells plated.

### 6.2 EXAMPLE 2: PHENOTYPIC CHARACTERIZATION OF AMNION DERIVED ADHERENT CELLS

### 6.2.1 Gene and Protein Expression Profiles

This Example describes phenotypic characterization of amnion derived adherent cells, including characteristic cell surface marker, mRNA, and proteomic expression.

*Sample preparation:* Amnion derived adherent cells were obtained as described in Example 1. The cells at passage 6 were grown to approximately 70% confluence in growth medium as described in Example 1, above, trypsinized, and washed in PBS. NTERA-2 cells (American Type Culture Collection, ATCC Number CRL-1973) were grown in DMEM containing 4.5 g/L glucose, 2 mM glutamine and 10% FBS. Nucleated cell counts were performed to obtain a minimum of 2 x 10⁶ to 1 x 10⁷ cells. The cells were then lysed using a Qiagen RNeasy kit (Qiagen, Valencia, CA), utilizing a QIAshredder, to obtain the lysates. The RNA isolation was then performed using a Qiagen RNeasy kit. RNA quantity and quality were determined using a Nanodrop ND1000 spectrophotometer, 25 ng/µL of RNA/reaction. The cDNA reactions were prepared using an Applied Biosystems (Foster City, CA) High Capacity cDNA Archive Kit. Real time PCR reactions were performed using TAQMAN^{®} universal PCR master mixes from Applied Biosystems. Reactions were run in standard mode on an Applied Biosystems 7300 Real time PCR system for 40 cycles.

*Simple analysis and result:* Using the real time PCR methodology and specific TAQMAN^{®} gene expression probes and/or the TAQMAN^{®} human angiogenesis array (Applied Biosystems), cells were characterized for expression of stem cell-related, angiogenic and cardiomyogenic markers. Results were expressed either as the relative expression of a gene of interest in comparison to the pertinent cell controls, or the relative expression (delta Ct) of the gene of interest in comparison to a ubiquitously expressed housekeeping gene (for example, GAPDH, 18S, or GUSB).

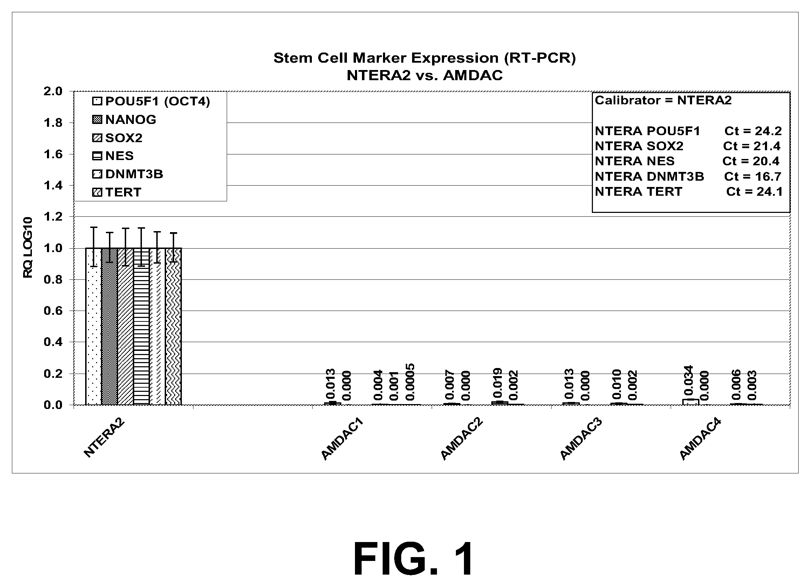

Amnion derived adherent cells expressed various, stem-cell related, angiogenic and cardiomyogenic genes and displayed a relative absence of OCT-4 expression in comparison to NTERA-2 cells. Table 8 summarizes the expression of selected angiogenic, cardiomyogenic, and stem cell genes, and Figure 1 demonstrates the lack of expression in AMDACs of the stem cell-related genes POU5F1 (OCT-4), NANOG, SOX2, NES, DNMT3B, and TERT..

**Table 8: Gene expression profile of amnion derived adherent cells as determined by RT-PCR.**

| **AMDAC Marker** | **Positive** | **Negative** |
|---|---|---|
| | | |
| **ACTA2** | **X** | |
| **ACTC1** | **X** | |
| **ADAMTS1** | **X** | |
| **AMOT** | **X** | |
| **ANG** | **X** | |
| **ANGPT1** | **X** | |
| **ANGPT2** | **X** | |
| **ANGPT4** | | **X** |
| **ANGPTL1** | **X** | |
| **ANGPTL2** | **X** | |
| **ANGPTL3** | | **X** |
| **ANGPTL4** | **X** | |
| **BAI1** | **X** | |
| **BGLAP** | | **X** |
| **c-myc** | **X** | |
| **CD31** | | **X** |
| **CD34** | | **X** |
| **CD44** | **X** | |
| **CD140a** | **X** | |
| **CD140b** | **X** | |
| **CD200** | **X** | |
| **CD202b** | **X** | |
| **CD304** | **X** | |
| **CD309 (VEGFR2/KDR)** | **X** | |
| **CDH5** | | **X** |
| **CEACAM1** | **X** | |
| **CHGA** | **X** | |
| **COL15A1** | **X** | |
| **COL18A1** | **X** | |
| **COL4A1** | **X** | |
| **COL4A2** | **X** | |
| **COL4A3** | **X** | |
| **Connexin-43** | **X** | |
| **CSF3** | **X** | |
| **CTGF** | **X** | |
| **CXCL10** | | **X** |
| **CXCL12** | **X** | |
| **CXCL2** | **X** | |
| **DLX5** | | **X** |
| **DNMT3B** | **X** | |
| **ECGF1** | **X** | |
| **EDG1** | **X** | |
| **EDIL3** | **X** | |
| **ENPP2** | **X** | |
| **EPHB2** | **X** | |
| **F2** | **X** | |
| **FBLN5** | **X** | |
| **FGA** | | **X** |
| **FGF1** | **X** | |
| **FGF2** | **X** | |
| **FGF4** | | **X** |
| **FIGF** | **X** | |
| **FLT3** | | **X** |
| **FLT4** | **X** | |
| **FN1** | **X** | |
| **FOXC2** | **X** | |
| **Follistatin** | **X** | |
| **Galectin-1** | **X** | |
| **GRN** | **X** | |
| **HEY1** | **X** | |
| **HGF** | **X** | |
| **HLA-G** | | **X** |
| **HSPG2** | **X** | |
| **IFNB1** | **X** | |
| **IFNG** | | **X** |
| **IL-8** | **X** | |
| **IL-12A** | **X** | |
| **ITGA4** | **X** | |
| **ITGAV** | **X** | |
| **ITGB3** | **X** | |
| **KLF-4** | **X** | |
| **LECT1** | | **X** |
| **LEP** | | **X** |
| **MDK** | **X** | |
| **MMP-13** | | **X** |
| **MMP-2** | **X** | |
| **MYOZ2** | **X** | |
| **NANOG** | | **X** |
| **NESTIN** | | **X** |
| **NRP2** | **X** | |
| **PDGFB** | **X** | |
| **PF4** | **X** | |
| **PGK1** | **X** | |
| **PLG** | | **X** |
| **POU5F1 (OCT-4)** | | **X** |
| **PRL** | | **X** |
| **PROK1** | | **X** |
| **PROX1** | **X** | |
| **PTN** | **X** | |
| **SEMA3F** | **X** | |
| **SERPINB5** | **X** | |
| **SERPINC1** | **X** | |
| **SERPINF1** | **X** | |
| **SOX2** | | **X** |
| **TERT** | | **X** |
| **TGFA** | **X** | |
| **TGFB1** | **X** | |
| **THBS1** | **X** | |
| **THBS2** | **X** | |
| **TIE1** | **X** | |
| **TIMP2** | **X** | |
| **TIMP3** | **X** | |
| **TNF** | **X** | |
| **TNFSF15** | **X** | |
| **TNMD** | | **X** |
| **TNNC1** | **X** | |
| **TNNT2** | **X** | |
| **VASH1** | **X** | |
| **VEGF** | **X** | |
| **VEGFB** | **X** | |
| **VEGFC** | **X** | |
| **VEGFR1/FLT-1** | **X** | |
| **XLKD1** | | **X** |

In a separate experiment, AMDACs were additionally found to express genes for Aryl hydrocarbon receptor nuclear translocator 2 (ARNT2), nerve growth factor (NGF), brain-derived neurotrophic factor (BDNF), glial-derived neurotrophic factor (GDNF), neurotrophin 3 (NT-3), NT-5, hypoxia-Inducible Factor 1α (HIF1A), hypoxia-inducible protein 2 (HIG2), heme oxygenase (decycling) 1 (HMOX1), Extracellular superoxide dismutase [Cu-Zn] (SOD3), catalase (CAT), transforming growth factor β1 (TGFB1), transforming growth factor β1 receptor (TGFB1R), and hepatoycte growth factor receptor (HGFR/c-met).

### 6.2.2 Flow Cytometry for Evaluation of Amnion Derived Adherent Cells

Flow cytometry was used as a method to quantify phenotypic markers of amnion derived adherent cells to define the identity of the cells. Cell samples were obtained from frozen stocks. Prior to thaw and during reagent preparation, cell vials were maintained on dry ice. Subsequently, samples were thawed rapidly using a 37°C water bath. Pre-freeze cell counts were used for calculations for initial post-thaw cell number-dependent dilutions. Briefly, cryovials were thawed in a 37°C water bath for approximately 30 seconds with gentle agitation. Immediately following thawing, approximately 100-200 µL of cold (2 to 8°C) thawing solution (PBS with 2.5% albumin and 5% Gentran 40) was added to the cryovial and mixed. After gentle mixing, the total volume in the cryovials was transferred into a 15 mL conical tube containing an equal volume of cold (2 to 8°C) thawing solution. The cells were centrifuged in a conical tube at 400 g for 5 minutes at room temperature before removing the supernatant. The residual volume was measured with a pipette (estimation); the residual volume and cell pellet were resuspended at room temperature in 1% FBS in PBS to achieve a cell concentration of 250 x 10³ cells/100 µL buffer. For example, 1 x 10⁶ cells would be resuspended in 400 µL 1% FBS. The cell suspension was placed into pre-labeled 5 mL FACS tubes (Becton Dickinson (BD), Franklin Lakes, NJ). For each primary antibody isotype, 100 µL of cell suspension was aliquoted into one isotype control tube. Prior to phenotype analysis, the concentrations of all antibodies were optimized to achieve good signal to noise ratios and adequate detection of CD antigens across a potential four-log dynamic range. The volume of each isotype and sample antibody that was used to stain each sample was determined. To standardize the amount of antibody (in µg) in the isotype and sample tubes, the concentration of each antibody was calculated as (1/actual antibody concentration (µg/µL)) x (desired final quantity of antibody in µg for 2.5 x 10⁵ cells) = # µL of antibody added. A master mix of antibodies for both the isotype and the sample was made with the appropriate amount of antibody added to each tube. The cells were stained for 15-20 minutes at room temperature in the dark. After staining, unbound antibody in each sample was removed by centrifugation (400 g x 5 minutes) followed by washing using 2 mL 1% FBS PBS (room temperature) before resuspension in 150 µL of room temperature 1% FBS PBS. The samples were then analyzed on Becton Dickinson FACSCalibur, FACSCantoI or BD FACSCantoII flow cytometers prepared for use per manufacturer's instructions. Multi-parametric flow cytometry data sets (side scatter (SSC), forward scatter (FSC) and integrated fluorescence profiles (FL)) were acquired without setting on-the-fly instrument compensation parameters. Compensation parameters were determined after acquisition using the FACSDiva software according to the manufacturer's instructions. These instrument settings were applied to each sample. Fluorophore conjugates used in these studies were Allophycocyanin (APC), AlexaFluor 647 (AF647), Fluorescein isothiocyanate (FITC), Phycoerythrin (PE) and Peridinin chlorophyll protein (PerCP), all from BD Biosciences. Table 9 summarizes the expression of selected cell-surface markers, including angiogenic markers.

**Table 9: Cell surface marker expression in amnion derived adherent cells as determined by flow cytometry.**

| **AMDAC Marker** | **Positive** | **Negative** |
|---|---|---|
| **CD6** | | **X** |
| **CD9** | **X** | |
| **CD10** | **X** | |
| **CD31** | | **X** |
| **CD34** | | **X** |
| **CD44** | **X** | |
| **CD45** | | **X** |
| **CD49b** | **X** | |
| **CD49c** | **X** | |
| **CD49d** | **X** | |
| **CD54** | **X** | |
| **CD68** | **X** | |
| **CD90** | **X** | |
| **CD98** | **X** | |
| **CD105** | **X** | |
| **CD117** | | **X** |
| **CD133** | | **X** |
| **CD143** | | **X** |
| **CD144 (VE-cadherin)** | | **X** |
| **CD146** | | **X** |
| **CD166** | **X** | |
| **CD184** | | |
| **CD200** | **X** | |
| **CD202b** | **X** | |
| **CD271** | | **X** |
| **CD304** | **X** | |
| **CD309 (VEGFR2/KDR)** | **X** | |
| **CD318** | **X** | |
| **CD349** | **X** | |
| **CytoK** | **X** | |
| **HLA-ABC+ B2 Micro+** | **X** | |
| **Invariant Chain+ HLA-DR-DP-DQ+** | | **X** |
| **PDL-1** | **X** | |
| **VEGFR1/FLT-1** | **X** | |

In another experiment, AMDAC cells were labeled with anti-human CD49f (Clone GoH3, phycoerythrin-conjugated; BD Pharmingen Part No. 555736), and analyzed by flow cytometry. Approximately 96% of the AMDACs labeled with anti-CD49f (that is, were CD49f⁺).

In other experiments, AMDACs were additionally found by immunolocalization to express CD49a, CD 106, CD119, CD130, c-met (hepatocyte growth factor receptor; HGFR), CXC chemokine receptor 1 (CXCR1), PDGFRA, and PDGFRB by immunolocalization. AMDACs were also found, by immunolocalization, to lack expression of CD49e, CD62E, fibroblast growth factor receptor 3 (FGFR3), tumor necrosis factor receptor superfamily member 12A (TNFRSF12A), insulin-like growth factor 1 receptor (IGF-1R), CXCR2, CXCR3, CXCR4, CXCR6, chemokine receptor 1 (CCR1), CCR2, CCR3, CCR4, CCR5, CCR6, CCR7, CCR8, CCR9, epidermal growth factor receptor (EGF-R), insulin receptor (CD220), interleukin receptor 4 (IL4-R; CD124), IL6-R (CD126), TNF-R1a and 1b (CD120a, b), and erbB2/Her2.

### 6.2.3 Immunohistochemistry (IHC)/Immunofluorochemistry (IFC) for Evaluation of Angiogenic Potency of Amnion Derived Adherent Cells

Amnion derived adherent cells from passage 6 were grown to approximately 70% confluence on 4-well chamber slides and fixed with a 4% formalin solution for 30 minutes each. After fixation, the slides were rinsed with PBS two times for 5 minutes. The slides were then incubated with 10% normal serum from the same host as the secondary antibody, 2x casein, and 0.3% Triton X100 in PBS, for 20 minutes at room temperature in a humid chamber. Excess serum was blotted off and the slides were incubated with primary antibody (goat polyclonal IgG (Santa Cruz; Santa Cruz, CA) in a humidified chamber. Time and temperature for incubations were determined by selecting the optimal conditions for the antibody being used. In general, incubation times were 1 to 2 hours at 37°C or overnight at 4°C. The slides were then rinsed with PBS three times for 5 minutes each and incubated for 20-30 minutes at room temperature in a humid chamber with fluorescent-conjugated anti-immunoglobulin secondary antibody directed against the host of the primary antibody (rabbit anti-goat antibody (Santa Cruz)). Thereafter, the slides were rinsed with PBS three times for 5 minutes each, mounted with a coverslip utilizing DAPI VECTASHIELD® (Vector Labs) mounting solution to counterstain nuclei. Cell staining was visualized utilizing a Nikon fluorescence microscope. All pictures were taken at equal exposure time normalized against the background of the corresponding isotype (goat IgG (Santa Cruz)). Table 10 summarizes the results for the expression of angiogenic proteins by amnion derived adherent cells.

**Table 10: Angiogenic markers present or absent on amnion derived adherent cells.**

| **AMDAC Marker** | **Positive** | **Negative** |
|---|---|---|
| | | |
| **CD31** | | **X** |
| **CD34** | | **X** |
| **(VEGFR2/KDR)** | **X** | |
| **Connexin-43** | **X** | |
| **Galectin-1** | **X** | |
| **TEM-7** | **X** | |

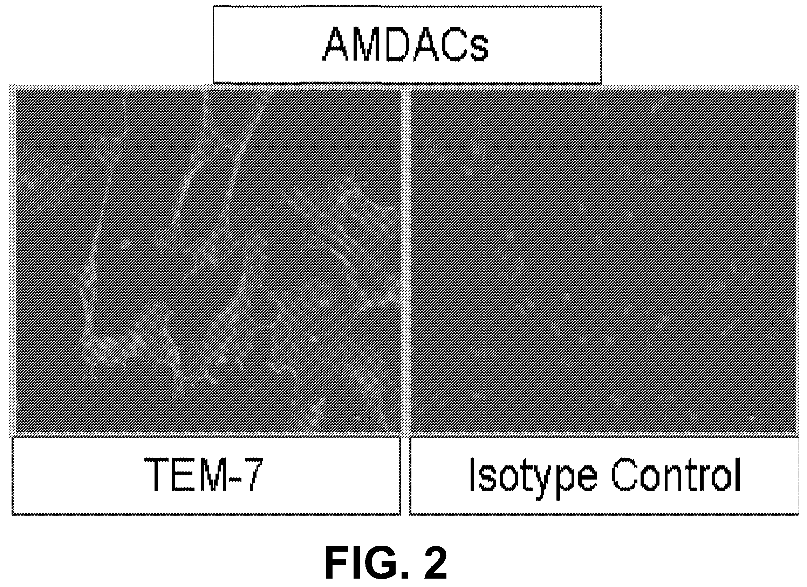

Amnion derived adherent cells expressed the marker tumor endothelial marker 7 (TEM-7), one of the proteins shown in Table 10. *See* Figure. 2.

### 6.2.4 Membrane Proteomics for Evaluation of Angiogenic Potency of Amnion Derived Adherent Cells

*Membrane Protein Purification*: Cells at passage 6 were grown to approximately 70% confluence in growth medium, trypsinized, and washed in PBS. The cells were then incubated for 15 minutes with a solution containing protease inhibitor cocktail (P8340, Sigma Aldrich, St. Louis, MO) prior to cell lysis. The cells were then lysed by the addition of a 10 mM HCl solution (thus avoiding the use of detergents) and centrifuged for 10 minutes at 400 g to pellet and remove the nuclei. The post-nuclear supernatant was transferred to an ultracentrifugation tube and centrifuged using a WX80 ultracentrifuge with a T-1270 rotor (Thermo Fisher Scientific, Asheville, NC) at 100,000 g for 150 minutes generating a membrane protein pellet.

*Generation, Immobilization and Digestion of Proteoliposomes:* The membrane protein pellet was washed several times using Nanoxis buffer (10 mM Tris, 300 mM NaCl, pH 8). The membrane protein pellet was suspended in 1.5 mL of Nanoxis buffer and then tip-sonicated using a VIBRA-CELL™ VC505 ultrasonic processor (Sonics & Materials, Inc., Newtown, CT) for 20 minutes on ice. The size of the proteoliposomes was determined by staining with FM1-43 dye (Invitrogen, Carlsbad, CA) and visualization with fluorescence microscopy. The protein concentration of the proteoliposome suspension was determined by a BCA assay (Thermo Scientific). The proteoliposomes were then injected onto an LPI™Flow Cell (Nanoxis AB, Gothenburg, Sweden) using a standard pipette tip and allowed to immobilize for 1 hour. After immobilization, a series of washing steps were carried out and trypsin at 5 µg/mL (Princeton Separations, Adelphi, NJ) was injected directly onto the LPI™ Flow Cell. The chip was incubated overnight at 37°C and the tryptic peptides were eluted from the LPI™ chip and then desalted using a Sep-Pak cartridge (Waters Corporation, Milford, MA).

*LTQ Linear Ion Trap LC*/*MS*/*MS Analysis:* Each tryptic digest sample was separated on a 0.2 mm x 150 mm 3µm 200 Å MAGIC C18 column (Michrom Bioresources, Inc., Auburn, CA) that was interfaced directly to an axial desolvation vacuum-assisted nanocapillary electrospray ionization (ADVANCE) source (Michrom Bioresources, Inc.) using a 180 minute gradient (Buffer A: Water, 0.1% Formic Acid; Buffer B: Acetonitrile, 0.1% Formic Acid). The ADVANCE source achieves a sensitivity that is comparable to traditional nanoESI while operating at a considerably higher flow rate of 3 µL/min. Eluted peptides were analyzed on an LTQ linear ion trap mass spectrometer (Thermo Fisher Scientific, San Jose, CA) that employed ten data-dependent MS/MS scans following each full scan mass spectrum. Seven analytical replicate datasets were collected for each biological sample.

*Bioinformatics:* Seven RAW files corresponding to the 7 analytical replicate datasets that were collected for each cell line were searched as a single search against the IPI Human Database using an implementation of the SEQUEST algorithm on a Sorcerer Solo™ workstation (Sage-N Research, San Jose, CA). A peptide mass tolerance of 1.2 amu was specified, oxidation of methionine was specified as a differential modification, and carbamidomethylation was specified as a static modification. Scaffold software implementation of the Trans-Proteomic Pipeline (TPP) was used to sort and parse the membrane proteomic data. Proteins were considered for analysis if they were identified with a peptide probability of 95%, protein probability of 95% and 1 unique peptide. Comparisons between membrane proteomic datasets were made using custom Perl scripts developed in-house.

*Results:* As shown in Table 11, amnion derived adherent cells expressed various angiogenic and cardiomyogenic markers.

**Table 11: Cardiomyogenic or angiogenic markers expressed by amnion derived adherent cells.**

| **AMDAC Marker** | **Positive** | **Negative** |
|---|---|---|
| | | |
| **Activin receptor type IIB** | **X** | |
| **ADAM 17** | **X** | |
| **Alpha-actinin 1** | **X** | |
| **Angiotensinogen** | **X** | |
| **Filamin A** | **X** | |
| **Macrophage acetylated LDL receptor I and II** | **X** | |
| **Megalin** | **X** | |
| **Myosin heavy chain non muscle type A** | **X** | |
| **Myosin-binding protein C cardiac type** | **X** | |
| **Wnt-9** | **X** | |

### 6.2.5 Secretome Profiling for Evaluation of Angiogenic Potency of Amnion Derived Adherent Cells

*Protein Arrays:* Amnion derived adherent cells at passage 6 were plated at equal cell numbers in growth medium and conditioned media were collected after 4 days. Simultaneous qualitative analysis of multiple angiogenic cytokines/growth factors in cell-conditioned media was performed using RayBiotech Angiogenesis Protein Arrays (Norcross, GA). In brief, protein arrays were incubated with 2 mL 1X Blocking Buffer (Ray Biotech) at room temperature for 30 minutes (min) to block membranes. Subsequently, the Blocking Buffer was decanted and the membranes were incubated with 1 mL of sample (growth medium conditioned by the respective cells for 4 days) at room temperature for 1 to 2 hours. The samples were then decanted and the membranes were washed 3 x 5 min with 2 mL of 1X Wash Buffer I (Ray Biotech) at room temperature with shaking. Then, the membranes were washed 2 x 5 min with 2 mL of 1X Wash Buffer II (Ray Biotech) at room temperature with shaking. Thereafter, 1 mL of diluted biotin-conjugated antibodies (Ray Biotech) was added to each membrane and incubated at room temperature for 1-2 hours and washed with the Wash Buffers as described above. Diluted HRP-conjugated streptavidin (2 mL) was then added to each membrane and the membranes were incubated at room temperature for 2 hours. Finally, the membranes were washed again, incubated with the ECL™ detection kit (Amersham) according to specifications and the results were visualized and analyzed using the Kodak Gel Logic 2200 Imaging System. The secretion of various angiogenic proteins by AMDACs is shown in Figure 3.

*ELISAs:* Quantitative analysis of single angiogenic cytokines/growth factors in cell-conditioned media was performed using commercially available kits from R&D Systems (Minneapolis, MN). In brief, ELISA assays were performed according to manufacturer's instructions and the amount of the respective angiogenic growth factors in the conditioned media was normalized to 1 X 10⁶ cells. Amnion derived adherent cells (n = 6) exhibited approximately 4500 pg VEGF per million cells and approximately 17,200 pg IL-8 per million cells.

**Table 12: ELISA results for angiogenic markers**

| **AMDAC Marker** | **Positive** | **Negative** |
|---|---|---|
| | | |
| **ANG** | **X** | |
| **EGF** | **X** | |
| **ENA-78** | **X** | |
| **FGF2** | **X** | |
| **Follistatin** | **X** | |
| **G-CSF** | **X** | |
| **GRO** | **X** | |
| **HGF** | **X** | |
| **IL-6** | **X** | |
| **IL-8** | **X** | |
| **Leptin** | **X** | |
| **MCP-1** | **X** | |
| **MCP-3** | **X** | |
| **PDGFB** | **X** | |
| **PLGF** | **X** | |
| **Rantes** | **X** | |
| **TGFB1** | **X** | |
| **Thrombopoietin** | **X** | |
| **TIMP1** | **X** | |
| **TIMP2** | **X** | |
| **uPAR** | **X** | |
| **VEGF** | **X** | |
| **VEGFD** | **X** | |

In a separate experiment, AMDACs were confirmed to also secrete angiopoietin-1, angiopoietin-2, PECAM-1 (CD31; platelet endothelial cell adhesion molecule), laminin and fibronectin. Other experiments confirmed that the AMDACs additionally secreted matrix metalloprotein (MMP) 1, MMP7, MMP9 and MMP10.

### 6.2.6 AMDAC MicroRNA Expression

This Example demonstrates that AMDACs express higher levels of certain micro-RNAs (miRNAs), and lower levels of certain other miRNAs, each of which correlated with angiogenic function, than bone marrow-derived mesenchymal stem cells.

It is known that pro-angiogenic miR-296 regulates angiogenic function through regulating levels of growth factor receptors. For example, miR-296 in endothelial cells contributes significantly to angiogenesis by directly targeting the hepatocyte growth factor-regulated tyrosine kinase substrate (HGS) mRNA, leading to decreased levels of HGS and thereby reducing HGS-mediated degradation of the growth factor receptors VEGFR2 and PDGFRb. *See* Würdinger et al., Cancer Cell 14:382-393 (2008). In addition, miR-15b and miR-16 have been shown to control the expression of VEGF, a key pro-angiogenic factor involved in angiogenesis, and that hypoxia-induced reduction of miR-15b and miR-16 contributes to an increase in VEGF, a pro-angiogenic cytokine. *See* Kuelbacher et al., Trends in Pharmacological Sciences, 29(1):12-15 (2007).

AMDACs were prepared as described in Example 1, above. AMDACs and BM-MSC cells (used as a comparator) were subjected to microRNA (miRNA) preparation using a MIRVANA™ miRNA Isolation Kit (Ambion, Cat# 1560). 0.5 x 10⁶ to 1.5 x 10⁶ cells were disrupted in a denaturing lysis buffer. Next, samples were subjected to acid-phenol+chloroform extraction to isolate RNA highly enriched for small RNA species. 100% ethanol was added to bring the samples to 25% ethanol. When this lysate/ethanol mixture was passed through a glass fiber filter, large RNAs were immobilized, and small RNA species were collected in the filtrate. The ethanol concentration of the filtrate was then increased to 55%, and the mixture was passed through a second glass fiber filter where the small RNAs became immobilized. This RNA was washed, and eluted in a low ionic strength solution. The concentration and purity of the recovered small RNA was determined by measuring its absorbance at 260 and 280 nm.

AMDACs were found to express the following angiogenic miRNA: miR-17-3p, miR-18a, miR-18b, miR-19b, miR-92, miR-20a, miR-20b, (members of the of the angiogenic miRNA cluster 17-92), miR-296, miR-221, miR-222, miR-15b, miR-16. AMDACs were also found to express higher levels of the following angiogenic miRNA when compared to bone marrow-derived mesenchymal stem cells (BM-MSCs): miR-17-3p, miR-18a, miR-18b, miR-19b, miR-92 (members of the of the angiogenic miRNA cluster 17-92), miR-296. These results correlate well with the observation that AMDACs express high levels of VEGFR2/KDR *(see* above). Conversely, AMDACs were found to express lower levels of the following angiogenic miRNA when compared to BM-MSCs: miR-20a, miR-20b, (members of the of the angiogenic miRNA cluster 17-92), miR-221, miR-222, miR-15b, miR-16. The reduced expression of miR-15b and miR-16 correlated with the higher levels of expression of VEGF seen in AMDACs.

### 6.3 EXAMPLE 3: DIFFERENTIATION OF AMNION DERIVED ADHERENT CELLS

### 6.3.1 Example 3.1: Osteogenic Non-Differentiation of Amnion Derived Adherent Cells

This Example demonstrates that amnion derived adherent cells (AMDACs) do not differentiate into osteogenic cells, as established by, *e.g*., von Kossa staining, which stains for mineralization, *e.g*., calcium deposited by cells.

Cryopreserved OCT-4⁻ AMDACs, obtained as described in Example 1 above, were thawed, washed to remove dimethylsulfoxide (DMSO) and re-suspended in growth medium. The cells were seeded at 5000 cells/cm² in 24-well plates and 6-well plates in growth medium and incubated overnight. Subsequently, the medium was removed and replaced with osteogenic medium comprising DMEM-low glucose, 10% v/v fetal bovine serum (FBS), 10 mM beta glycerophosphate (Sigma), 100 nM dexamethasone (Sigma), 100 µM ascorbic acid phosphate salt (Sigma), fungizone (Gibco), 50 units/ml penicillin, and 50 µg/ml streptomycin (Gibco). The osteogenic medium was supplemented with 20 ng/ml transforming growth factor-betal (TGF-β1) (Sigma), and 40 ng/ml human recombinant bone morphogenetic protein-2 (BMP-2) (Sigma). Culture of the AMDACs was continued in osteogenic medium for a total of 28 days with media changes every 3-4 days. At the end of the culture period, the cells were collected, washed, and stained as detailed below for evaluation of mineralization, an indicator or osteogenic differentiation. When observed under a microscope, the cell layer was fully confluent with fibroblastoid morphology (e.g., non-cuboidal in appearance), with no nodules observed.

As controls, dermal fibroblasts and bone marrow-derived mesenchymal stem cells (BM-MSCs) were cultured in the osteogenic medium as well. Adult normal human dermal fibroblasts (NHDF) were acquired from Lonza (Walkersville, MD, USA) and neonatal NHDF wew acquired from ATCC (Manassas, VA, USA). Three BM-MSC lines from different origin were evaluated: one from ScienCell Laboratories (Carlsbad, CA, USA), a second from Lonza (Walkersville, MD, USA), and a third was isolated from fresh whole normal bone marrow aspirates, obtained from AllCells (Emeryville, CA, USA).

Cells were fixed with 10% (v/v) neutral buffered methanol. After fixation, the cells were washed in deionized water and incubated in 5% Silver Nitrate (Aldrich) for 1 hour under indirect UV light. The cells were then washed in deionized water and incubated in 5% (w/v) sodium thiosulphate for 5 minutes. The cells were then washed again in distilled water and examined by light microscopy.

Differential expression levels of osteogenic differentiation-related genes bone sialoprotein (IBSP) and osteocalcin (BGLAP), before and after induction, were evaluated by RT-PCR. Specifically, the AMDACs were received at the end of the osteogenesis differentiation assay, then lysed using RLT lysis buffer (Qiagen). Cell lysates were stored at -80°C. AMDAC cell lysates were thawed, and RNA was isolated using an RNEasy kit (Qiagen) per manufacturer's instructions with DNAse treatment. RNA was then eluted with DEPC treated water, and the RNA quantity was determined using a Nanodrop ND1000 spectrophotometer. cDNA was made from the RNA using Applied Biosystems reverse transcription reagents. Real time PCR reactions were done using Taqman Universal PCR master mix from Applied Biosystems. Taqman gene expression assays used were Hs00173720 Bone Sialoprotein, Hs00609452 Osteocalcin, and GAPDH. Real time PCR reactions were run in an ABI 7300 system as shown below:

| Stage | Repetitions | Temperature | Time | Ramp Rate |
|---|---|---|---|---|
| 1 | 1 | 50.0°C | 2:00 | 100 |
| 2 | 1 | 95.0°C | 10:00 | 100 |
| 3 | 40 | 95.0°C | 0:15 per | 100 |
| | | 60.0°C | 1:00 per | 100 |

Interpretation of Threshold Cycle (Ct) values:
Average Ct 1-10 very high expression
Average Ct 10-20 high expression
Average Ct 20-30 medium level expression
Average Ct 30-35 low expression
Average Ct 35-40 very low expression

Expression values (Ct) of each gene were normalized to that of the housekeeping gene GAPDH. The normalized expression values (dCt) of each Sample were then compared pre- and post- induction. The relative differences, in terms of fold-change, were reported as "RQ". Due to the typical variability in dCt of housekeeping genes, any induction fold difference of less than 3 was not considered to be significant.

Results: Von Kossa staining results demonstrated that AMDACs were clearly nonosteogenic, as no von Kossa staining was detected. Control fibroblasts showed minimal mineralization, while BM-MSC displayed various degrees of mineralization.

**Table 13: von Kossa Staining Results**

| Cell Type | Donor ID | von Kossa Staining Intensity |
|---|---|---|
| AMDAC | 1 | - (Negative) |
| AMDAC | 2 | - (Negative) |
| Dermal Fibroblast | 3 | + (Borderline Positive) |

| adult normal | | |
|---|---|---|
| Dermal Fibroblast neonatal normal | 4 | - (Negative) |
| Bone Marrow MSC | 5 | ++++ (Positive) |
| Bone Marrow MSC | 6 | ++ (Positive) |
| Bone Marrow MSC | 7 | + (Borderline Positive) |

With respect to gene expression, all cells tested displayed moderate basal expression of osteocalcin (Ct 27.5 - 30.9). AMDACs demonstrated a marginal (< 2 fold) induction of osteocalcin expression that was not deemed to be significant when compared to the induction of osteocalcin expression observed for fibroblasts or BM-MSC. As such, the induction of osteocalcin expression by AMDACs was not indicative of osteogenic potential. In contrast, 2 out of 3 BM-MSC lines showed substantial up-regulation upon induction. Variation in BM-MSCs for induction of bone sialoprotein is possibly due to donor variation.

**Table 14: Gene Expression Results**

| Cell Type | Donor ID | Condition | BGLAP Ct | Avg. dCt | dCt St. Dev. | Fold Induction | GAPDH Ct |
|---|---|---|---|---|---|---|---|
| BGLAP (Osteocalcin) | | | | | | | |
| AMDAC | 2 | Basal | 28.2 | 10.2 | 0.07 | 1.6 | 18.0 |
| | | Induced | 29.2 | 9.5 | 0.12 | | 19.7 |
| Fibroblast | 3 | Basal | 28.2 | 10.0 | 0.11 | 0.8 | 18.2 |
| | | Induced | 28.4 | 10.4 | 0.12 | | 18.0 |
| | 4 | Basal | 29.5 | 10.8 | 0.24 | 0.6 | 18.6 |
| | | Induced | 30.7 | 11.6 | 0.18 | | 19.1 |
| BM-MSC | 5 | Basal | 27.7 | 9.9 | 0.09 | 0.3 | 17.8 |
| | | Induced | 30.9 | 11.7 | 0.14 | | 19.1 |
| | 6 | Basal | 27.5 | 9.9 | 0.12 | 0.3 | 17.6 |
| | | Induced | 29.8 | 11.6 | 0.07 | | 18.3 |
| | 7 | Basal | 27.0 | 9.5 | 0.10 | 0.3 | 17.6 |
| | | Induced | 29.8 | 11.0 | 0.16 | | 18.7 |

| IBSP (Bone Sialoprotein) | | | | | | | |
|---|---|---|---|---|---|---|---|
| AMDAC | 2 | Basal | > 40 | 17.7 | 0.10 | 0.13 | 18.0 |
| | | Induced | 38.6 | 20.6 | 0.12 | | 19.7 |
| Fibroblast | 3 | Basal | 35.8 | ND | 0.11 | NA | 18.2 |
| | | Induced | 38.6 | 18.9 | 0.12 | | 18.0 |
| | 4 | Basal | >40 | ND | 0.24 | NA | 18.6 |
| | | Induced | 38.2 | 19.1 | 0.18 | | 19.1 |
| BM-MSC | 5 | Basal | 33.6 | 15.8 | 0.09 | 0.066 | 17.8 |
| | | Induced | 38.9 | 19.7 | 0.14 | | 19.1 |
| | 6 | Basal | 35.7 | 18.1 | 0.12 | 4405 | 17.6 |
| | | Induced | 24.2 | 6.0 | 0.07 | | 18.3 |
| | 7 | Basal | 32.5 | 15.0 | 0.10 | 1508 | 17.6 |
| | | Induced | 23.1 | 4.4 | 0.16 | | 18.7 |

| | | | | | | | |
|---|---|---|---|---|---|---|---|
| ND - Not detected NA - Not able to calculate because uninduced condition was not detected (that is, no Ct value was determinable) | | | | | | | |

Fold induction values of 3 or less presented in Table 14 are not considered to be significant because of variability of expression of housekeeping genes used as comparators. Thus, based on the above results, it was concluded that AMDACs do not exhibit osteogenic potential.

### 6.3.2 Example 3.2: Chondrogenic Non-Differentiation of Amnion Derived Adherent Cells

This Example demonstrates that amnion derived adherent cells, as described herein, do not differentiate along a chondrogenic pathway.

OCT-4⁻ AMDACs as described elsewhere herein were used in a chondrogenesis assay, along with dermal fibroblasts and BM-MSCs as controls. For each test sample, 0.25×10⁶ cells were placed in a 15 mL conical tubes and centrifuged at 200×g for 5 minutes at room temperature to form a spherical pellet. Pellets were cultured either in chondrogenic induction medium (Lonza Chondrocyte Medium (Lonza PT-3003)) containing TGF beta-3 (10 ng/mL), recombinant human growth/differentiation factor-5 (rhGDF-5) (500 ng/mL), or a combination of TGF beta-3 (10 nanogram/milliliter), and rhGDF-5 (500 ng/mL)) or in growth medium (DMEM-low glucose (Gibco) + FBS (2% v/v) (Hyclone) + Penicillin and Streptomycin) for three weeks. During culture, full exchanges of media were performed twice a week.

At the end of the culture period, cell pellets were fixed in 10% formalin for 24 hours. All samples were then dehydrated through graded alcohols and were embedded in paraffin. Sections were cut to a thickness of 5 µm and then stained according to protocols as described below. The histological sections were examined using light microscopy.

Alcian Blue Staining: When used in a 3% acetic acid solution (pH 2.5), Alcian Blue stains both sulfated and carboxylated acid mucopolysaccharides and sulfated and/or carboxylated sialomucins. 1% Alcian Blue (Sigma-Aldrich # 23655-1) in 3% Acetic Acid was used, followed by a 0.1% Nuclear fast red (Sigma-Aldrich # 22911-3) counterstain. In brief, the sections were deparaffinized and hydrated through graded alcohols to distilled water, stained in Alcian Blue for 30 minutes, washed in running tap water for two minutes, rinsed in distilled water, then counterstained in nuclear fast red solution for 5 minutes, washed in running tap water for 1 minute, dehydrated through graded alcohols, cleared in xylene and finally mounted with resinous mounting medium.

Type II Collagen Staining: The presence of Type II Collagen in cell culture samples before and after chondrogenic differentiation conditions are evaluated by immunohistochemistry as outlined below. Collagen II production by the cells was assessed using antibody 5B2.5 (Abcam Cat. # ab3092), a mouse monoclonal highly specific to type II collagen and which displays no cross reaction with types I, III, IV, V, VI, IX, X, or XI collagens, and no cross-reaction with pepsin-digested type II collagen. The assay used goat anti-mouse AF 594 (Invitrogen IgG2a, Cat#A21135) as a secondary antibody. Cell pellets were fixed in 10% formalin for a minimum of 4 hours to overnight and were infiltrated in paraffin.

All cell samples were washed in PBS and exposed to protein blocking solution containing PBS, 4% goat serum and 0.3% Triton-100X for 30 minutes at room temperature. Primary antibodies diluted in blocking solution (1:50 and 1:100) were then applied overnight at 4°C. Next morning, samples were washed in PBS, and secondary antibodies (goat-anti-mouse AF594) diluted in blocking solution (1:500) were applied for 1 hr at room temperature. The cells were then washed in PBS and 600 nM DAPI solution was applied for 10 minutes at room temperature to visualize nuclei.

BM-MSCs and fibroblasts formed cell pellets in chondrogenic induction medium. Chondrocytes formed large cell pellet with no distinct cell populations apically or centrally. In contrast, AMDACs failed to form a cell pellet during the culture period. No staining results were obtained for AMDACs for either collagen II or Alcian Blue because AMDACs failed to form cell pellets. Therefore, it was concluded that AMDACs are non-chondrogenic.

### 6.3.3 Example 3.3: Neural Differentiation of Amnion Derived Adherent Cells

This Example demonstrates that amnion derived adherent cells can be differentiated to cells with characteristics of neural cells. Neural differentiation of the AMDACs was compared to that of normal human neuroprogenitors (Lonza), dermal fibroblasts, neonatal normal (Donor 3), Bone Marrow MSC (Donors 5 and 6).

In a first short term neural differentiation procedure, AMDACs and the other cells were thawed and expanded in their respective growth media after seeding at about 5000/cm² until they were sub confluent. Cells were trypsinized and seeded at 6000 cells per well in tissue culture-coated plate. All cells were initially expanded for 4 days in DMEM/F12 medium (Invitrogen) containing 15% v/v FBS (Hyclone), with basic fibroblast growth factor (bFGF) at 20 ng/ml, epidermal growth factor (EGF) at 20 ng/ml (Peprotech) and Penicillin/Streptomycin (PenStrep, Invitrogen). After 4 days, the cells were rinsed in PBS

(Invitrogen). The cells were then cultured in DMEM/F12 with 20% v/v FBS, PenStrep for about 24 hours. After 24 hours, the cells were rinsed with PBS (Invitrogen) and cultured in induction medium consisting of DMEM/F12, serum free, containing 200 mM butylated hydroxyanisole, 10 nM potassium chloride, 5 mgs/mL insulin, 10 nM forskolin, 4 nM valproic acid, and 2 nM hydrocortisone (Sigma). The cells were subsequently fixed at -20°C with 100% methanol. Fixed samples were then evaluated by immunohistochemistry (IHC) for expression of human nestin using an anti-nestin antibody (Alexa-Fluor 594 (Red) conjugated), with counterstaining with DAPI for nuclei.

In a second short term neural differentiation protocol, all cells were initially expanded for 4 days in DMEM/F12 medium (Invitrogen) containing 15% FBS (Hyclone), with basic FGF at 20 ng/ml, EGF at 20 ng/ml and PenStrep (Invitrogen). After 4 days, the cells were rinsed in PBS (Invitrogen) and were cultured in DMEM/F12 with 20% v/v FBS, PenStrep. After 24hrs, cells were rinsed with PBS. The media were then switched to Neural Progenitor Expansion medium (NPE), which comprised NEUROBASAL™-A basal medium (Gibco), with B27 (Gibco), 4 mM L-glutamine, 1 µM retinoic acid (Sigma), and PenStrep. After four days, the medium was removed from each well and cells were fixed with ice cold 4% w/v paraformaldehyde for 10 minutes at room temperature. Fixed samples were then evaluated by IHC for expression of GFAP (glial fibrillary acidic protein) for astrocyte phenotype, and TuJ1 (neuron-specific class III tubulin) for neuronal phenotype, respectively.

In the first differentiation protocol, all cell types transformed into a cell type with bipolar morphology and stained positive with nestin. Neuroprogenitors constitutively expressed nestin as expected. In the second differentiation protocol, expression of neuronal-related (Tuj 1) and astrocyte-related (GFAP) markers were evaluated. Upon induction, AMDAC, and BM-MSC expressed low levels of Tuj1. Expression on fibroblasts was found to be borderline positive which could be due to background. AMDACs, and one BM-MSC cell line, exhibited low-level expression of GFAP. The positive control cell line (neuroprogenitors) constitutively expressed both Tuj 1 and GFAP, as expected.

Thus, AMDACs are able, under neural inducing conditions, to exhibit morphological and biochemical changes consistent with neural differentiation.

### 6.4 EXAMPLE 4: IMMUNOMODULATION USING AMNION DERIVED ANGIOGENIC CELLS

This example demonstrates that AMDACs display immunosuppressive function in vitro in an assay utilizing bead-stimulated T cells.

### 6.4.1 AMDAC-Mediated Suppression of T Cell Proliferation

AMDACs were obtained as described in Example 1, above. CD4⁺ and CD8⁺ T cells were obtained from human peripheral blood.

The T cells were labeled with carboxyfluorescein succinimidyl ester (CFSE) and mixed with anti-CD3 anti-CD28-coated Dynabeads, followed by culture in the absence of the AMDACs or a coculture with the AMDACs in a manner that allowed cell to cell contact, also known as a Bead T-lymphocyte reaction (BTR). Coculture with the AMDACs was performed by mixing 100,000 T-lymphocytes with anti-CD3 and anti-CD28 coated DynaBeads (Invitrogen) at a bead:T-lymphocyte ratio of 1:3 in a well of a 96-well plate, in the presence or absence of 20,000 AMDAC cells. The mixed (coculture) and unmixed cell cultures were incubated at 37°C, 5% CO₂, and 90% relative humidity for 5 days. Normal human dermal fibroblasts (NHDF), which do not possess substantial T cell inhibitory activity were used as a negative control, and subjected to the same conditions as the AMDACs.

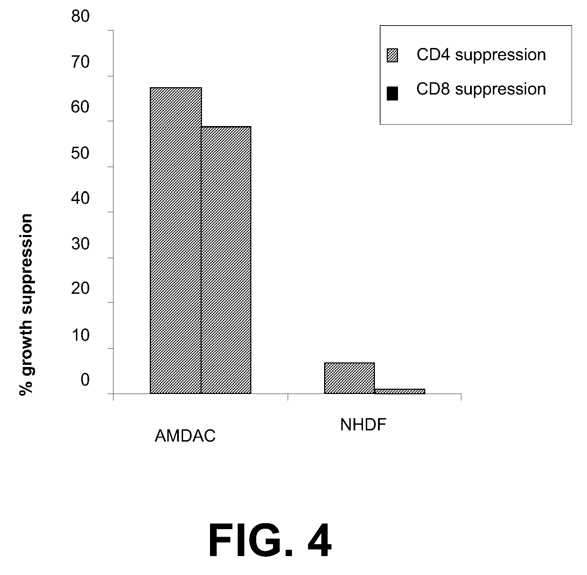

Following the 5 days, CFSE fluorescence on the CD4+ and CD8+ T cells was detected using flow cytometry, and the percentage of suppression of T cell growth was calculated based on the increased fraction of non-proliferated (CFSE high) T cells compared to the culture of CFSE-labeled T cells that were not co-cultured with AMDACs or NHDF. As demonstrated in **Figure** 4, AMDACs inhibit the proliferation of CD4⁺ and CD8⁺ T cells in vitro, indicating that AMDACs are immunomodulatory.

### 6.4.2 Media Conditioned by AMDACs Inhibits Secretion of TNF-Alpha by T cells

AMDACs were obtained as described in Example 1, above. T cells were obtained from human peripheral blood.

The AMDACs were seeded on tissue culture plates and incubated overnight to form an adherent monolayer. The next day, the AMDAC culture was stimulated with IL-1 beta, which has previously been shown to be a potent inducer of AMDAC-derived antiinflammatory factors. After 16 h of IL-1 beta stimulation, the medium conditioned by the AMDACs was collected and mixed at a 9:1 volume ratio with human peripheral blood T cells coated with anti-CD3 anti-CD28-coated Dynabeads. A separate population of human peripheral blood T cells coated with anti-CD3 anti-CD28-coated Dynabeads was maintained as a control. The T cells mixed with AMDAC-conditioned medium and the unmixed population of T cells were incubated at 37°C, 5% CO₂, and 90% relative humidity for 72 h. Medium conditioned by normal human dermal fibroblasts (NHDF), which do not possess substantial TNF-alpha inhibitory activity was used as a negative control, and subjected to the same conditions as the AMDACs.

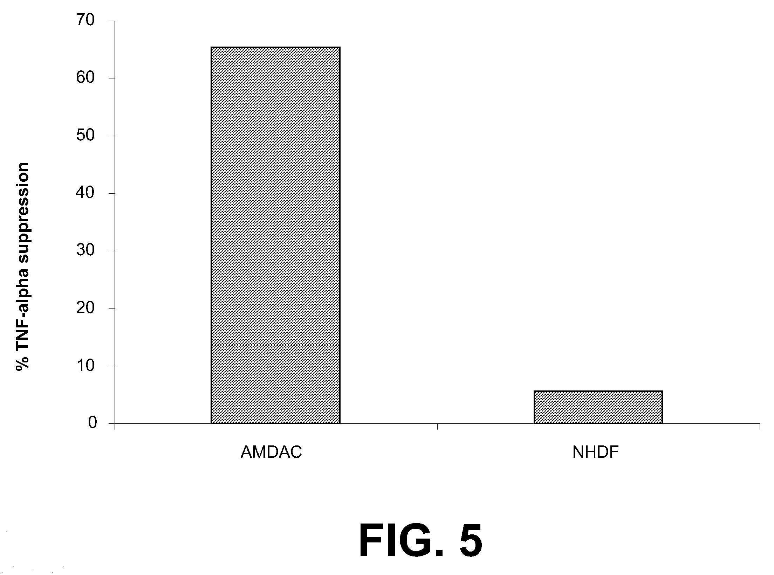

Following the 72 h culture, the concentration of T-cell derived TNF-alpha was measured in the T cell culture supernatants using a cytometric bead-based ELISA method. The percent suppression of TNF-alpha secretion was calculated based on the decrease of TNF-alpha concentration in the presence of AMDAC-conditioned medium compared to the control T cell culture which was not mixed with AMDAC-conditioned medium. As demonstrated in Figure 5, the culture of the T cells in the presence of AMDAC-conditioned medium induced the suppression of production of T cell derived TNF-alpha.

### 6.5 EXAMPLE 5: AMDACS MODULATE THE T CELL COMPARTMENT

This Example demonstrates that amnion derived adherent cells (AMDACs), obtained as described in Example 1, are able to influence skewing in the Th1, Th17 and FoxP3 T_{reg} subsets.

### 6.5.1 Methods

### T-lymphocyte proliferation assays

Mixed lymphocyte reactions (MLR) were performed by mixing 100,000 HLA-mismatched carboxyfluorescein succinimidyl ester (CFSE)-labeled T-lymphocytes with 10,000 mature dendritic cells (mDC) in each well of a FALCON flat bottom 96 well tissue culture plate (Fisher Scientific, Pittsburg, PA) in the presence or absence of 20,000 AMDAC cells, isolated as described in Example 1, above. The mixed cell culture was incubated at 37°C, 5% CO₂, and 90% relative humidity for 6 days. At day 6 all cells were recovered and stained with anti-CD4-PE and anti-CD8-APC (R&D systems, Minneapolis, MN).

Bead T-lymphocyte reactions (BTR) were performed by mixing 100,000 T-lymphocytes with anti-CD3 and anti-CD28 coated DynaBeads (Invitrogen) at a bead:T-lymphocyte ratio of 1:3 in a well of a 96-well plate. The BTR reaction was performed in the presence or absence of 20,000 AMDAC cells. The mixed cell culture was incubated at 37°C, 5% CO₂, and 90% relative humidity for 6 days. At day 6 all cells were recovered and stained with anti-CD4-PE and anti-CD8 APC (R&D systems, Minneapolis, MN).

T-lymphocyte proliferation was measured by analysis of CFSE fluorescent intensity on CD4 and CD8 single positive cells with a FACS Canto II machine (BD, Franklin Lake, NJ). All FACS data in this study were analyzed by using flowjo 8.7.1 software (Tree Star, InC. Ashland OR).

### T cell skewing (polarization)

Th1 skewing was carried out using BTR reactions with an additional Th1 skewing cytokine cocktail containing IL-2 (200 IU/ml), IL-12 (2 ng/ml) and anti-IL-4 (0.4 µg/ml).

For Th17 skewing, 5 x 10⁵ total T-lymphocytes were stimulated with 5x10⁵ sorted CD14⁺ monocytes, 50 ng/mL anti-CD3 antibody (BD BioScienences) and 100 ng/mL LPS (Sigma Aldrich) in either the presence or absence of 50,000 AMDACs for 6 days. The Th17 cell population was analyzed by intracellular cytokine staining (ICCS) staining of IL-17 on the CD4 positive population.

### Intracellular cytokine and Foxp3 staining

The Th1 cell subset was enumerated as follows. T cells from BTR reactions were re-activated with 50 ng/mL phorbol myristate acetate (PMA) and 750 ng/mL ionomycin (PI) (Sigma Aldrich) for 5 hours. GOLGISTOP™ (Becton Dickinson; a protein transport inhibitor) was added during the last 3 hours. Cells were then surface stained with PE labeled anti-CD4 antibody and subsequently with APC conjugated anti-IFN-γ antibody with the Cytofix/Cytoperm kit (Becton Dickinson) according to the manufacturer's instructions.

In order to enumerate the Th17 cell subset, T cells from a Th17 skewing activation reaction were re-activated with 50 ng/mL PMA and 750 ng/mL ionomycin (Sigma Aldrich) for 5 hours with GOLGISTOP™ (Becton Dickinson) present during the last 3 hours. Cells were then stained with PE labeled anti-CD4 antibody and subsequently with APC conjugated anti-IL-17 antibody with the Cytofix/Cytoperm kit (Becton Dickinson) according to the manufacturer's instructions.

In order to enumerate the Treg cell subset, T cells from BTR reactions were surface stained with PE labeled anti-CD4 antibody and subsequently with APC conjugated anti-Foxp3 antibody using the Foxp3 staining kit (eBioscience, San Diego, CA) according to the manufacturer's instructions.

### Dendritic cell differentiation and stimulation

Immature DC (iDC) were generated from a magnetically sorted CD14⁺ monocyte population by mitogen-directed differentiation. Briefly, iDCs were obtained from monocytes cultured at 1 x10⁶/ml with GM-CSF (20 ng/ml) and IL-4 (40 ng/ml) for 4 days. iDCs (1 x 10⁵ cells) were then stimulated with 1 µg/ml LPS for 24 hours in either the absence or presence of 1 x 10⁵ AMDACs in each well of a FALCON 24 well tissue culture plate (Fisher Scientific, Pittsburgh, PA). Culture supernatant was collected and the cytokine profile was analyzed by Cytometric Bead Array (CBA).

### Cytometric Bead Array (CBA) analysis

Cytokine concentrations were measured in culture supernatants using the Cytometric Bead Array system (CBA; Becton Dickinson) for the simultaneous quantitative detection of multiple soluble analytes according to the manufacturer's instructions. Briefly, samples of BTR culture supernatants were incubated with a mix of capture beads for specific detection of the following cytokines produced by activated T cells: IL-2, IL-4, IL-5, IL-10, TNF, lymphotoxin-alpha (LT-α) and IFN-γ. Subsequently, bead bound cytokines were coupled with fluorescently labeled detection reagents and detected using the FACSCanto II flow cytometer following the manufacturer's protocols. Data was acquired and analyzed using the FACS-DIVA 6.0 software (Becton Dickinson), followed by calculation of cytokine concentrations using the FCAP Array 1.0 program (Becton Dickinson).

### IL-21 ELISA

Soluble IL-21 was measured in supernatant obtained from Th17 skewing cultures with the IL-21 ELSAI kit from eBioscience (88-7216) according to the manufacturer's protocol.

### NK proliferation assay and NK cytotoxicity assay

Human NK cells were isolated from PBMC using an NK cell isolation kit (Miltenyi Biotech, Auburn, CA) according to the manufacturer's instructions. NK cell proliferation was determined by culturing 2.5 X 10⁵ NK cells in 1 ml IMDM containing 10% fetal bovine sera (FBS) (Hyclone) supplemented with 35 µg/ml transferrin, 5 µg/ml insulin, 20 µM ethanolamine, 1µg/ml oleic acid, 1µg/ml linoleic acid, 0.2 µg/ml palmitic acid, 2.5 µg/ml BSA, 0.1 µg/ml PHA (Sigma-Aldrich) and 200 IU/ml human IL-2 (R&D), together with mitomycin C treated (16 g/ml) feeder cells (either 1 x 10⁶ human allogeneic PBMC or 1 x 10⁵ K562 cells). Cells were incubated at 37°C in 5% CO₂ with the addition of an equal volume of IMDM (10% FBS, 2% human serum and 400 IU/ml IL-2) every 3 days. NK cell number was determined by FACS every seven days as follows. Briefly, total NK cells were collected from the tissue culture well. After washing with PBS, cells were then stained with 2 uM TO-PRO3. Finally, 10 µl counting beads (Spherotech, Cat# ACBP-50-10) were added to each sample which served as an internal standard for calibration of total cell number. Relative NK number was calculated based on the number of total live NK cells per 1000 counting beads collected.

The NK cytotoxicity assay was carried out by mixing NK cells with target cells at different effector/target (E/T) ratios. After overnight culture, target cell numbers were determined using the counting beads method described above plus cell surface markers to differentiate NK cells from target cells. For NK cytotoxicity of K562 cells, FITC conjugated anti-HLA-ABC antibodies were used as the NK cell marker, because K562 cells are HLA-ABC negative. For AMDAC cells, CD90-PE was used to distinguish AMDACs from NK and K562 cells. Percent cytotoxicity was calculated as (1 - total target number in sample ÷ total target cells in a control containing no NK cells) X 100.

### 6.5.2 AMDACs Skewing of T Cell Compartment

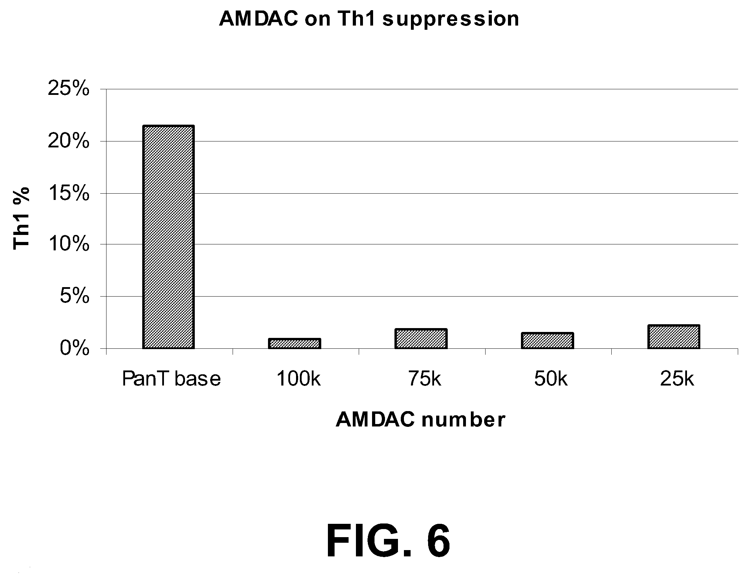

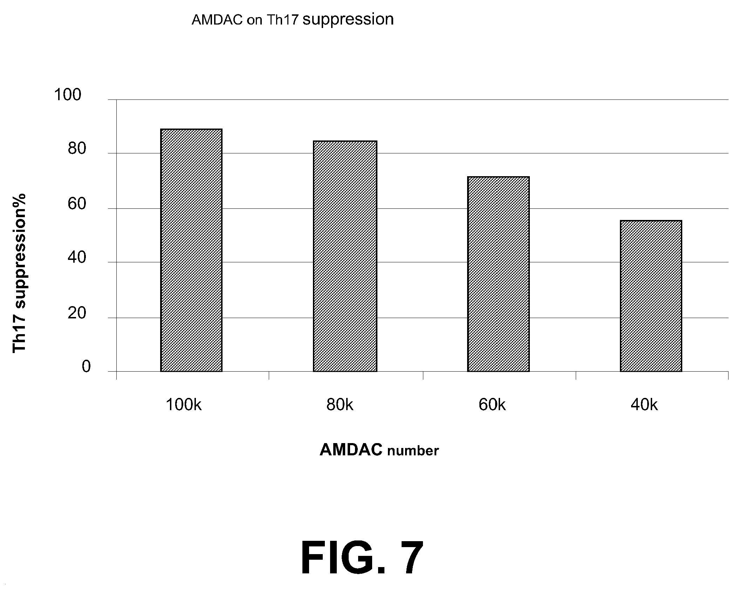

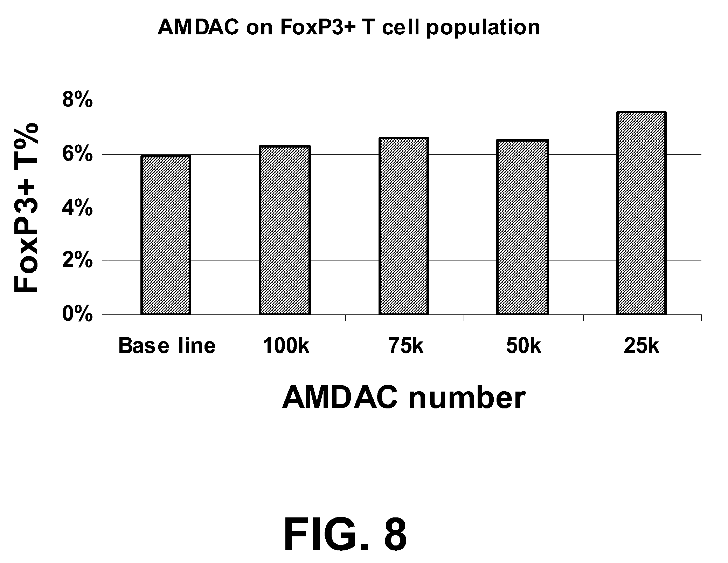

The ability of AMDACs to influence skewing in the T cell compartment was examined by measuring cytokine producing T cells in Th1 and Th17 skewing assays using T cell and AMDAC co-cultures. Briefly, in the Th1 skewing assay, AMDACs were pre-plated. The following day, 1x10⁶/ml T cells, Dynabeads at 6x10⁵/ml, IL-2 (200 IU/ml), IL-12 (2 ng/ml), and anti-IL-4 (0.4 µg/ml) were added and mixed with the AMDACs. Four days later, the percentage of Th1 cells was analyzed by interferon-gamma (IFN-γ) intracellular staining. As shown in Figure 6, AMDACs greatly reduced the Th1 percentage in a dose dependent manner. Similarly, in a Th17 skewing assay, AMDACs were pre-plated overnight. A mixture of T cells (1x10⁶/ml), CD14⁺ cells (1x10⁶/ml), anti-CD3 (50 ng/ml) and bacterial lipopolysaccharide (LPS) (100 ng/ml) was then added to the plate containing AMDACs. After a six day culture, the Th17 percentage was examined by IL-17 intracellular staining. As shown in Figure 7, AMDACs suppressed the Th17 percentage in a dose dependent manner. To investigate the effect of AMDACs on a FoxP3 positive T cell population, 1X 10⁶ PBMC were co-cultured with AMDACs for 6 days. The FoxP3 positive population was analyzed by FoxP3 intracellular staining. As shown in Figure 8, AMDACs slightly increased the FoxP3 positive T cell population.

### 6.5.3 AMDAC Mediated Modulation of DC Maturation and Function

This experiment demonstrates that AMDACs modulate the maturation and differentiation of immature dendritic cells (DCs).

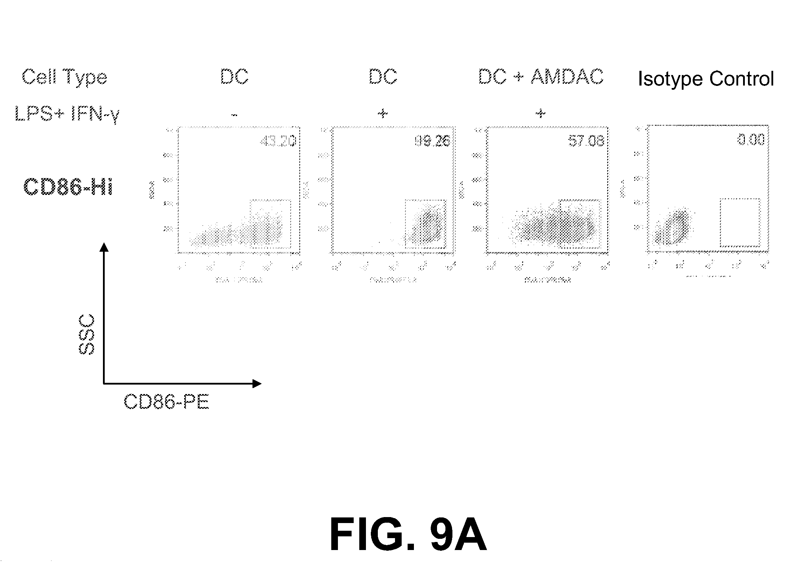

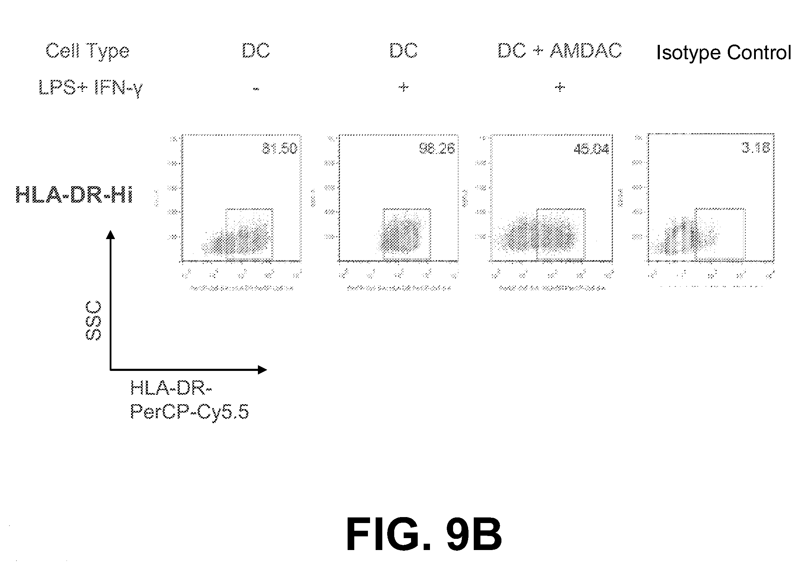

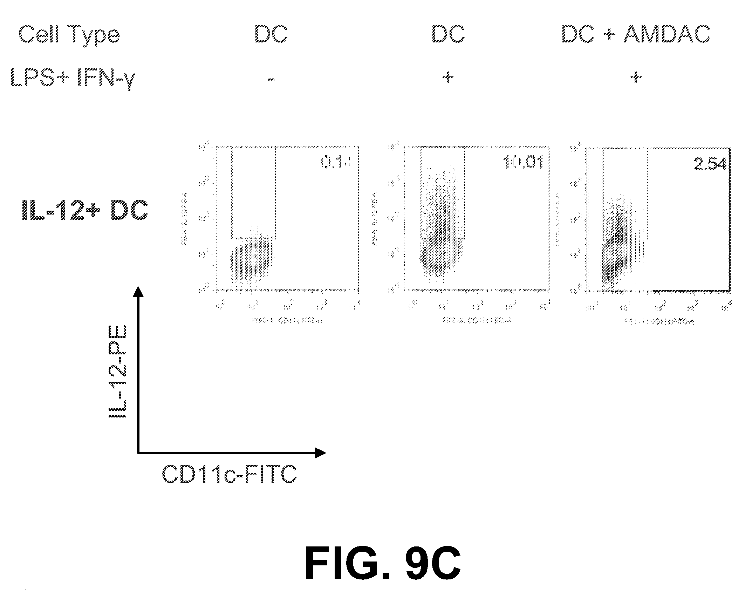

To explore the AMDAC mediated modulation of DC maturation and function, monocyte derived immature DCs were treated with LPS alone or a combination of LPS plus IFN-γ in the absence or presence of AMDACs to further drive the DC maturation process. DC maturation was analyzed by FACS staining of DC maturation markers CD86 and HLA-DR. DC function was assessed by intracellular staining of IL-12 and measurement of soluble cytokine production by CBA. As shown in Figures 9A and 9B, AMDACs strongly suppressed LPS and LPS plus IFN-γ-induced DC maturation by down-modulation of CD86 (Figure 9A) and HLA-DR expression (Figure 9B) on DCs. Further, as shown in Figure 9C, AMDACs significantly suppressed the LPS plus IFN-γ-stimulated IL-12-producing DC population by ~70%. AMDACs were further found to be able to suppress TNF-α and IL-12 production by LPS-stimulated DCs. *See* Figure 10.

### 6.5.4 AMDACs Suppress IL-21 Production in a Th17 Skewing Culture

IL-21 is an important cytokine required for maintenance of a Th17 population. To investigate whether AMDACs are able to modulate IL-21 production, AMDACs were introduced into a Th17 skewing culture as described in the Methods section. AMDACs suppressed IL-21 production by 72.35% in AMDAC-Th17 co-cultures in comparison to a Th17 skewing culture without AMDAC cells. Additionally, AMDACs reduced the population of Th17 T cells by 72.65% as compared to culture in the absence of AMDACs.

### 6.5.5 AMDAC modulation of NK cell cytotoxicity and proliferation

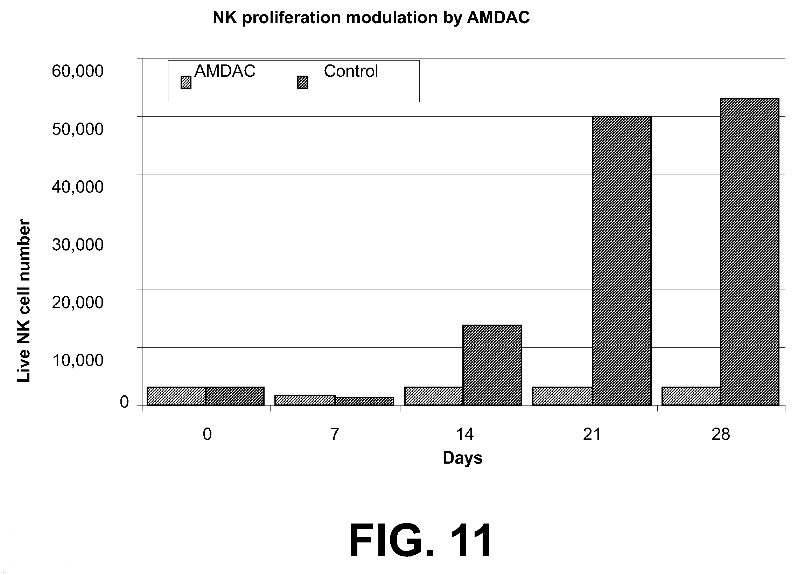

NK cells are a type of cytotoxic lymphocyte that constitutes a major component of the innate immune system. NK cells play a major role in the rejection of tumors and cells infected by viruses as well as allogeneic cells and tissues. To investigate the immunomodulatory effect of AMDACs on NK cells, NK cell proliferation and cytotoxicity assays were established. As shown in Figure 11, AMDACs suppressed human NK cell proliferation in comparison to a control having no AMDAC cells.

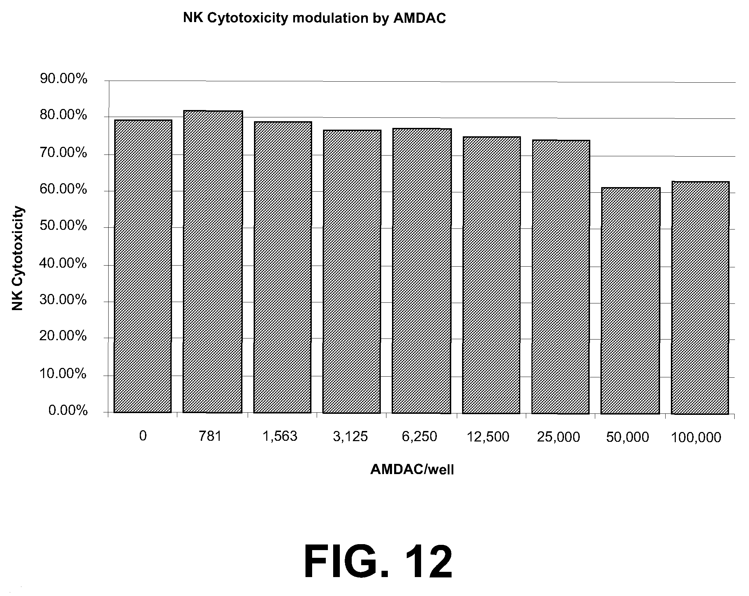

In addition, the effect of AMDACs on NK cell cytotoxicity was investigated. In this assay, AMDACs were introduced into an NK cytotoxicity assay as described in the Methods section above. Briefly, 1 x 10⁶ NK cells were mixed with 1 x 10⁵ K562 cells (E/T ratio of 10:1) with a 2 fold titration of pre-seeded AMDACs (1 x 10⁵ cells). The NK cells and K562 cells were co-cultured overnight, and NK cell cytotoxicity was determined according to the protocol described in the Methods section above. As shown in Figure 12, AMDAC cells suppressed human NK cell cytotoxicity in a dose dependent manner.

### 6.6 EXAMPLE 6: METHODS OF TREATMENT USING AMNION DERIVED ADHERENT CELLS

### 6.6.1 Treatment of Inflammatory Bowel Disease Using AMDACs

An individual presents with abdominal cramps and bloody diarrhea. The symptoms have, intermittently, gradually worsened over the past year. After excluding colon cancer, a diagnosis of ulcerative colitis is made. The individual is administered 1-5 x 10⁸ OCT-4⁻ amnion derived adherent cells (AMDACs) in a 0.9% NaCl solution intravenously. The individual is monitored over the subsequent two weeks to assess reduction in one or more of the symptoms, *e.g*., reduction in the amount of blood in stools. The individual is additionally monitored over the course of the following year, and AMDACs in the same dose are administered as needed, *e.g.,* if symptoms return.

### 6.6.2 Treatment of Crohn's Disease Using AMDACs

An individual presents with ileocolitis, a form of Crohn's disease, and is experiencing abdominal pain, bloody diarrhea, and fever. The individual is administered 1-5 x 10⁸ OCT-4⁻ amnion derived adherent cells (AMDACs) in a 0.9% NaCl solution intravenously. The individual is monitored over the subsequent two weeks to assess reduction in one or more of the symptoms. The individual is additionally monitored over the course of the following year, and AMDACs in the same dose are administered as needed, *e.g.,* if symptoms return.

### 6.6.3 Treatment of Rheumatoid Arthritis Using AMDACs

An individual presents with rheumatoid arthritis in three or more joints. The individual is administered a combination of OCT-4⁻ AMDACs and OCT-4⁻ AMDACs that have been modified to produce a fusion polypeptide comprising IL-1Ra and DHFR, wherein the two types of stem cells are administered in a 1:1 ratio. The engineered and non-engineered cells are 1-5 x 10⁸ OCT-4⁻ AMDACs in a 0.9% NaCl solution. The individual is given methotrexate at a standard dosage and monitored for reduction in joint inflammation. The individual is additionally monitored over the course of the following year, and AMDACs in the same dose are administered as needed, *e.g*., if symptoms return.

### 6.6.4 Treatment of Diabetes Using AMDACs

An individual presents with blurred vision, frequent urination, increased thirst and increased hunger that has developed over the previous two months. Blood tests confirm elevated blood glucose levels (greater than 7.0 mmol/L glucose or greater than 126 mg/dL glucose). A diagnosis of diabetes mellitus, Type 1, is made. The individual is administered OCT-4⁻ amnion derived adherent cells (AMDACs) in a 0.9% NaCl solution intravenously, and the individual's blood glucose is monitored for the next six months. The administration is considered successful if the individual's blood glucose levels drop to less than 7.0 mmol/L glucose, or less than 126 mg/dL glucose. Administration is repeated if, upon further follow up, the individual's blood glucose levels are again found to exceed 7.0 mmol/L glucose or 126 mg/dL glucose.

### 6.6.5 Combination Treatment of Graft-Versus-Host Disease Using AMDACs

An individual awaiting an allogeneic bone marrow transplant is administered OCT-4⁻ amnion derived adherent cells (AMDACs) in a 0.9% NaCl solution intravenously within 24 hours prior to bone marrow transplantation. Administration of the stem cells is repeated within 24 hours after bone marrow transplantation. The individual is monitored over the next 100 days, and is administered a follow-up dose of 5-10 x 10⁸ OCT-4-AMDACs if GVHD develops and progresses beyond Grade I.

### 6.7 EXAMPLE 7: USE OF AMDACS FOR THE TREATMENT OF ADULTS WITH MODERATE-TO-SEVERE CROHN'S DISEASE.

Twelve patients with active moderate-to-severe Crohn's disease who are unresponsive to at least 1 prior therapy are given 2 infusions of OCT4- AMDACs one week apart. Patients are then monitored every week for 4 additional weeks, being reassessed at 6 months post infusion. The first 6 patients receive 2 infusions of 2 x 10⁸ cells (Low Dose) and the next 6 receive 2 infusions of 8 x 10⁸ cells (High Dose). Concomitant medications are maintained throughout the study. Disease activity is measured using the Crohn's Disease Activity Index (CDAI). Subjects also complete the Inflammatory Bowel Disease Questionnaire (IBDQ). The mean age of the patients is 35-40 years. All patients are monitored for improvement in the Crohn's disease by the CDAI that exists for at least 2 consecutive visits, *e.g*., at 4 weeks and 6 months, and for remission (CDAI <150) that is maintained for at least 2 consecutive visits.

### 6.8 EXAMPLE 8: AMDAC EFFECTIVENESS IN A MULTIPLE SCLEROSIS MODEL

This Example demonstrates the ability of OCT-4⁻, CD49f+ AMDACs to improve survival times, and reduce symptoms, in a mouse Experimental Autoimmune Encephalitis (EAE) model of chronic multiple sclerosis. EAE symptoms were induced by a combination of Myelin Oligodendrocyte Glycoprotein (MOG33-55) in Complete Freund's Adjuvant (CFA) emulsion, and Pertussis Toxin (PTx).

Experimental female C57BL/6 mice (Taconic), age 30 days, were separated into four Groups: (1) 1.5 x 10⁶ cells of an OCT4-, CD49f+ population of AMDACs; (2) Vehicle control; (3) Disease control group (CFA/PTx, no MOG35-55 treatment); and (4) untreated mice receiving MOG35-55/CFA/PTx, but no AMDACs. Groups 1-3 and 5 consisted of 10 mice each, and Group 4 consisted of 5 mice. Each mouse in Groups 1-4 was administered 400 microliters of MOG35-55/CFA/PTx and AMDACs, vehicle, CFA/PTx, or MOG35-55/CFA/PTx, respectively. Symptoms of multiple sclerosis were induced in mice in Groups 1, 2 and 4 by administration of MOG35-55/CFA, in combination with PTx. MOG35-55 was administered on Day 0, and PTx was administered on Day 1, with or without AMDACs. Group 4 received CFA and PTX, but no MOG35-55. Animal weights were taken every 2-3 days post-administration, and assessment of development of symptoms over the 32 days following administration were scored every day post-administration using the following scale:

| | |
|---|---|
| 0 | No obvious changes in motor functions of the mouse in comparison to non-immunized mice. When picked up by the tail, the tail has tension and is erect. Hind legs are usually spread apart. When the mouse is walking, no gait or head tilting. |
| 1 | The tail is limp. Instead of being erect, the tail drapes over fingers. |
| 2 | Limp tail coupled with hind limb weakness. When picked up by the tail, the mouse's hind legs are not spread apart. Mouse has a clearly wobbly walk. |
| 3 | Mouse displays limp tail and complete hind limb paralysis, or |
| | Mouse displays limp tail and at least one paralyzed forelimb, or |
| | Mouse displays all of head tilting, walking along cage periphery, pushing at cage wall, and spinning when picked up by tail. |
| 4 | Limp tail plus complete hind limb and partial forelimb paralysis, though mouse is alert and feeding. |

Scores were given half values at the experimenters' discretion. Onset of symptoms typically occurred at or around Day 10.

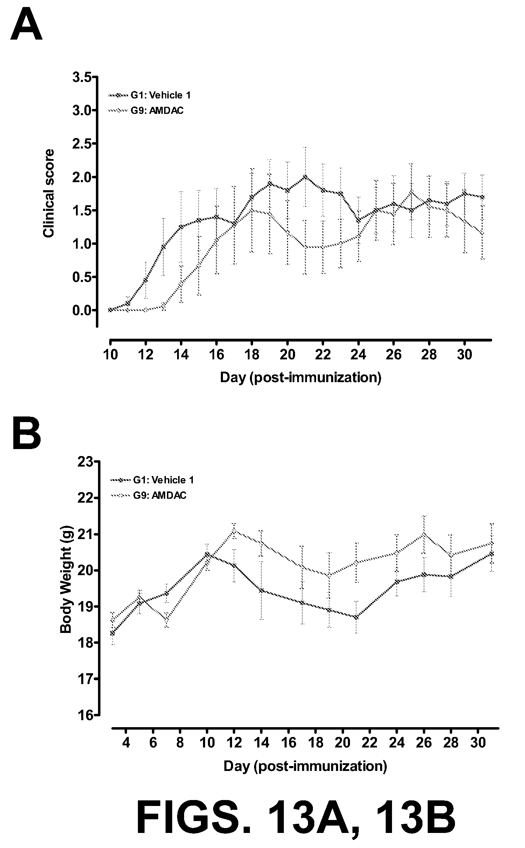

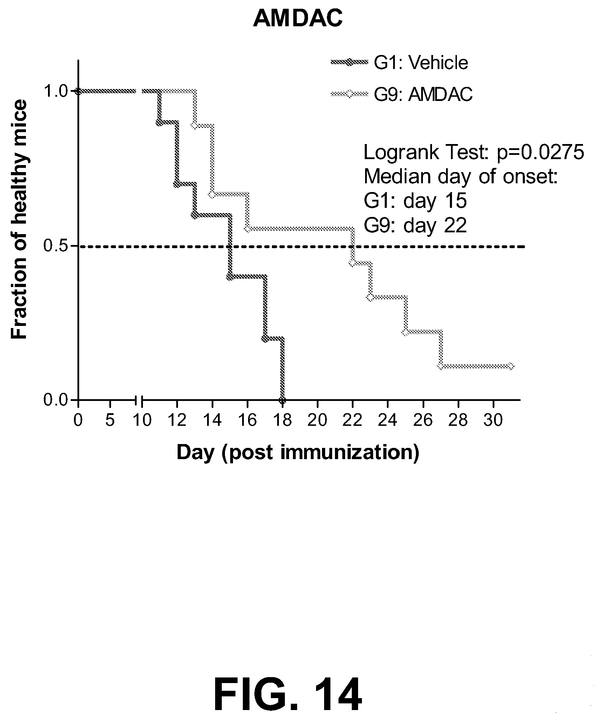

Clinical scores for mice receiving AMDACs (G9) showed distinct improvement over mice receiving vehicle (G1) in Days 12-24 (Figure 13A), and mouse body weights for AMDACs-receiving mice were notably higher than vehicle-receiving mice in Days 12-28 (Figure 13B). Moreover, mice receiving AMDACs developed symptoms at a significantly later median Day post-administration than mice receiving only vehicle (p=0.0275; Figure 14). Mice receiving no treatment developed symptoms as expected, and mice receiving no MOG33-55 did not develop significant symptoms over the course of the experiment.

Thus, administration of AMDACs improved not only the degree of symptoms and ameliorated loss of body weight seen in symptomatic mice, but delayed onset of symptoms, indicating that AMDACs have therapeutic benefit in the treatment of multiple sclerosis.

## Claims

1. Amnion-derived adherent cells (AMDACs) for use in a method of treatment of an individual having or at risk of developing asthma, graft-versus-host disease, inflammatory bowel disease, multiple sclerosis, rheumatoid arthritis, psoriasis, lupus erythematosus, diabetes, mycosis fungoides, or scleroderma, wherein the method comprises administering to the individual a therapeutically effective amount of said amnion-derived adherent cells (AMDACs), wherein said therapeutically effective amount is an amount sufficient to cause a detectable improvement in one or more symptoms of said asthma, graft-versus-host disease, inflammatory bowel disease, multiple sclerosis, rheumatoid arthritis, psoriasis, lupus erythematosus, diabetes, mycosis fungoides, or scleroderma, and wherein said AMDACs are OCT-4⁻ as determinable by RT-PCR and CD200⁺ as determinable by flow cytometry, and are adherent to tissue culture plastic.

2. The AMDACs for use of claim 1, wherein said disease or disorder is caused by or mediated by a Th1 or Th17 pro-inflammatory response, and wherein said administering comprises contacting T cells that mediate said Th1 or Th17 response with said AMDACs.

3. The AMDACs for use of claim 1 or 2, wherein said AMDACs:
(i) reduce Th1 cell maturation in said individual;
(ii) upregulate a regulatory T cell phenotype in said individual;
(iii) reduce expression in dendritic cells (DCs) and/or macrophages of biomolecules that promote a Th1 and/or Th17 response in said individual; or
(iv) detectably reduce production of one or more of lymphotoxin-1a (LT-1α), interleukin-1β (IL-1β), IL-12, IL-17, IL-21, IL-23, tumor necrosis factor alpha (TNFα) and/or interferon gamma (IFNγ) by said T cells.

4. The AMDACs for use of any one of claims 1 to 3, wherein the method comprises additionally administering a second type of stem cells to said individual, wherein said second type of stem cells are embryonic stem cells, stem cells isolated from peripheral blood, stem cells isolated from placental blood, stem cells isolated from placental perfusate, non-AMDAC stem cells isolated from placental tissue, stem cells isolated from umbilical cord blood, umbilical cord stem cells, bone marrow-derived mesenchymal stem cells, adipose-derived stem cells, hematopoietic stem cells, or somatic stem cells.

5. The AMDACs for use of any one of claims 1 to 4, wherein said inflammatory bowel disease is
(i) Crohn's disease, preferably gastroduodenal Crohn's disease, jejunoileitis, ileocolitis, or Crohn's colitis; or
(ii) ulcerative colitis, preferably pancolitis, limited colitis, distal colitis, or proctitis.

6. The AMDACs for use of any one of claims 1 to 4, wherein said scleroderma is diffuse scleroderma, limited scleroderma (CREST syndrome), morphea, or linear scleroderma.

7. The AMDACs for use of any one of claims 1 to 4, wherein said mycosis fungoides is in the patch phase, in the skin tumor phase, in the skin redness (erythroderma) stage, or in the lymph node stage.

8. The AMDACs for use of claim 1 or 5, wherein said symptom of inflammatory bowel disease is one or more of inflammation and swelling of a part of the GI tract, abdominal pain, frequent emptying of the bowel, diarrhea, rectal bleeding, anemia, weight loss, arthritis, skin problems, fever, thickening of the intestinal wall, formation of scar tissue in the intestine of the individual, formation of sores or ulcers in the intestine of the individual, development of one or more fistulas in the wall of the intestinal of the individual, development of one or more fissures in the anus of the individual, development of nutritional deficiencies (e.g., deficiencies in one or more of proteins, calories, vitamins), development of kidney stones, development of gallstones, abdominal pain, bloody diarrhea, , nausea, abdominal cramps, fatigue, loss of appetite, loss of bodily fluids and nutrients, skin lesions, joint pain, growth failure, osteoporosis, eye inflammation, or liver disease.

9. The AMDACs for use of claim 1 or 6, wherein said symptom of scleroderma is one or more of hardening of the skin of the face, hardening of the skin of the fingers, Reynaud's syndrome, inappropriate vasoconstriction in an extremity, calcinosis, telangiectasia, or esophageal dysmotility.

10. The AMDACs for use of claim 1, wherein said symptom of psoriasis is psoriatic arthritis; or is one or more of scaling of the skin, redness of the skin, thickening of the skin, formation of plaques, discoloration under the nail plate, pitting of the nails, lines going across the nails, thickening of the skin under the nail, onycholysis, development of pustules, joint or connective tissue inflammation, inflammation of the skin, or exfoliation of the skin.

11. The AMDACs for use of claim 1, wherein said symptom of multiple sclerosis is one or more of a sensory disturbance in a limb of the individual, optic nerve dysfunction, pyramidal tract dysfunction, bladder dysfunction, bowel dysfunction, sexual dysfunction, ataxia, or diplopia.

12. The AMDACs for use of claim 1, wherein said rheumatoid arthritis involves one or more of pyoderma gangrenosum, neutrophilic dermatosis, Sweet's syndrome, viral infection, erythema nodosum, lobular panniculitis, atrophy of digital skin, palmar erythema, diffuse thinning (rice paper skin), skin fragility, subcutaneous nodules on an exterior surface, *e.g*., on the elbows, fibrosis of the lungs (*e.g*., as a consequence of methotrexate therapy), Caplan's nodules, vasculitic disorders, nail fold infarcts, neuropathy, nephropathy, amyloidosis, muscular pseudohypertrophy, endoscarditis, left ventricular failure, valulitis, scleromalacia, mononeuritis multiplex, atlanto-axial subluxation.

13. The AMDACs for use of claim 1, wherein said symptom of lupus erythematosus is one or more of malar rash, development of thick red scaly patches on the skin, alopecia, mouth ulcers, nasal ulcers, vaginal ulcers, skin lesions, joint pain, anemia deficiency, iron deficiency, lower than normal platelet and white blood cell counts, antiphospholipid antibody syndrome, presence of anticardiolipin antibody in the blood, pericarditis, myocarditis, endocarditis, lung inflammation, pleural inflammation, pleuritis, pleural effusion, lupus pneumonitis, pulmonary hypertension, pulmonary emboli, pulmonary hemorrhage, autoimmune hepatitis, jaundice, presence of antinuclear antibody (ANA) in the blood, presence of smooth muscle antibody (SMA) in the blood, presence of liver/kidney microsomal antibody (LKM-1) in the blood, presence of anti-mitochondrial antibody (AMA) in the blood, hematuria, proteinuria, lupus nephritis, renal failure, development of membranous glomerulonephritis with "wire loop" abnormalities, seizures, psychosis, abnormalities in the cerebrospinal fluid, deficiency in CD45 phosphatase and/or increased expression of CD40 ligand in T cells of the individual, lupus gastroenteritis, lupus pancreatitis, lupus cystitis, autoimmune inner ear disease, parasympathetic dysfunction, retinal vasculitis, systemic vasculitis, increased expression of FcεRIγ, increased and sustained calcium levels in T cells, increase of inositol triphosphate in the blood, reduction in protein kinase C phosphorylation, reduction in Ras-MAP kinase signaling, or a deficiency in protein kinase A I activity.

14. The AMDACs for use of claim 1, wherein said symptom of diabetes is one or more of abnormally high blood sugar, lack of insulin resistance as determined by a glucose tolerance test, fatigue, or loss of consciousness.

15. The AMDACs for use of claim 1 or 7, wherein said symptom of mycosis fungoides is one or more of development of flat red patches that are itchy, development of flat, red patches that are raised and hard (plaques), development of raised lumps (nodules), development of large red itchy scaly areas over the body, cracking of the skin of the palms and soles, thickening of the skin of the palms and soles, or inflammation of the lymph nodes.

## Patentansprüche

1. Amnion-abgeleitete adhärente Zellen (AMDACs) für die Verwendung in einem Verfahren zur Behandlung eines Individuums mit oder dem Risiko der Entwicklung von Asthma, Graft-versus-Host Erkrankung, chronisch-entzündlicher Darmerkrankung, Multipler Sklerose, rheumatoider Arthritis, Psoriasis, Lupus erythematosus, Diabetes, Mycosis fungoides oder Sklerodermie, wobei das Verfahren das Verabreichen einer therapeutisch wirksamen Menge der Amnion-abgeleiteten adhärenten Zellen (AMDACs) an das Individuum umfasst, wobei die therapeutisch wirksame Menge eine Menge ist, die ausreicht, eine nachweisbare Verbesserung eines oder mehrerer Symptome von Asthma, Graft-versus-Host Erkrankung, chronisch-entzündlicher Darmerkrankung, Multipler Sklerose, rheumatoider Arthritis, Psoriasis, Lupus erythematosus, Diabetes, Mycosis fungoides oder Sklerodermie zu bewirken, und wobei die AMDACs OCT-4⁻, wie durch RT-PCR bestimmbar, und CD200⁺, wie durch Durchflusszytometrie bestimmbar, sind und an Gewebekulturkunststoff anhaften.

2. AMDACs für die Verwendung nach Anspruch 1, wobei die Erkrankung oder Krankheit durch eine Th1 oder Th17 pro-Entzündungsantwort verursacht oder vermittelt wird, und wobei das Verabreichen das In-Kontakt-bringen der T Zellen, die die Th1 oder Th17 Antwort vermitteln, mit den AMDACs umfasst.

3. AMDACs für die Verwendung nach Anspruch 1 oder 2, wobei die AMDACs:
(i) Th1 Zellmaturation in dem Individuum verringern;
(ii) einen regulatorischen T Zell-Phänotyp in dem Individuum hochregeln;
(iii) die Expression in dendritischen Zellen (DCs) und/oder Macrophagen von Biomolekülen, die eine Th1 und/oder Th17 Antwort fördern, in dem Individuum verringern; oder
(iv) nachweisbar die Erzeugung von einem oder mehreren von Lymphotoxin-1α (LT-1α), Interleukin-1β (IL-1β), IL-12, IL-17, IL-21, IL-23, Tumornekrosefaktor alpha (TNFα) und/oder Interferon gamma (IFNγ) durch die T Zellen verringern.

4. AMDACs für die Verwendung nach einem der Ansprüche 1 bis 3, wobei das Verfahren zusätzlich das Verabreichen einer zweiten Art von Stammzellen an das Individuum umfasst, wobei die zweite Art von Stammzellen embryonale Stammzellen, aus peripherem Blut isolierte Stammzellen, aus plazentarem Blut isolierte Stammzellen, aus plazentarem Perfusat isolierte Stammzellen, aus plazentarem Gewebe isolierte nicht-AMDAC Stammzellen, aus Nabelschnurblut isolierte Stammzellen, Nabelschnurstammzellen, von Knochenmark abgeleitete mesenchymale Stammzellen, Körperfett-abgeleitete Stammzellen, blutbildende Stammzellen oder somatische Stammzellen sind.

5. AMDACs für die Verwendung nach einem der Ansprüche 1 bis 4, wobei die chronisch-entzündliche Darmerkrankung
(i) Morbus Crohn, bevorzugt gastroduodenaler Morbus Crohn, Jejunoileitis, Ileocolitis oder Colitis Crohn; oder
(ii) Colitis ulcerosa, bevorzugt Pancolitis, limitierte Colitis, distale Colitis oder Proktitis ist.

6. AMDACs für die Verwendung nach einem der Ansprüche 1 bis 4, wobei die Sklerodermie diffuse Sklerodermie, limitierte Sklerodermie (CREST Syndrom), Morphea oder lineare Sklerodermie ist.

7. AMDACs für die Verwendung nach einem der Ansprüche 1 bis 4, wobei die Mycosis fungoides in der Patch-Phase, in der Hauttumor-Phase, im Hautrötestadium (erythroderma) oder im Lympknotenstadium ist.

8. AMDACs für die Verwendung nach Anspruch 1 oder 5, wobei das Symptom der chronisch-entzündlichen Darmerkrankung eines oder mehrere von Entzündung und Schwellen eines Teils des GI Traktes, abdominaler Schmerz, häufige Darmentleerung, Diarrhö, rektales Bluten, Anämie, Gewichtsverlust, Arthritis, Hautprobleme, Fieber, Verdickung der Darmwand, Bildung von Narbengewebe im Darm des Individuums, Bildung von Wunden oder Geschwüren im Darm des Individuums, Bildung von einer oder mehreren Fisteln in der Darmwand des Individuums, Bildung von einer oder mehreren Fissuren im Anus des Individuums, Bildung von Ernährungsmängeln (*z*.*B*. Mängel an einem oder mehreren von Proteinen, Kalorien, Vitaminen), Bildung von Nierensteinen, Bildung von Gallensteinen, Bauchschmerzen, blutige Diarrhö, Übelkeit, Magenkrämpfe, Müdigkeit, Appetitlosigkeit, Verlust von Körperflüssigkeiten und Nährstoffen, Hautläsionen, Gelenkschmerzen, Wachstumsstörung, Osteoporose, Augenentzündung oder Lebererkrankungen ist.

9. AMDACs für die Verwendung nach Anspruch 1 oder 6, wobei das Symptom von Sklerodermie eines oder mehrere von Verhärten der Gesichtshaut, Verhärten der Haut der Finger, Reynaud-Syndrom, unerwünschte Vasokonstriktion in einer Extremität, Calcinose, Telangiektasie oder esophageale Dysmotilität ist.

10. AMDACs für die Verwendung nach Anspruch 1, wobei das Symptom von Psoriasis Psoriasis arthritis ist; oder eines oder mehrere von Hautverkrustung, Hautrötung, Hautverdickung, Bildung von Plaque, Verfärbung unter der Nagelplatte, Nagellochfraß, Linien, die entlang der Nägel verlaufen, Verdickung der Haut unter den Nägeln, Onycholyse, Entwicklung von Pusteln, Gelenk- oder Bindegewebsentzündung, Hautentzündung oder Abblättern der Haut ist.

11. AMDACs für die Verwendung nach Anspruch 1, wobei das Symptom von Multipler Sklerose eines oder mehrere von sensorische Beeinträchtigung in einer Extremität des Individuums, optische Nervendysfunktion, Pyramidenbahndysfunktion, Blasendysfunktion, Darmdysfunktion, funktionelle Sexualstörung, Ataxie oder Diplopie ist.

12. AMDACs für die Verwendung nach Anspruch 1, wobei die rheumatoide Arthritis eines oder mehrere von Pyoderma gangraenosum, neutrophile Dermatose, Sweet-Syndrom, virale Infektion, Erythema nodosum, lobuläre Pannikulitis, Atrophie von digitaler Haut, Palmarerythem, diffuse Ausdünnung (Reispapierhaut), Hautfragilität, subkutane Knötchen an einer äußeren Fläche, *z.B.* an den Elbogen, Fibrose der Lunge (*z.B.* als eine Konsequenz einer Methotrexat-Therapie), Caplan-Syndrom, vaskuläre Erkrankungen, Nagelfalteninfarkte, Neuropathie, Nephropathie, Amyloidose, muskuläre Pseudohypertrophie, Endoskarditis, linkes Kammerversagen, Valulitis, Skleromalakie, Mononeuritis multiplex, atlanto-axiale Subluxation beinhaltet.

13. AMDACs für die Verwendung nach Anspruch 1, wobei das Symptom von Lupus erythematodes eines oder mehrere von Schmetterlingserythem, Entwicklung von dicken roten schuppigen Flecken auf der Haut, Alopezie, Mundgeschwüre, Nasengeschwüre, Vaginageschwüre, Hautläsionen, Gelenkschmerz, Mangelanämie, Eisenmangel, geringer Anzahl als normal an Blutplättchen und weißen Blutzellen, antiphospholipid Antikörper-Syndrom, Vorhandensein von Anticardiolipin-Antikörper im Blut, Perikarditis, Myokarditis, Endokarditis, Lungenentzündung, Pleuraentzündung, Pleuritis, Pleuraerguss, Lupus pneumonitis, Lungenhochdruck, Lungenembolie, Lungenhämorrhage, Autoimmunhepatitis, Gelbsucht, Vorhandensein von antinuklearem Antikörper (ANA) im Blut, Vorhandensein von glatter Muskel-Antikörper (SMA) im Blut, Vorhandensein von Leber/Nieren mikrosomalem Antikörper (LKM-1) im Blut, Vorhandensein von anti-mitochondrialem Antikörper (AMA) im Blut, Hämaturie, Proteinurie, Lupus nephritis, Nierenversagen, Bildung membranöser Glomerulonephritis mit "wire loop" Abnormalitäten, Krämpfe, Psychose, Abnormalitäten in der Zerebrospinalflüssigkeit, Mangel in CD45 Phosphatase und/oder erhöhte Exprimierung von CD40 Ligand in T Zellen des Individuums, Lupus gastroenteritis, Lupus pancreatitis, Lupus cystitis, Autoimmun-Innenohrerkrankung, parasympathetische Dysfunktion, retinale Vaskulitis, systemische Vaskulitis, erhöhte Exprimierung von FcεRIγ, erhöhte und anhaltende Calciumniveaus in T Zellen, Anstieg von Inositoltriphosphat im Blut, Verringerung in Proteinkinase C Phosphorylierung, Verringerung in Ras-MAP Kinasesignalgebung oder ein Mangel in Proteinkinase A I Aktivität ist.

14. AMDACs für die Verwendung nach Anspruch 1, wobei das Symptom von Diabetes eines oder mehrere von abnormal hohem Blutzucker, Mangel an Insulinresistenz wie durch Glukosetoleranztest bestimmt, Müdigkeit oder Bewusstlosigkeit ist.

15. AMDACs für die Verwendung nach Anspruch 1 oder 7, wobei das Symptom von Mycosis fungoides eines oder mehrere von der Entwicklung flachen roten Flecken, die jucken, Bildung von flachen roten Flecken, die hervorstehen und hart sind (Plaques), Bildung von hervorstehenden Geschwülsten (Knötchen), Bildung von großen roten juckenden schuppigen Bereichen am Körper, Rissbildung der Haut an den Handflächen und Fußsohlen, Verdickungen der Haut an den Handflächen und Fußsohlen oder Entzündung der Lympknoten ist.

## Revendications

1. Cellules adhérentes dérivées de la membrane amniotique (AMDAC) à utiliser dans un procédé de traitement d'une personne affectée, ou présentant le risque de développer, de l'asthme, la maladie du greffon *versus* l'hôte, la maladie inflammatoire chronique de l'intestin, la sclérose en plaques, l'arthrite rhumatoïde, le psoriasis, le lupus érythémateux, le diabète, une mycose fongoïde, ou une sclérodermie, dans lesquelles le procédé comprend l'administration à la personne d'une quantité thérapeutiquement efficace desdites cellules adhérentes dérivées de la membrane amniotique (AMDAC), dans lesquelles ladite quantité thérapeutiquement efficace est une quantité suffisante pour provoquer une amélioration détectable de l'un ou de plusieurs symptômes dudit asthme, de ladite maladie du greffon *versus* de l'hôte, de la maladie inflammatoire chronique de l'intestin, de la sclérose en plaques, de l'arthrite rhumatoïde, du psoriasis, du lupus érythémateux, du diabète, de ladite mycose fongoïde, ou de ladite sclérodermie, et dans lesquelles lesdites AMDAC sont l'OCT-4⁻ comme cela peut être déterminé par PCR-TI et CD200⁺ comme cela peut être déterminé par cytométrie en flux, et sont adhérentes au plastique des cultures de tissu.

2. AMDAC à utiliser selon la revendication 1, dans laquelle ladite maladie ou ledit trouble est provoqué par ou médié par une réponse pro-inflammatoire Th1 ou Th17, et dans laquelle ladite administration comprend la mise en contact des cellules T qui assurent la médiation de ladite réponse Th1 ou Th17 avec lesdites AMDAC.

3. AMDAC à utiliser selon la revendication 1 ou 2, dans laquelle lesdites AMDAC :
(i) réduisent la maturation des cellules Th1 chez ladite personne ;
(ii) régulent à la hausse un phénotype régulateur de cellules T chez ladite personne ;
(iii) réduisent l'expression des biomolécules qui favorisent une réponse Th1 et/ou Th17 dans les cellules dendritiques (DC) et/ou dans les macrophages chez ladite personne ; ou
(iv) réduisent de manière détectable la production d'une ou plusieurs parmi la lymphotoxine-1α(LT-1α), l'interleukine-1β (IL-1β), l'IL-12, l'IL-17, l'IL-21, l'IL-23, le facteur de nécrose des tumeurs alpha (TNFα) et/ou l'interféron gamma (IFNγ) par lesdites cellules T.

4. AMDAC à utiliser selon l'une quelconque des revendications 1 à 3, dans laquelle le procédé comprend l'administration de manière supplémentaire d'un second type de cellules souches à ladite personne, dans laquelle ledit second type de cellules souches sont des cellules souches embryonnaires, des cellules souches isolées du sang périphérique, des cellules souches isolées du sang placentaire, des cellules souches isolées du perfusat placentaire, des cellules souches non-AMDAC isolées du tissu placentaire, des cellules souches isolées du sang du cordon ombilical, des cellules souches du cordon ombilical, des cellules souches mésenchymateuses dérivées de la moelle osseuse, des cellules souches dérivées de l'adipose, des cellules souches hématopoïétiques, ou des cellules souches somatiques.

5. AMDAC à utiliser selon l'une quelconque des revendications 1 à 4, dans laquelle ladite maladie inflammatoire chronique de l'intestin est
(i) la maladie de Crohn, de préférence la maladie gastroduodénale de Crohn, la jéjuno-iléite, l'iléocolite, ou la colite de Crohn ; ou
(ii) la rectocolite hémorragique, de préférence la pancolite, la colite limitée, la colite distale, ou la proctite.

6. AMDAC à utiliser selon l'une quelconque des revendications 1 à 4, dans laquelle ladite sclérodermie est la sclérodermie diffuse, la sclérodermie limitée (syndrome de CREST), la morphée, ou la sclérodermie linéaire.

7. AMDAC à utiliser selon l'une quelconque des revendications 1 à 4, dans laquelle ladite mycose fongoïde se trouve en phase plaque (« patch phase»), en phase tumeur cutanée, au stade rougeur cutanée (érythroderme), ou au stade noeud lymphatique.

8. AMDAC à utiliser selon la revendication 1 ou 5, dans laquelle ledit symptôme de maladie inflammatoire chronique de l'intestin est l'une ou plusieurs parmi l'inflammation et le gonflement d'une partie du tractus GI, de la douleur abdominale, de la vidange fréquente des intestins, de la diarrhée, du saignement rectal, de l'anémie, de la perte de poids, de l'arthrite, des problèmes de peau, de la fièvre, de l'épaississement de la paroi intestinale, de la formation de tissu cicatriciel dans l'intestin de la personne, de la formation de plaies ou d'ulcères dans l'intestin de la personne, du développement d'une ou plusieurs fistules dans la paroi intestinale de la personne, du développement d'une ou plusieurs fissures dans l'anus de la personne, du développement de carences nutritionnelles (par exemple, carences en une ou plusieurs protéines, calories, vitamines), du développement de calculs rénaux, du développement de calculs biliaires, de la douleur abdominale, de la diarrhée sanguinolente, de la nausée, des crampes abdominales, de la fatigue, de la perte d'appétit, de la perte de liquides corporels et de nutriments, des lésions cutanées, des douleurs articulaires, des troubles de la croissance, de l'ostéoporose, de l'inflammation oculaire, ou de la maladie hépatique.

9. AMDAC à utiliser selon la revendication 1 ou 6, dans laquelle ledit symptôme de sclérodermie est l'un ou plusieurs parmi le durcissement de la peau du visage, le durcissement de la peau des doigts, le syndrome de Reynaud, la vasoconstriction inappropriée au niveau d'une extrémité, la calcinose, la télangiectasie, ou la dysmotilité oesophagienne.

10. AMDAC à utiliser selon la revendication 1, dans laquelle ledit symptôme de psoriasis est l'arthrite psoriasique ; ou est l'un ou plusieurs parmi la desquamation de la peau, la rougeur de la peau, l'épaississement de la peau, la formation de plaques, la décoloration sous la plaque des ongles, les ongles de couturière, les lignes traversant les ongles, l'épaississement de la peau sous l'ongle, l'onycholyse, le développement de pustules, l'inflammation du tissu articulaire ou conjonctif, l'inflammation de la peau, ou l'exfoliation de la peau.

11. AMDAC à utiliser selon la revendication 1, dans laquelle ledit symptôme de sclérose en plaques est l'un ou plusieurs parmi une perturbation sensorielle au niveau d'un membre d'une personne, le dysfonctionnement du nerf optique, le dysfonctionnement du tractus pyramidal, le dysfonctionnement de la vessie, le dysfonctionnement des intestins, le dysfonctionnement sexuel, l'ataxie, ou la diplopie.

12. AMDAC à utiliser selon la revendication 1, dans laquelle ladite arthrite rhumatoïde implique l'un ou plusieurs parmi le *pyoderma gangrenosum*, la dermatose neutrophile, le syndrome de Sweet, l'infection virale, l'érythème nodosum, la panniculite lobulaire, l'atrophie de la peau des doigts, l'érythème palmaire, l'amincissement diffus (peau en papier de riz), la fragilité de la peau, les nodules sous-cutanés sur une surface externe, par exemple, sur les coudes, la fibrose des poumons (par exemple, en conséquence d'une thérapie au méthotrexate), les nodules de Caplan, les troubles vasculitiques, l'infarctus des plis des ongles, la neuropathie, la néphropathie, l'amyloïdose, la pseudohypertrophie musculaire, l'endoscardite, l'insuffisance du ventricule gauche, la valulite, la scléromalacie, la mononévrite multiplexe, la sous-luxation atlanto-axiale.

13. AMDAC à utiliser selon la revendication 1, dans laquelle ledit symptôme de lupus érythémateux est l'un ou plusieurs parmi l'érythème malaire, le développement de plaques squameuses épaisses rouges sur la peau, l'alopécie, les ulcères buccaux, les ulcères nasaux, les ulcères vaginaux, les lésions de la peau, la douleur articulaire, la carence anémique, la carence en fer, les numérations des plaquettes et des globules blancs inférieures à la normale, le syndrome des anticorps antiphospholipides, la présence d'anticorps anticardiolipine dans le sang, la péricardite, la myocardite, l'endocardite, l'inflammation des poumons, l'inflammation de la plèvre, la pleurite, l'épanchement pleural, la pneumonie lupique, l'hypertension pulmonaire, l'embolie pulmonaire, l'hémorragie pulmonaire, l'hépatite auto-immune, la jaunisse, la présence d'anticorps antinucléaires (ANA) dans le sang, la présence d'anticorps du muscle lisse (SMA) dans le sang, la présence d'anticorps microsomiaux du foie/des reins (LKM-1) dans le sang, la présence d'anticorps anti-mitochondries (AMA) dans le sang, l'hématurie, la protéinurie, la néphrite lupique, l'insuffisance rénale, le développement de la glomérulonéphrite membraneuse avec des anomalies en « boucle de câble », les attaques, la psychose, les anomalies du liquide cérébrospinal, la carence en phosphatase CD45 et/ou l'expression accrue du ligand CD40 dans les cellules T de patients, la gastro-entérite lupique, la pancréatite lupique, la cystite lupique, la maladie auto-immune de l'oreille interne, le dysfonctionnement parasympathique, la vasculite rétinienne, la vasculite systémique, l'expression accrue de FcεRIγ, les niveaux accrus et durables de calcium dans les cellules T, l'augmentation de l'inositol triphosphate dans le sang, la réduction de la phosphorylation de la protéine kinase C, la réduction dans la signalisation de la Ras-MAP kinase, ou une carence dans l'activité de la protéine A I.

14. AMDAC à utiliser selon la revendication 1, dans laquelle ledit symptôme de diabète est l'un ou plusieurs parmi la glycémie anormalement élevée, le manque de résistance à l'insuline tel que déterminé par un test de tolérance au glucose, la fatigue, ou la perte de conscience.

15. AMDAC à utiliser selon la revendication 1 ou 7, dans laquelle ledit symptôme de mycose fongoïde est l'un ou plusieurs parmi le développement de plaques plates et rouges qui démangent, le développement de plaques rouges plates qui sont surélevées et dures (plaques), le développement de grosseurs élevées (nodules), le développement de grandes zones squameuses rouges qui démangent sur le corps, la fissuration de la peau de la paume des mains et de la plante des pieds, l'épaississement de la peau de la paume des mains et de la plante des pieds, ou l'inflammation des noeuds lymphatiques.
